# EUROPEAN PATENT APPLICATION

(11) **EP 2 540 710 A1**
(43) Date of publication of application: **02.01.2013**
(21) Application number: 11075154.2
(22) Date of filing: 30.06.2011
(51) Int. Cl.: C07D 249/04, C07D 249/18, C07D 471/04

(54) **New precursors for direct radiosynthesis of protected derivatives of O-([18F]Fluoromethyl) tyrosine**

(71) Applicant: Bayer Schering Pharma Aktiengesellschaft, 13353 Berlin (DE)
(72) Inventor: Brumby,Thomas, Dr., 12163 Berlin (DE); Graham, Keith, 10405 Berlin (DE); Krüger, Martin, 13465 Berlin (DE)

(57) **Abstract**

The invention describes novel and stable precursors for the direct radiosynthesis of protected derivatives of *O*-([¹⁸F]Fluoromethyl) tyrosines, and methods for obtaining thoses compounds.

## Description

### Field of invention:

The invention describes novel and stable precursors for the direct radiosynthesis of protected derivatives of *O*-([¹⁸F]Fluoromethyl)tyrosines, and methods for obtaining thoses compounds.

### Background art:

Both *O*-(Fluoromethyl)-D-tyrosine and *O*-(Fluoromethyl)-L-tyrosine have been described as PET-tracers for in vivo imaging of various tumor types. (D-FMT: WO 2005115971*;* Eur. J. Nucl. Med. Mol. Imag. 2006, p1017*;* J. Nucl. Med. 50, p290, 2009*;* J. Nucl. Med. 47, p679, 2006*;* Nuc. Med. Biol. 2009 p295*;* L-FMT, WO 2005009928*;* J. Label. Comp. Radiopharm. 46, p555, 2003). In all syntheses reported to date for these compounds, a so called "indirect" labelling has been employed. It consists of preparation of a labelled ¹⁸F-synthon (e.g. Fluoromethyl bromide, Fluoromethyl-tosylate, -mesylate or -triflate), which is then reacted with tyrosine to give the desired tracer. The radiochemical practise knows a variety of such labelling reagents, but not only for synthesis of O-fluoromethyltyrosines, as detailed in the table 1.

**Table 1: Reagents for indirect synthesis of ¹⁸F-Fluoromethyltracers**

| Tracer (trivial name) | CAS | Literature | | Reagent (F-synthon) |
|---|---|---|---|---|
| | 1083103-28-9 | WO 2008141249 GE | | F-CH₂OTs |
| | 1059188-91-8 | Journal of Medicinal Chemistry (2008), 51(18), 5833-5842 | | F-CH₂-Br |
| | 947395-20-2 | WO 2007096193 | A3 | F-CH₂-Br |
| [¹⁸F]FM-SA4503 | 1004511-89-0 | Nuclear Medicine and Biology (2007), 34(5), 571-577 | | Indirect |
| | 934200-18-7 | WO 2007041025 | A3 | F-CD₂-Br |
| [¹⁸F]SPA-RQ | 262598-99-2 | Journal of Labelled Compounds and Radio pharmaceuticals (2006), 49(1), 17-31 and 49(11), 935-937 (correction) Synapse (2007), 61 (4), 242-251 | | F-CH₂-Br |
| | 863887-82-5 | WO 2005079391 | A3 | F-CH₂-Br |
| | 849469-05-2 | WO 2005030723 | A1 | F-CH₂OTf |
| di-deutero FMDAA1106 | 848769-79-9 | Bioorganic & Medicinal Chemistry (2005), 13(5), 1811-1818 | | FCH₂I |
| D-FMT | 870452-26-9 | WO 2005115971 | A1 | indirect |
| L-FMT | 627092-21-1 | WO 2005009928 | A3 | indirect |
| | 867281-18-3 | Journal of Labelled Compounds & Radio pharmaceuticals (2005), 48(1), 1-10 | | BrCH₂F |
| | | Journal of Nuclear Medicine and Molecular Imaging (2006), 33(10),1134-1139 | | |
| FMDAA1106 | 505084-40-2 | US 6870069 | B2 | FCH₂I |
| | | Journal of Medicinal Chemistry (2004), 47(9), 2228-2235 | | Indirect and direct |
| | | Bioorganic & Medicinal Chemistry Letters (2003), 13(2), 201-204 | | FCH₂I |
| | 844446-45-3 | Synapse (New York, NY, US) (2004), 53(2), 57-67 | | BrCD₂F |
| (S,S)-[¹⁸F] FMeNER | 844446-44-2 | Synapse (2004), 53(2), 57-67 Psychopharmacology (2006), 188(1), 119-127 | | BrCH₂F |
| | 686768-01-4 | WO 2004038374 | A3 | BrCD₂F |
| | 677000-29-2 | WO 2004029024 | A3 | BrCD₂F |
| | 262598-99- | WO 2004029006 | A3 | FCH₂l, |
| | 2P | WO 2000018403 | A1 | FCH₂Br |
| | | Journal of Nuclear Medicine(2007),48(1),100-107 | | |
| | 844446-45-3 | Nuclear Medicine and Biology (2008), 35(7), 733-740 | | indirect |
| | 870452-26-9 | Journal of Nuclear Medicine (2006), 47(4), 679-688 | | indirect |
| | | European Journal of Nuclear Medicine and Molecular Imaging (2006), 33(9), 1017-1024 | | |
| | 851014-76-1 | Journal of Fluorine Chemistry (2004), 125(12), 1879-1886 | | FCH₂I |

Generally, such indirect syntheses require more steps compared to direct syntheses and some of the employed reagents are gaseous, thus requiring special equipment not present in every laboratory. Thus a direct approach could be much easier. Although chloromethyl ethers of phenols are known and some are commercially available, they have to date not been used as precursors to ¹⁸F-labelled Fluoromethylarylethers. This might be due to stability problems. For example, the chloromethyl ether of BOC tyrosine methyl ester was synthesized and found to be chemically not stable (Angew. Chem. Int. Ed. 2002, 3449). Other authors found such compounds stable, but very reactive when dissolved in solvents containing water (J. Appl. Chem. 1953, 266). As usual labelling conditions (Kryxptofix /KF/K₂CO₂/polar aprotic solvent) are rarely completely anhydrous, this might explain the failure of chloromethylaryl ethers as precursors to labelled fluoromethylaryl ethers. There is only one report for use of an iodomethylaryl ether as a labelling precursor to make a fluoromethoxy-labelled tracer (Bioorganic & Medicinal Chemistry Letters (2003), 13(2), 201-204). This report stated, that the labelled fluoromethoxy compound "could be obtained, but the radiochemical yield was not reproducible (0-35%)". In the end, the authors relied for production of the tracer on the established indirect methods. Aromatic tosyloxymethoxy-compounds can be synthesized (e.g. Synthesis 1971, 150), but such compounds have not been used for synthesis of ¹⁸F-tracers. Thus it appears, that methoxy-compounds substituted with leaving groups commonly used for aliphatic nucleophilic substitution reactions (e.g. OCH₂-Hal, OCH₂-OTs, OCH₂-OMs or OCH₂-OTf), are not of practical use for the synthesis of ¹⁸F-fluoromethoxy-compounds (OCH₂-F).

So for direct synthesis of ¹⁸F fluoromethoxy compounds, precursors are lacking, which are stable compounds with a long shelf life unlike those compounds mentioned above, which do not decompose under standard labelling conditions and give reproducible results in the labelling reaction.

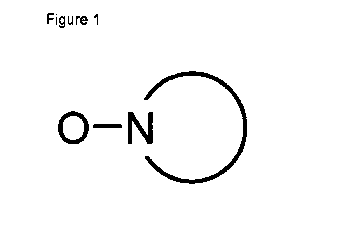

Activating groups consisting of a heterocyclic ring containing one to four nitrogen atoms with an oxygen atom directly attached to one of the nitrogens (further referred to as O-N-activating groups, Fig 1) are known and are commonly used in amide-forming reactions. (e.g. *N*-Hydroxybenzotriazole (HOBt), 7-aza-*N*-Hydroxybenzotriazole (HOAt), 6-chloro-*N-*hydroxybenzotriazole, 3-Hydroxy-1,2,3-benzotriazin-4(3H)-one, 1-Hydroxypyridinone, ethyl-1-hydroxy-1*H*-1,2,3 -triazole-4-carboxylate) (e.g. Houben-Weyl E22, 2003, p 443ff and 522ff). Such groups have also been used as leaving groups in aromatic nucleophilic substitution reactions to form ¹⁸F-substituted aromatic compounds (WO2008/104203A2).

Surprisingly, such O-N-activating groups can be used as leaving groups in aliphatic nucleophilic substitutions to form Fluoromethoxy-groups. They form stable precursors for reliable and reproducible synthesis of fluoromethoxy-compounds.

### Summary of the Invention:

The present invention is directed to novel compounds of Formula **I** and Formula **III** and methods for obtaining those compounds.

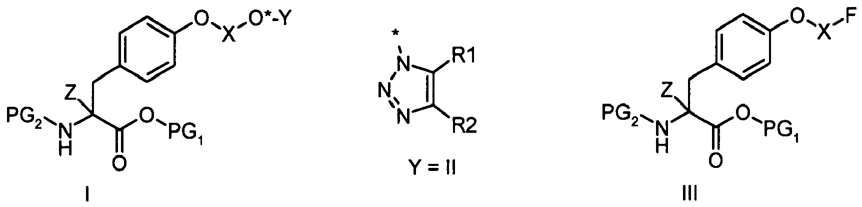

### Figure:

Figure 2. HPLC final product DFMT (QC).
Figure 3. HPLC final product DFMT (QC) + co-injection with cold reference.
Figure 4. HPLC final product DFMT (chiral).
Figure 5. HPLC final product DFMT (chiral) + co-injection with cold reference.

### Detailed Description of the Invention:

In a **first aspect,** the present invention is directed to compounds of Formula **I** 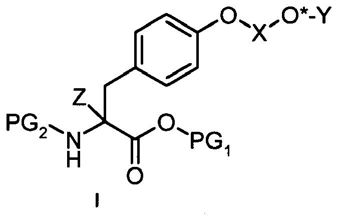 wherein:
- **X**: is CH₂, CHD or CD₂;
- **Y**: is a substituted or unsubstituted heteroaromatic ring containing one to four Nitrogen atoms (N) with the proviso that the oxygen (O*) is directly bound to one of the Nitrogen atoms (N) of the heteroaromatic ring and **O*-Y** acts as leaving group;
- **Z**: is Hydrogen or methyl;
- **PG1**: is a carboxylic acid protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula **I** and is also meant to comprise single isomers, diastereomers, enantiomers and mixtures thereof of Formula **I.**

### Preferred features:

Preferably, **Y** is 5 to 10 membered heteroaromatic ring containing one to four Nitrogen atoms (N).

The heteroaromatic ring is a single ring (preferably 5 or 6 membered with up to three Nitrogen atoms (N)) or a fused ring (preferably 9 or 10 membered with up to four Nitrogen atoms (N)).

A substituted heteroaromatic ring is substituted with halogen, NO2, CN, COOR3, SO2R3 or CF₃ wherein R3 is defined below.

Preferably, the heteroaromatic ring comprises 2 to 4, Nitrogen atoms (N)) more preferably 3 to 4 or 3.

More preferably, **Y** is a moiety of Formula **II** 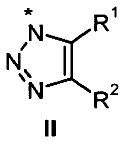 wherein
* indicates the position of the covalent bond to the Oxygen (O*) in Formula **I**;
- **R¹**: is H, CN, or COO**R⁴**, and **R²** is H, CN, or COO**R⁴**, or
- **R¹**: and **R²** form together a 6 membered aromatic ring, which optionally comprise 1 Nitrogen atoms (N) and 1 methine of the 6 membered ring is optionally substituted with halogen, NO₂, CN, COO**R³**, SO₂**R³** or CF₃,
- **R³**: is C₁-C₃ alkyl, and **R⁴** is C₁-C₆ alkyl.

Preferably, **R¹** and **R²** form together a 6 membered aromatic ring, which optionally comprise 1 Nitrogen atoms (N) and 1 methine of the 6 membered ring is optionally substituted with halogen, NO₂, or CF₃.

Preferably, **R³** is C₁ alkyl (methyl).

Preferably, **R⁴** is C₁ alkyl (methyl) or C₂ alkyl (ethyl).

Preferably, halogen is chloro (Cl).

Even more preferably, **Y** is 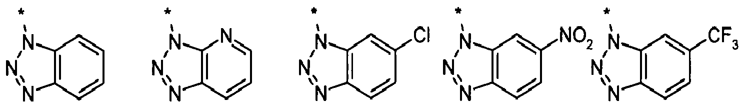 or 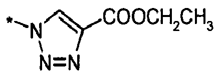 * indicates the position of the covalent bond to the Oxygen (O*) in Formula **I**. Even more preferably, **Y** is 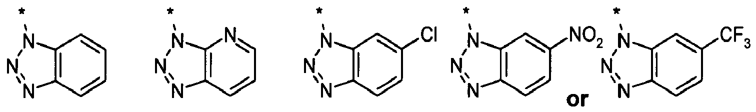 * indicates the position of the covalent bond to the Oxygen (O*) in Formula I.

Preferably, O*-**Y** acts as a leaving group suitable for introducing a fluoride.

**PG1** is a carboxylic acid protecting group (forming an ester) containing up to 20 carbon atoms, optionally containing independently one ore more O, N or S atoms; and compatible with radiolabeling conditions.

Preferably, **PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or
fluorenylmethyl
wherein
Alkyl is a branched or linear C₁-C₆ alkyl, and optionally substituted with C₁-C₃-alkoxy, and
Phenyl is optionally substituted with up to three C₁-C₃ alkyl, C₁-C₃ alkoxy or halogen.

PG1 is defined with the proviso that PG1 contains up to 20 carbon atoms,

Preferably, branched or linear C₁-C₆ alkyl is a C₁-C₃ alkyl. More preferably, C₁-C₆ alkyl is C₁-alkyl (methyl) when substituted and C₄-alkyl (e.g.tert-Butyl) when unsubstituted.

Preferably, branched or linear C₁-C₆ alkyl substituted with one phenyl is a branched or linear C₁-C₃ alkyl substituted with one phenyl. More preferably, branched or linear C₁-C₆ alkyl substituted with one phenyl is a methyl-phenyl (benzyl), ethyl-phenyl or i-Propyl-phenyl (e.g. Cumyl). Preferably, methyl-phenyl (benzyl), ethyl-phenyl and i-Propyl-phenyl (e.g. Cumyl) are is substituted with up to two methoxy-groups.

Preferably, C₁-C₃ alkoxy is C₁-alkoxy (methoxy).

Preferably, branched or linear C₁-C₆ alkyl substituted with one or two C₃-C₆ cycloalkyl is a branched or linear C₁-C₃ alkyl substituted with one or two cyclo-propyl.

Preferably, branched or linear C₁-C₆ alkyl substituted with one phenyl and one C₃-C₆ cycloalkyl is a branched or linear C₁-C₃ alkyl substituted with one phenyl and one C₃-C₆ cycloalkyl wherein the C₃-C₆ cycloalkyl is preferably C₃ cycloalkyl (cyclo-propyl),

Fluorenylmethyl is

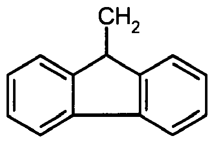

More preferably, **PG1** is
Alkyl,
Alkyl substituted with one phenyl,
Alkyl substituted with one or two C₃ cycloalkyl,
Alkyl substituted with one phenyl and one C₃ cycloalkyl, or fluorenylmethyl
wherein
Alkyl is a branched or linear C₁-C₄ alkyl, and optionally substituted with C₁-C₃-alkoxy, and
Phenyl is optionally substituted with up to three C₁-C₃ alkyl, C₁-C₃ alkoxy or halogen.

Even more preferably, **PG1** is 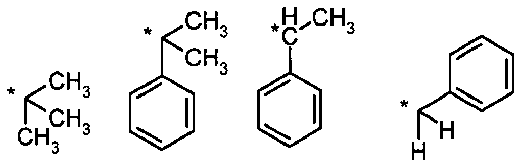 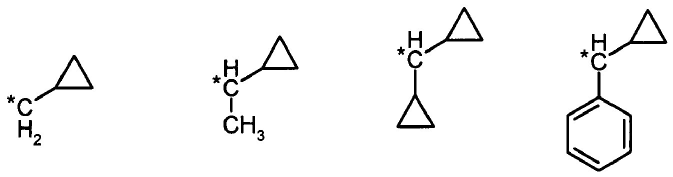 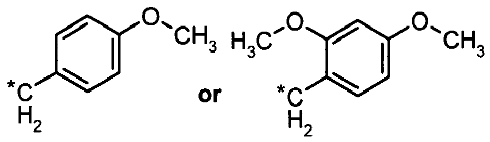 wherein * indicates the position of the Oxygen (O) forming the ester bond in Formula I.

Even more preferably, PG1 is 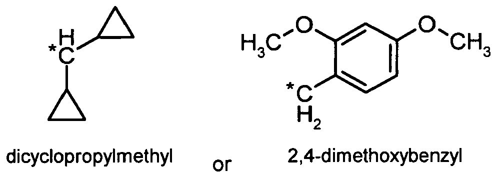 wherein * indicates the position of the Oxygen (O) forming the ester bond in Formula I.

**PG2** is an amino protecting group containing up to 20 carbon atoms, optionally containing one ore more O, N or S atoms and are optionally substituted with one to three halogen atoms, and compatible with radiolabeling conditions.

Preferably, PG2 is a carbamate or an arylalkyl protecting group containing up to 20 carbon atoms.

More preferably **PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

Even more preferably, **PG2** is tert-Butoxycarbonyl (BOC) or Triphenylmethyl (trityl).

Preferably, **Z** is Hydrogen.

### Preferred compound of Formula I:

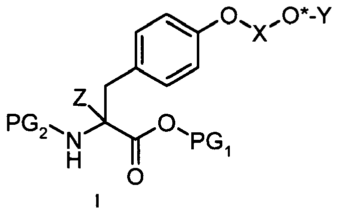

wherein:
- **X**: is CH₂ or CD₂;
- **Y**: is

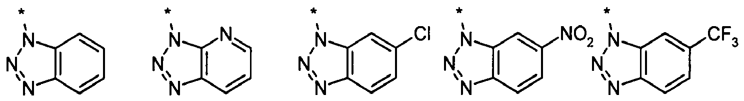

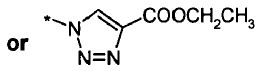

- **Z**: is Hydrogen or methyl;
- **PG1**: is dicyclopropylmethyl or 2,4-dimethoxybenzyl; and
- **PG2**: is tert-Butoxycarbonyl (BOC) or Triphenylmethyl (trityl).

In a **first** embodiment, the invention is directed to compounds of Formula **I** 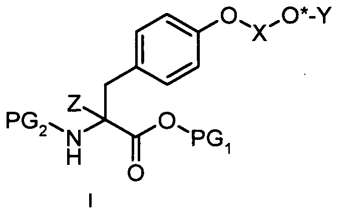 wherein:
- **X**: CH₂;
- **Y**: is a substituted or unsubstituted heteroaromatic ring containing one to four Nitrogen atoms (N) with the proviso that the oxygen (O*) is directly bound to one of the Nitrogen atoms (N) of the heteroaromatic ring and **O*-Y** acts as leaving group;
- **Z**: Hydrogen;
- **PG1**: is a carboxylic acid protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula I and is also meant to comprise single isomers, diastereomers, enantiomers and mixtures thereof of Formula I.

Formula **(la)** correspond to the Markush Formula below

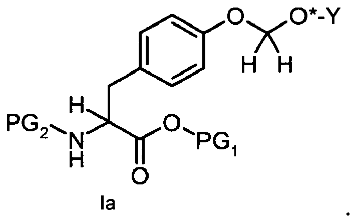

Preferred features disclosed above in respect **of Y, PG1** and **PG2** are incorporated herein.

Preferably, the invention is directed to compounds of Formula **(la)** wherein
**Y** is a moiety of Formula **II** 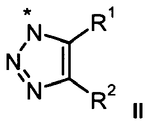 wherein
* indicates the position of the covalent bond to the Oxygen (O*) in Formula **Ia;**
**R¹** is H, CN, or COO**R⁴**, and **R²** is H, CN, or COO**R⁴**, or
**R¹** and **R²** form together a 6 membered aromatic ring, which optionally comprise 1 Nitrogen atoms (N) and 1 methine of the 6 membered ring is optionally substituted with halogen, NO₂, CN, COO**R³**, SO₂**R³** or CF₃,
**R³** is C₁-C₃ alkyl,
**R⁴** is C₁-C₆ alkyl;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

More preferably, compounds of Formula (**la**) is wherein
**Y** is 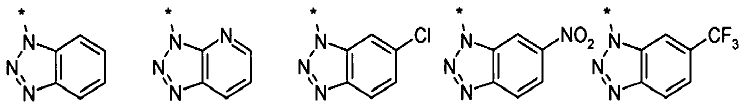 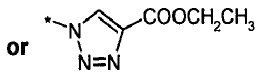 **PG1** is dicyclopropylmethyl or 2,4-dimethoxybenzyl, and
**PG2** is tert-Butoxycarbonyl (BOC) or Triphenylmethyl (trityl).

In a **second** embodiment, the invention is directed to compounds of Formula I 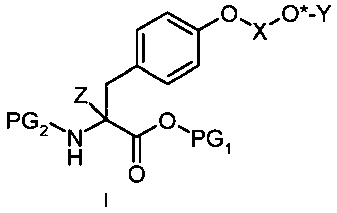 wherein:
- **X**: is CD₂;
- **Y**: is a substituted or unsubstituted heteroaromatic ring containing one to four Nitrogen atoms (N) with the proviso that the oxygen ( O *) is directly bound to one of the Nitrogen atoms (N) of the heteroaromatic ring and **O*-Y** acts as leaving group;
- **Z**: is Hydrogen;
- **PG1**: is a carboxylic acid protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula **I** and is also meant to comprise single isomers, diastereomers, enantiomers and mixtures thereof of Formula **I**.

Formula **(lb)** corresponds to the Markush Formula below

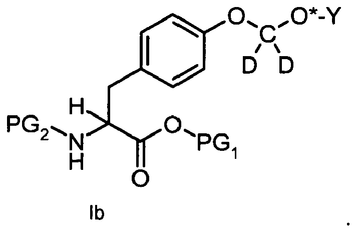

Preferred features disclosed above in respect of **Y, PG1** and **PG2** are incorporated herein.

Preferably, the invention is directed to compounds of Formula **(lb)** wherein
**Y** is a moiety of Formula **II** 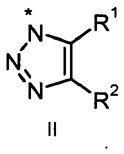 wherein
* indicates the position of the covalent bond to the Oxygen (O*) in Formula **Ib;**
**R¹** is H, CN, or COO**R⁴**, and **R²** is H, CN, or COO**R⁴,** or
**R¹** and **R²** form together a 6 membered aromatic ring, which optionally comprise 1 Nitrogen atoms (N) and 1 methine of the 6 membered ring is optionally substituted with halogen, NO₂, CN, COO**R³**, SO₂**R³** or CF₃,
**R³** is C₁-C₃ alkyl,
**R⁴** is C₁-C₆ alkyl;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

More preferably, compounds of Formula (**Ib**) is wherein
**Y** is 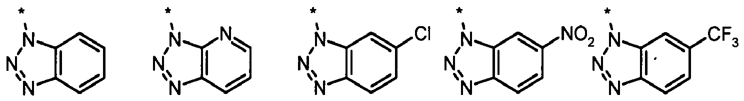 or 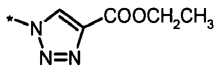 **PG1** is dicyclopropylmethyl or 2,4-dimethoxybenzyl, and
**PG2** is tert-Butoxycarbonyl (BOC) or Triphenylmethyl (trityl).

In a **third** embodiment, the invention is directed to compounds of Formula I 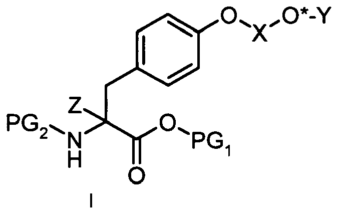 wherein:
- **X**: is CH₂;
- **Y**: is a substituted or unsubstituted heteroaromatic ring containing one to four Nitrogen atoms (N) with the proviso that the oxygen (O*) is directly bound to one of the Nitrogen atoms (N) of the heteroaromatic ring and **O*-Y** acts as leaving group;
- **Z**: is methyl;
- **PG1**: is a carboxylic acid protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula **I** and is also meant to comprise single isomers, diastereomers, enantiomers and mixtures thereof of Formula **I**.

Formula **(Ic)** corresponds to to the Markush Formula below

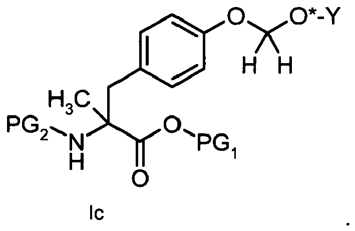

Preferred features disclosed above in respect of **Y**, **PG1** and **PG2** are incorporated herein.

Preferably, the invention is directed to compounds of Formula (**Ic**) wherein
**Y** is a moiety of Formula **II** 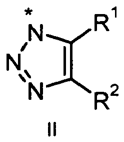 wherein
* indicates the position of the covalent bond to the Oxygen (O*) in Formula **Ic**;
**R¹**. is H, CN, or COO**R⁴**, and **R²** is H, CN, or COO**R⁴**, or
**R¹** and **R²** form together a 6 membered aromatic ring, which optionally comprise 1 Nitrogen atoms (N) and 1 methine of the 6 membered ring is optionally substituted with halogen, NO₂, CN, COO**R³**, SO₂**R³** or CF₃,
**R³** is C₁-C₃ alkyl,
**R⁴** is C₁-C₆ alkyl;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

More preferably, compounds of Formula **(Ic)** is wherein
Y is 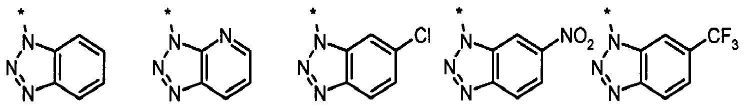 or 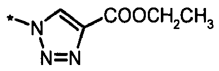 **PG1** is dicyclopropylmethyl or 2,4-dimethoxybenzyl, and
**PG2** is tert-Butoxycarbonyl (BOC) or Triphenylmethyl (trityl).

In a **fourth** embodiment, the invention is directed to compounds of Formula I 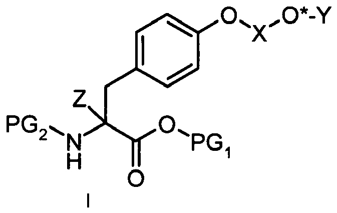 wherein:
- **X**: is CD₂;
- **Y**: is a substituted or unsubstituted heteroaromatic ring containing one to four Nitrogen atoms (N) with the proviso that the oxygen (O*) is directly bound to one of the Nitrogen atoms (N) of the heteroaromatic ring and **O*-Y** acts as leaving group;
- **Z**: is methyl;
- **PG1**: is a carboxylic acid protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula I and is also meant to comprise single isomers, diastereomers, enantiomers and mixtures thereof of Formula **I**.

Formula (**Id**) ) corresponds to to the Markush Formula below

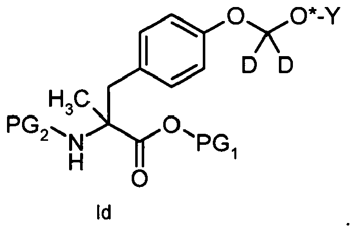

Preferred features disclosed above in respect of **Y, PG1** and **PG2** are incorporated herein.

Preferably, the invention is directed to compounds of Formula **(Id)** wherein
**Y** is a moiety of Formula **II** 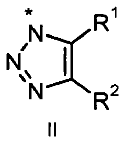 wherein
* indicates the position of the covalent bond to the Oxygen (O*) in Formula **Id;**
**R¹** is H, CN, or COO**R⁴**, and **R²** is H, CN, or COO**R⁴**, or
**R¹** and **R²** form together a 6 membered aromatic ring, which optionally comprise 1 Nitrogen atoms (N) and 1 methine of the 6 membered ring is optionally substituted with halogen, NO₂, CN, COO**R³**, SO₂**R³** or CF₃,
**R³** is C₁-C₃ alkyl,
**R⁴** is C₁-C₆ alkyl;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

More preferably, compounds of Formula **(Id)** is wherein
**Y** is 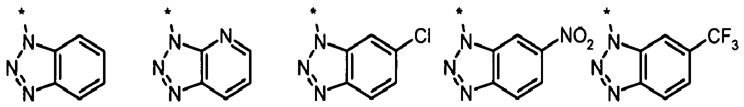 or 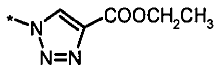 **PG1** is dicyclopropylmethyl or 2,4-dimethoxybenzyl, and
**PG2** is tert-Butoxycarbonyl (BOC) or Triphenylmethyl (trityl).

In a **fifth** embodiment, the invention is directed to compounds of Formula **(D-I), (D-Ia), (D-Ib), (D-Ic)** or **(D-Id)** wherein

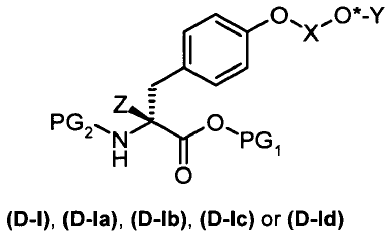

| **Formula\Substituent** | **Z** | **X** |
|---|---|---|
| **D-I** | H, CH₃ | CH₂,CD₂ |
| **D-la** | H | CH₂ |
| **D-Ib** | H | CD₂ |
| **D-Ic** | CH₃ | CH₂ |
| **D-Id** | CH₃ | CD₂ |

**Y, PG1** and **PG2** are disclosed as above and encompass preferred features as disclosed above.

The invention further refers to suitable salts of inorganic or organic acids, hydrates and solvates of the compounds of Formula **(D-I), (D-Ia)**, **(D-Ib), (D-Ic)** or **(D-Id)** and is also meant to comprise single isomers, diastereomers, enantiomers and mixtures thereof of Formula **(D-I), (D-Ia), (D-Ib), (D-Ic)** or **(D-Id).**

Embodiments and preferred features can be combined together and are within the scope of the invention.

Invention compounds are but not limited to
*tert*-Butyl *O*-[(1*H*-benzotriazol-1-yloxy)methyl]-*N*-(*tert*-butoxycarbonyl)-D-tyrosinate 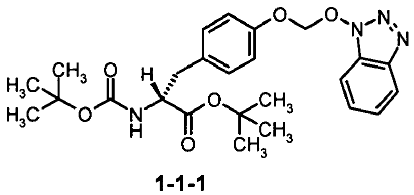
*tert*-Butyl *N*-(*tert*-butoxycarbonyl)-*O*-[(1*H*-1,2,3-triazolo[5,4-*b*]pyridin-1-yloxy)methyl] -D-tyrosinate 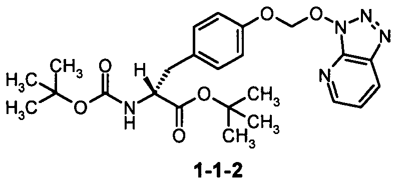
Dicyclopropylmethyl *O*-[(1*H*-benzotriazol-1-yloxy)methyl]-*N*-(*tert*-butoxycarbonyl) -D-tyrosinate 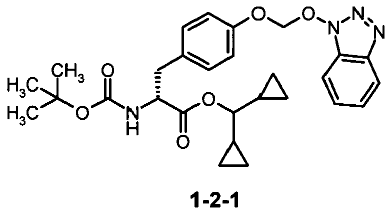
Dicyclopropylmethyl *O*-[(1*H*-benzotriazol-1-yloxy)methyl]-*N*-(*tert*-butoxycarbonyl) -L-tyrosinate 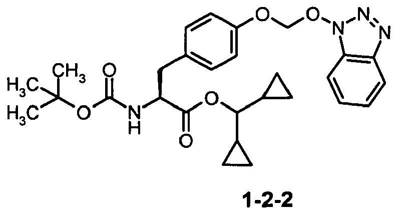
Dicyclopropylmethyl N-(*tert*-butoxycarbonyl)-O-[(6-nitro-1*H*-benzotriazol -1-yloxy)methyl]-D-tyrosinate 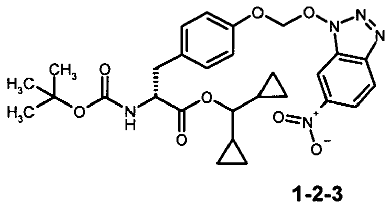
2,4-Dimethoxybenzyl *O*-[(1*H*-benzotriazol-1-yloxy)methyl]-*N*-(*tert*-butoxycarbonyl) -D-tyrosinate 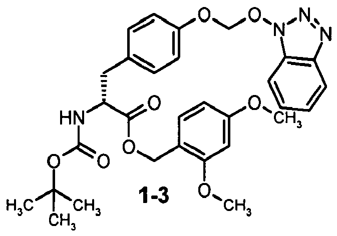
Cyclopropylmethyl *O*-[(1*H*-benzotriazol-1-yloxy)methyl]-*N*-(*tert*-butoxycarbonyl) -D-tyrosinate 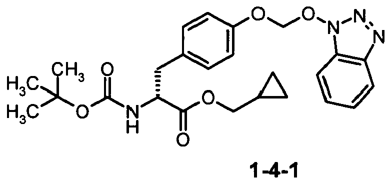
Cyclopropylmethyl *N*-(*tert*-butoxycarbonyl)-*O*-({[4-(ethoxycarbonyl)-1*H-*1,2,3-triazol-1-yl]oxy}methyl)-D-tyrosinate 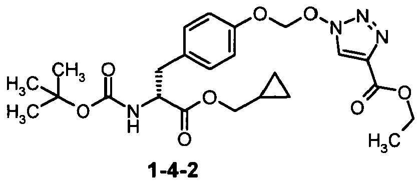
4-Methoxybenzyl *O*-[(1*H*-benzotriazol-1-yloxy)methyl]-*N*-(*tert*-butoxycarbonyl) -D-tyrosinate 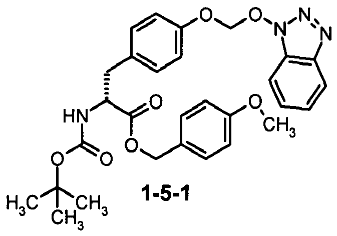
4-Methoxybenzyl *N*-(*tert*-butoxycarbonyl)-*O*-{[(6-chloro-1*H*-benzotriazol-1-yl) oxy]methyl}-D-tyrosinate 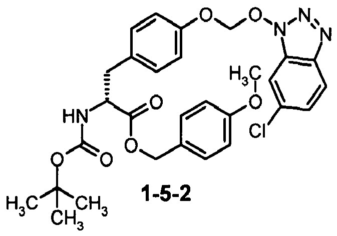
4-Methoxybenzyl *N*-(*tert*-butoxycarbonyl)-*O*-[(6-trifluoromethyl-1*H*-benzotriazol -1-yloxy)methyl]- D-tyrosinate 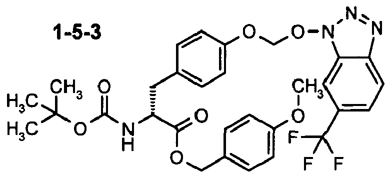
4-Methoxybenzyl *O*-[(6-trifluoromethyl-1*H*-benzotriazol-1-yloxy)methyl]-*N*-(*tert-*butoxycarbonyl)-L-tyrosinate. 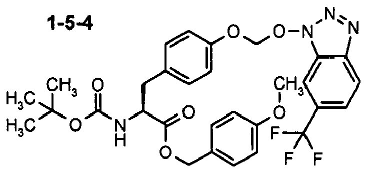
*alpha*-Methylbenzyl *O*-[(1*H*-benzotriazol-1-yloxy)methyl]-*N*-(*tert*-butoxycarbonyl) -D-tyrosinate 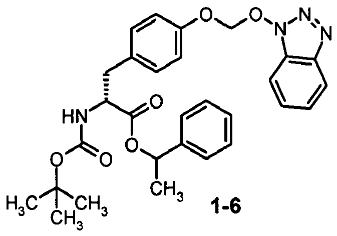
alpha,alpha-Dimethylbenzyl *O*-[(1*H*-benzotriazol-1-yloxy)methyl]-*N*-(*tert*-butoxycarbonyl) -D-tyrosinate 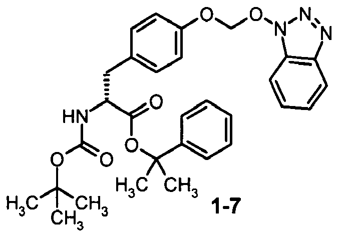
tert-Butyl *O*-[(1*H*-benzotriazol-1-yloxy)methyl]-*N*-trityl-D-tyrosinate 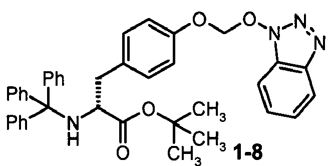
4-Methoxybenzyl *O*-[(1*H*-benzotriazol-1-yloxy)methyl]-*N*-trityl-D-tyrosinate 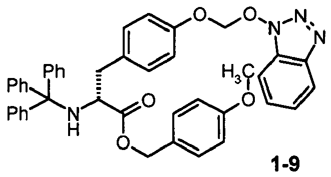
Cyclopropylmethyl *O*-[(1*H*-benzotriazol-1-yloxy)[²H₂]methyl]-*N*-(*tert*-butoxycarbonyl)-D-tyrosinate 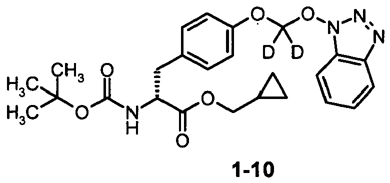
2,4-Dimethoxybenzyl *O*-[(1*H*-benzotriazol-1-yloxy)methyl]-*N*-trityl-D-tyrosinate 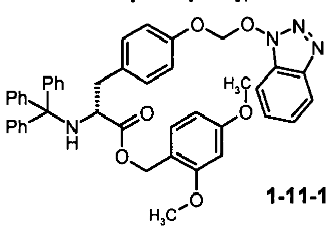
2,4-Dimethoxybenzyl *O*-{[(6-chloro-1*H*-benzotriazol-1-yl)oxy]methyl}-*N*-trityl-D-tyrosinate 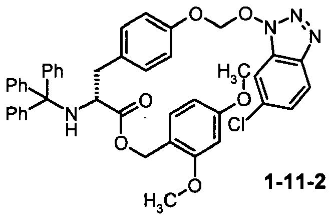
2,4-Dimethoxybenzyl *O*-{[(6-trifluoromethyl-1*H*-benzotriazol-1-yl)oxy]methyl}-*N*-trityl-D-tyrosinate 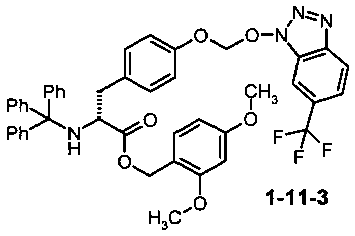
Methyl *O*-[(1*H*-benzotriazol-1-yloxy)methyl]-*N*-(*tert*-butoxycarbonyl)-*alpha-*methyltyrosinate

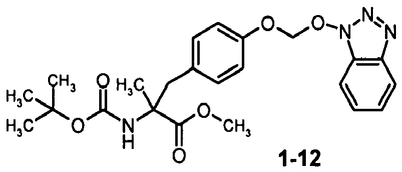

In a **second aspect,** the present invention is directed to compounds of Formula III 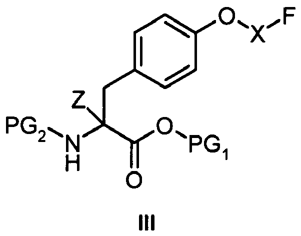 wherein:
- **X**: is CH₂, CHD or CD₂;
- **F**: is ¹⁸F or ¹⁹F;
- **Z**: is Hydrogen or methyl;
- **PG1**: is a carboxylic protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one or two halogens.

The invention further refers to suitable salts of inorganic or organic acids, hydrates and solvates of the compounds of Formula **III** and is also meant to comprise single isomers, diastereomers, enantiomers and mixtures thereof of Formula **III.**

### Preferred features:

**PG1** is a carboxylic acid protecting group (forming an ester) containing up to 20 carbon atoms, optionally containing independently one ore more O, N or S atoms; and compatible with radiolabeling conditions.

Preferably, **PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or
fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen.

Preferably, branched or linear C₁-C₆ alkyl is a C₁-C₃ alkyl. More preferably, C₁-C₆ alkyl is C₁-alkyl (methyl) when substituted and C₄-alkyl (e.g.tert-Butyl) when unsubstituted.

Preferably, branched or linear C₁-C₆ alkyl substituted with one phenyl is branched or linear C₁-C₃ alkyl substituted with one phenyl. More preferably, branched or linear C₁-C₆ alkyl substituted with one phenyl is methyl-phenyl (benzyl), ethyl-phenyl or i-Propyl-phenyl (e.g. Cumyl) Preferably, methyl-phenyl (benzyl), ethyl-phenyl and i-Propyl-phenyl (e.g. Cumyl) are is substituted with up to two methoxy-groups.

Preferably, C₁-C₃ alkoxy is C₁ alkoxy (methoxy).

Preferably, branched or linear C₁-C₆ alkyl substituted with one or two C₃-C₆ cycloalkyl is branched or linear C₁-C₃ alkyl substituted with one or two cyclo-propyl.

Preferably, branched or linear C₁-C₆ alkyl substituted with one phenyl and one C₃-C₆ cycloalkyl is branched or linear C₁-C₃ alkyl substituted with one phenyl and one C₃-C₆ cycloalkyl wherein the C₃-C₆ cycloalkyl is preferably C₃ cycloalkyl (cyclo-propyl),
Fluorenylmethyl is

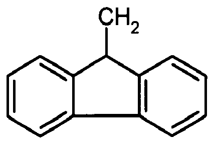

More preferably, **PG1** is
Alkyl,
Alkyl substituted with one phenyl,
Alkyl substituted with one or two C₃ cycloalkyl,
Alkyl substituted with one phenyl and one C₃ cycloalkyl, or fluorenylmethyl
wherein
Alkyl is a branched or linear C₁-C₄ alkyl, and optionally substituted with C₁-C₃ alkoxy, and
Phenyl is optionally substituted with up to three C₁-C₃ alkyl, C₁-C₃ alkoxy or halogen.

Even more preferably, **PG1** is 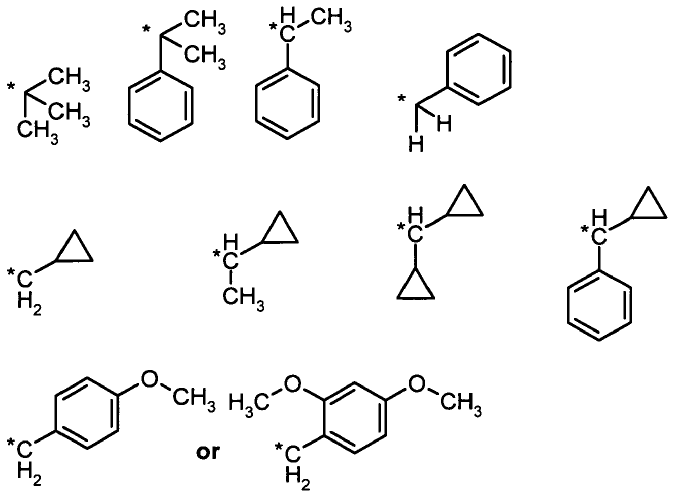 wherein * indicates the position of the Oxygen (O) forming the ester bond in Formula III; Even more preferably, **PG1** is 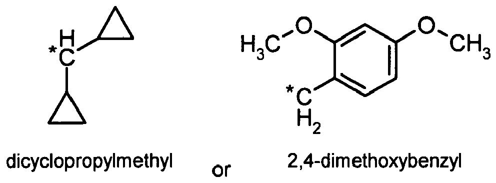 wherein * indicates the position of the Oxygen (O) forming the ester bond in Formula **III.**

**PG2** is an amino protecting group containing up to 20 carbon atoms, optionally containing one ore more O, N or S atoms and are optionally substituted with one to three halogens, and compatible with radiolabeling conditions.

Preferably, PG2 is a carbamate or an arylalkyl protecting group.

More preferably PG2 is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

Even more preferably, PG2 is tert-Butoxycarbonyl (BOC) or Triphenylmethyl (trityl).

Preferably, **F** is ¹⁸F.

Preferably, **F** is ¹⁹F.

Preferably, **Z** is Hydrogen.

### Preferred compounds of Formula III:

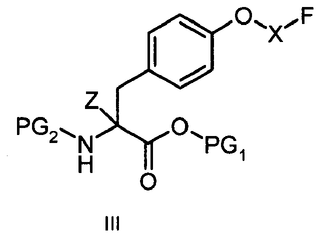

wherein:
- **X**: is CH₂ or CD₂;
- **F**: is ¹⁸F;
- **Z**: is Hydrogen or methyl;
- **PG1**: is dicyclopropylmethyl or 2,4-dimethoxybenzyl; and
- **PG2**: is tert-Butoxycarbonyl (BOC) or Triphenylmethyl (trityl).

In a first embodiment, the invention is directed to compounds of Formula III 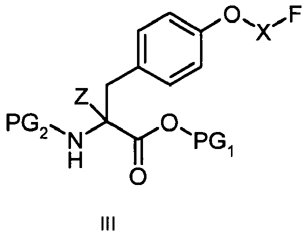 wherein:
- **X**: is CH₂;
- **F**: is ¹⁸F or ¹⁹F;
- **Z**: is Hydrogen;
- **PG1**: is a carboxylic acid protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula **III** and is also meant to comprise single isomers, diastereomers, enantiomers and mixtures thereof of Formula **III.**

Formula **(IIIa)** corresponds to the Markush Formula below

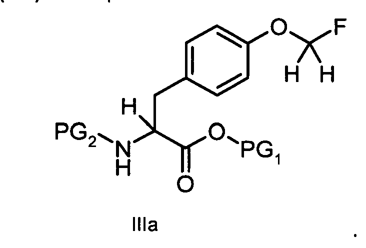

Preferred features disclosed above in respect of **F, PG1** and **PG2** are incorporated herein.

Preferably, the invention is directed to compounds of Formula **(IIIa)** wherein
**F** is ¹⁸ F or ¹⁹F;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

More preferably, compounds of Formula **(IIIa)** is wherein
**F** is ¹⁸F;
**PG1** is dicyclopropylmethyl or 2,4-dimethoxybenzyl, and
**PG2** is tert-Butoxycarbonyl (BOC) or Triphenylmethyl (trityl).

In a second embodiment, the invention is directed to compounds of Formula III 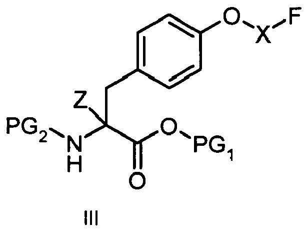 wherein:
- **X**: is CD₂ ;
- **F**: is ¹⁸F or ¹⁹F;
- **Z**: is Hydrogen;
- **PG1**: is a carboxylic acid protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula **III** and is also meant to comprise single isomers, diastereomers, enantiomers and mixtures thereof of Formula **III.**

Formula **(IIIb)** corresponds to the Markush Formula below

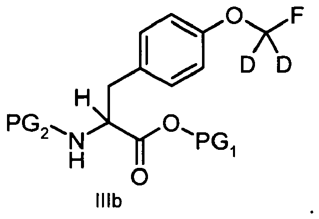

Preferred features disclosed above in respect of **F, PG1** and **PG2** are incorporated herein.

Preferably, the invention is directed to compounds of Formula **(IIIb)** wherein
**Y** is ¹⁸F or¹⁹F;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

More preferably, compounds of Formula (IIIb) is wherein
- **Y**: is ¹⁸F;
- **PG1**: is dicyclopropylmethyl or 2,4-dimethoxybenzyl, and
- **PG2**: is tert-Butoxycarbonyl (BOC) or Triphenylmethyl (trityl).

In a **third** embodiment, the invention is directed to compounds of Formula III 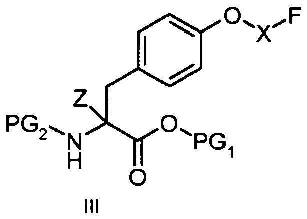 wherein:
- **X**: is CH₂ ;
- **F**: is ¹⁸F or ¹⁹F;
- **Z**: is methyl;
- **PG1**: is a carboxylic acid protecting group, containing up to 20 carbon atoms, optionally containing independently one ore more O, N or S atoms; and
- **PG2**: is an amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula **III** and is also meant to comprise single isomers, diastereomers, enantiomers and mixtures thereof of Formula **III.**

Formula (IIIc) corresponds to the Markush Formula below

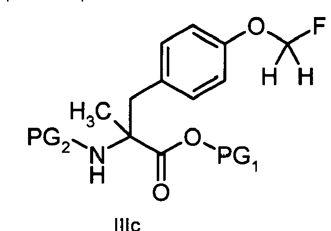

Preferred features disclosed above in respect of **F, PG1** and **PG2** are incorporated herein.

Preferably, the invention is directed to compounds of Formula (IIIc) wherein
**Y** is ¹⁸F or ¹⁹F;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

More preferably, compounds of Formula **(IIIc)** is wherein
**Y** is ¹⁸F;
**PG1** is dicyclopropylmethyl or 2,4-dimethoxybenzyl, and
**PG2** is tert-Butoxycarbonyl (BOC) or Triphenylmethyl (trityl).

In a **fourth** embodiment, the invention is directed to compounds of Formula **III** 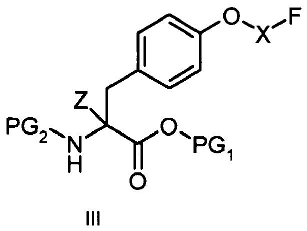 wherein:
- **X**: is CD₂ ;
- **F**: is ¹⁸F or ¹⁹F;
- **Z**: is methyl;
- **PG1**: is a suitable carboxylic acid protecting group, containing up to 20 carbon atoms, optionally containing independently one ore more O, N or S atoms; and
- **PG2**: is a suitable amino protecting group, containing up to 20 carbon atoms, optionally containing one ore more O, N or S atoms and are optionally substituted with one to three halogens.

The invention further refers to suitable salts of inorganic or organic acids, hydrates and solvates of the compounds of Formula **III** and is also meant to comprise single isomers, diastereomers, enantiomers and mixtures thereof of Formula **III.**

Formula **(IIId)** corresponds to the Markush Formula below

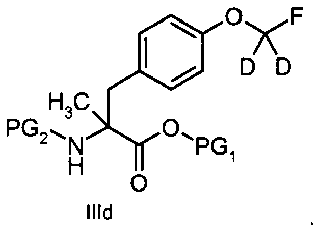

Preferred features disclosed above in respect of **F, PG1** and **PG2** are incorporated herein.

Preferably, the invention is directed to compounds of Formula **(IIId)** wherein
**Y** is ¹⁸F or ¹⁹F;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

Preferably, compounds of Formula **(IIId)** is wherein
**Y** is ¹⁸F;
**PG1** is dicyclopropylmethyl or 2,4-dimethoxybenzyl, and
**PG2** is tert-Butoxycarbonyl (BOC) or Triphenylmethyl (trityl).

In a **fifth** embodiment, the invention is directed to compounds of Formula **(D-III), (D-IIIa), (D-IIIb), (D-IIIc)** or **(D-IIId)** 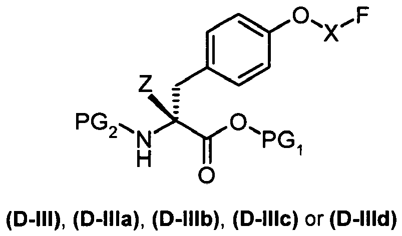 wherein

| **Formula\Substituent** | **Z** | **X** |
|---|---|---|
| **D-III** | H, CH₃ | CH₂ ,CD₂ |
| **D-IIIa** | H | CH₂ |
| **D-IIIb** | H | CD₂ |
| **D-IIIc** | CH₃ | CH₂ |
| **D-IIId** | CH₃ | CD₂ |

**F, PG1** and **PG2** are disclosed as above and encompass preferred features as disclosed above.

The invention further refers to suitable salts of inorganic or organic acids, hydrates and solvates of the compounds of Formula **(D-III), (D-IIIa), (D-IIIb), (D-IIIc)** or **(D-IIId)** and is also meant to comprise single isomers, diastereomers, enantiomers and mixtures thereof of Formula **(D-III), (D-IIIa), (D-IIIb), (D-IIIc)** or **(D-IIId).**

Embodiments and preferred features can be combined together and are within the scope of the invention.
¹⁹F-Invention compounds are but not limited to *tert*-Butyl *N*-(*tert*-butoxycarbonyl)-*O*-(fluoromethyl)-D-tyrosinate 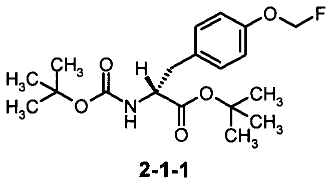
Dicyclopropylmethyl *N*-(*tert*-butoxycarbonyl)-*O*-(fluoromethyl)-D-tyrosinate 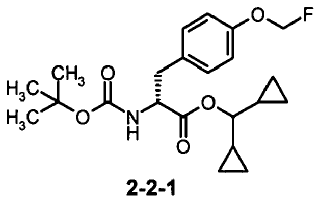
Dicyclopropylmethyl *N*-(*tert*-butoxycarbonyl)-*O*-(fluoromethyl)-L-tyrosinate 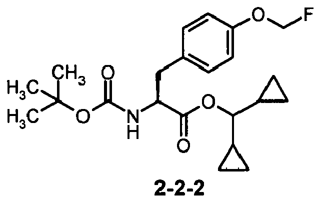
tert-Butyl *O*-(fluoromethyl)-*N*-trityl-D-tyrosinate 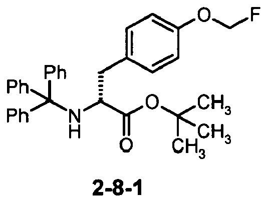
2,4-Dimethoxybenzyl *O*-(fluoromethyl)-*N*-trityl-D-tyrosinate 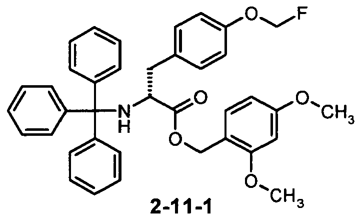
Methyl *N*-(*tert*-butoxycarbonyl)-*O*-(fluoromethyl)-*alpha*-methyl-D-tyrosinate 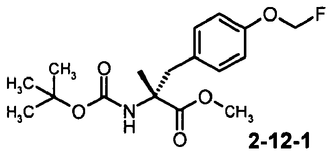
Methyl *N*-(*tert*-butoxycarbonyl)-*O*-(fluoromethyl)-alpha-methyl-L-tyrosinate 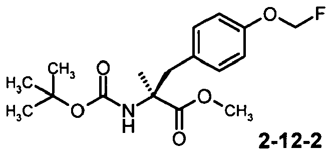
¹⁸F-Invention compounds are but not limited to *tert*-Butyl *N*-(*tert*-butoxycarbonyl)-O-([¹⁸F]fluoromethyl)-D-tyrosinate.
Labelling of 1-1-1 and 1-1-2
Dicyclopropylmethyl *N*-(*tert*-butoxycarbonyl)-*O*-([¹⁸F]fluoromethyl)-D-tyrosinate.
Labelling of 1-2-1 and 1-2-3
Dicyclopropylmethyl *N*-(*tert*-butoxycarbonyl)-O-([¹⁸F]fluoromethyl)-L-tyrosinate.
Labelling of 1-2-2
2,4-Dimethoxybenzyl *N*-(tert-butoxycarbonyl)-O-([¹⁸F]fluoromethyl)-D-tyrosinate.
Labelling of 1-3
Cyclopropylmethyl *N*-(tert-butoxycarbonyl)-O-([¹⁸F]fluoromethyl)-D-tyrosinate.
Labelling of 1-4-1 and 1-4-2
4-Methoxybenzyl *N*-(*tert*-butoxycarbonyl)-*O*-([¹⁸F]fluoromethyl)-D-tyrosinate.
Labelling of 1-5-1, 1-5-2 and 1-5-3
4-Methoxybenzyl *N*-(*tert*-butoxycarbonyl)-*O*-([¹⁸F]fluoromethyl)-L-tyrosinate.
Labelling of 1-5-4
*alpha*-Methylbenzyl *N*-(*tert*-butoxycarbonyl)-*O*-([¹⁸F]fluoromethyl)-D-tyrosinate.
Labelling of 1-6
*alpha,alpha*-Dimethylbenzyl *N*-(*tert*-butoxycarbonyl)-O-([¹⁸F]fluoromethyl)- D-tyrosinate.
Labelling of 1-7
*tert*-Butyl *O*-([¹⁸F]fluoromethyl)-*N*-trityl-D-tyrosinate.
Labelling of 1-8
4-Methoxybenzyl *O*-([¹⁸F]fluoromethyl)-*N*-trityl-D-tyrosinate.
Labelling of 1-9
Cyclopropylmethyl *N*-(tert-butoxycarbonyl)-*O*-([¹⁸F]fluoro[²H₂]methyl)-D-tyrosinate.
Labelling of 1-10
2,4-Dimethoxybenzyl *O*-([¹⁸F]fluoromethyl)-*N*-trityl-D-tyrosinate
Labelling of 1-11-1, 1-11-2 and 1-11-3
Methyl *N*-(tert-butoxycarbonyl)-*O*-([¹⁸F]fluoromethyl)-alpha-methyl-DL-tyrosinate.
Labelling of 1-12

In a **third aspect**, the present invention is directed to compositions comprising compound(s) of the Formula **III, IIIa, IIIb, IIIc, IIId, (D-III), (D-IIIa), (D-IIIb), (D-IIIc)** or **(D-IIId)** independently or mixtures thereof and reagents suitable for deprotection of the amino group and the ester function of the tyrosine, as exemplified in Greene, Wuts, Protecting Groups in Organic synthesis (third edition 1999 and Fourth Edition, Wiley 2007*).*

The person skilled in the art is familiar with auxiliaries, vehicles, excipients, diluents, carriers or adjuvants which are suitable for the desired deprotecting reaction leading to the unprotected fluoromethyl-tyrosines on account of his/her expert knowledge.

In a **fourth aspect,** the present invention is directed to compositions comprising compound(s) of the Formula **I, la, Ib, Ic, Id, (D-I), (D-Ia), (D-Ib), (D-Ic) or (D-Id)** independently or mixtures thereof and reagents suitable for fluoro labelling. The reagents, solvents and conditions which can be used for this fluorination are known to the person skilled in the field. See, *e.g.,* J. Fluorine Chem., 27 (1985):177-191.

In a **fifth aspect,** the present invention provides a kit comprising a sealed vial containing a predetermined quantity of a compound of Formula **I, Ia, Ib, Ic, Id, (D-I), (D-Ia), (D-Ib), (D-Ic)** or **(D-Id)** independently or mixtures thereof and suitable salts of inorganic or organic acids, hydrates and solvates. Optionally the kit comprises reagents for labelling, deprotection and a pharmaceutically acceptable carrier, diluent, excipient or adjuvant.

In a **sixth aspect,** the present invention is directed to methods for obtaining compounds of Formula I.

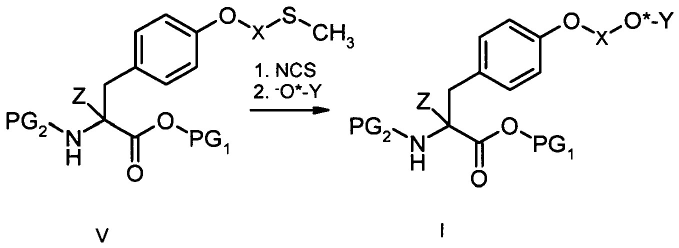

The methods for obtaining compounds of Formula **I** comprises the step of
- Reacting compound of Formula **V** first with N-Chloro-succinimide (NCS) and then with anion of H-O*-Y for obtaining compounds of Formula **I**,
wherein 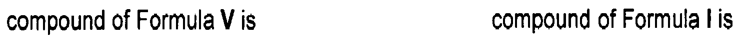 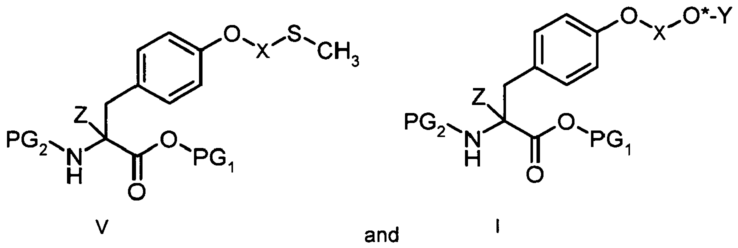 wherein Z, **PG1, PG2, X,** and **Y** are as defined above in first aspect.

Optionally, the method step is preceded by alkylation of a compound of Formula IV with Cl-X-SCH₃ for obtaining intermediate of Formula **V,** 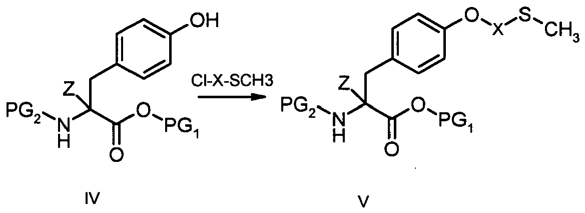 wherein **Z, PG1, PG2,** and **X** are as defined above in first aspect.

Preferred features disclosed above in respect of **Z, PG1, PG2 , X,** and **Y** are incorporated herein.

Preferably, the method for obtaining compounds of Formula I is defined as such that
- **X**: is CH₂ or CD₂;
- **Y**: is 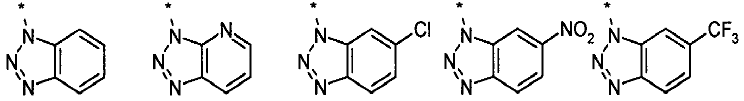
**or** 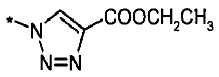 **Z** is Hydrogen or methyl; and
**PG1** is dicyclopropylmethyl or 2,4-dimethoxybenzyl, and
**PG2** is tert-Butoxycarbonyl (BOC) or Triphenylmethyl (trityl).

In a **seventh aspect,** the present invention is directed to a method for obtaining compounds of Formula **III.** 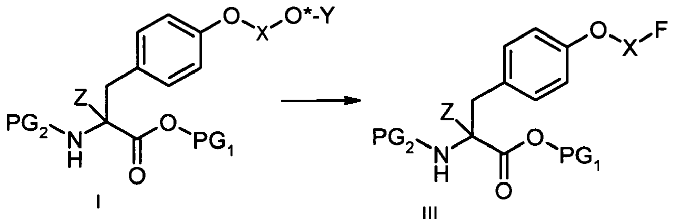 The method for obtaining compounds of Formula **III** comprises the step of
- Reacting compound of Formula **I with** a ¹⁸F-Fluorination agent, and
- [Optionally] converting obtained compound into a suitable salts of inorganic or organic bases thereof, hydrates, complexes, and solvates thereof
wherein 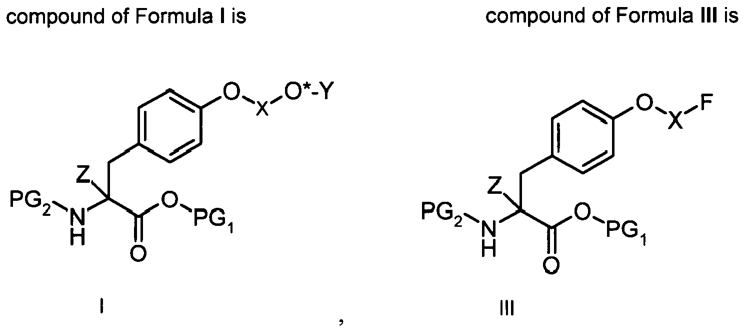 and **F, Z, PG1, PG2 , X,** and **Y are** as defined above in first aspect.

Preferred features disclosed above in respect of **F, Z, PG1, PG2 , X,** and **Y** are incorporated herein.

Preferably, the method for obtaining compounds of Formula III is defined as such that
- **X**: is CH₂ or CD₂;
- **Y**: is 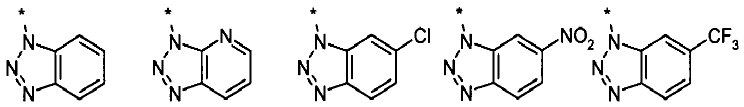
**or** 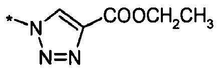
- **Z**: is Hydrogen or methyl;
- **F**: is ¹⁸F;
- **PG1**: is dicyclopropylmethyl and 2,4-dimethoxybenzyl; and
- **PG2**: is tert-Butoxycarbonyl (BOC) or Triphenylmethyl (trityl).

The ¹⁸F-Fluorination agent can be K¹⁸F, H¹⁸F, Rb¹⁸F, Cs¹⁸F, Na¹⁸F.

Optionally, the ¹⁸F-Fluorination agent comprises a chelating agent such as a cryptand (e.g.: 4,7,13,16,21,24-Hexaoxa-1,10-diazabicyclo[8.8.8]-hexacosane - Kryptofix®) or a crown ether (e.g.: 18-crown-6).

The ¹⁸F-Fluorination agent can also be a tetraalkylammonium salt of ¹⁸F⁻ or a tetraalkylphosphonium salt of ¹⁸F⁻, known to those skilled in the art, e.g.: tetrabutylammonium [¹⁸F]fluoride, tetrabutylphosphonium [¹⁸F]fluoride.

Preferably, the ¹⁸F-Fluorination agent is Cs¹⁸F, K¹⁸F, tetrabutylammonium [¹⁸F]fluoride.

The reagents, solvents and conditions which can be used for this fluorination are common and well-known to the skilled person in the field. See, *e.g.,* J. Fluorine Chem., 27 (1985):177-191; Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). Preferably, the solvents used in the present method are DMF, DMSO, acetonitrile, DMA, or mixtures thereof, preferably the solvent is acetonitrile, DMSO.

In a **eighth aspect,** the present invention is directed to a method for obtaining compounds of Formula **VI.**

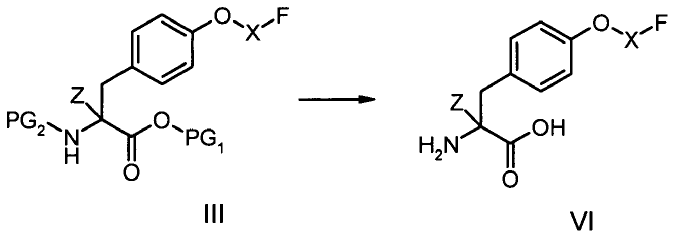

The method for obtaining compounds of Formula **VI** comprises the step of
- deprotecting the compound of Formula **III** for obtaining deprotected compound of Formula **VI,** and
- [Optionally] converting obtained compound into a suitable salts of inorganic or organic bases thereof, hydrates, complexes, and solvates thereof
wherein 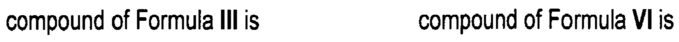 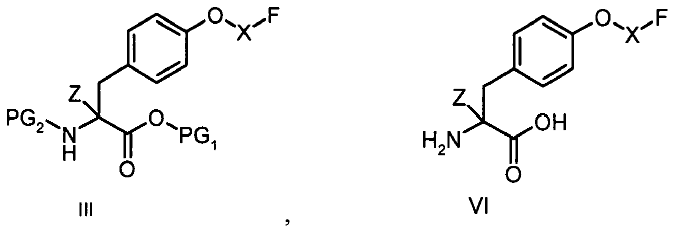 and **F, Z, PG1, PG2 , X,** and **Y are** as defined above in first and second aspects.

Preferred features disclosed above in respect of **F, Z, PG1, PG2 , X,** and **Y** are incorporated herein.

Deprotecting means removing the protecting groups PG1 and PG2. Deprotecting occurs under acid conditions wherein preferably the acid is HCl in organic- or aqueous- solvents or TFA with or without additives.

Preferably, the method for obtaining compounds of Formula VI is defined as such that
- **X**: is CH₂ or CD₂;
- **Z**: is Hydrogen or methyl;
- **F**: is ¹⁸F;
- **PG1**: is dicyclopropylmethyl or 2,4-dimethoxybenzyl; and
- **PG2**: is tert-Butoxycarbonyl (BOC) or Triphenylmethyl (trityl).

### Detailed description of the synthesis of compound of Formula Ia

### 1. N-protected tyrosine esters

N-protected tyrosines can be directly esterified by alkylation of the carboxylic acid without protection of the phenol function (e.g. Jung M.E. Tetrahedron 1997, 8815). The reaction of the caesium salts of N-protected tyrosines with suitable alkylation agents also gives the protected tyrosine esters.

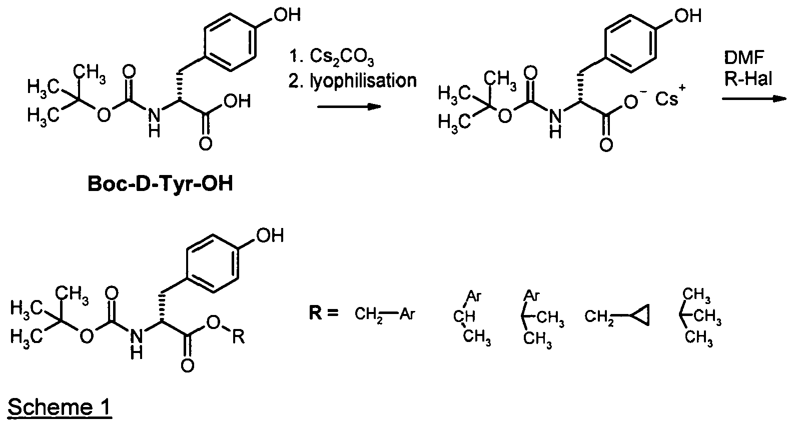

Alternatively, a tyrosine ester can be reacted with a dialkyldicarbonate to introduce a carbamate or a trityl-group as N-protection.

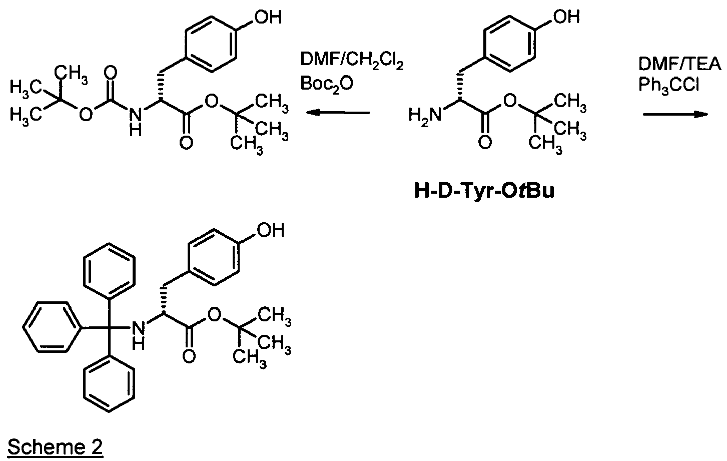

Finally, if no suitable alkylating agent is available or the tyrosine ester is not easily available, direct esterification methods can be employed using the corresponding alkohol. This is exemplary demonstrated in the synthesis of the dicyclopropylmethylester. In such case it is advisable to protect the phenol prior to esterification as shown in scheme 3.

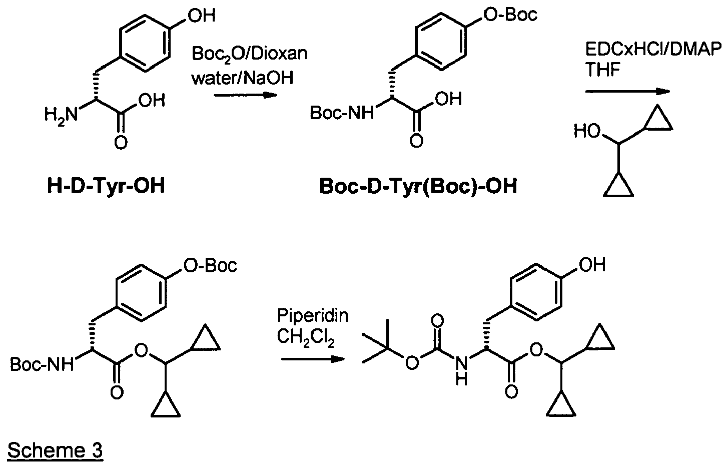

D-Tyrosine is bisbocylated according to Pozdnev, V. F.; Chemistry of Natural Compounds; English; 18; 1; 1982; 125 - 126 and then be esterified with a standard DMAP/Carbodiimide coupling method. Selective deprotection is done according to Nakamura K., Tetrahedron Lett. 2004, 495.

These examples show, that a wide choice of protected tyrosines can be easily synthesized.

### 2. Methylthio methyl ethers of N-protected tyrosine esters

The phenol group of the protected tyrosine esters is converted into a methylthiomethylether by alkylation with methylthiomethylchloride in a DMF/THF mixture using potassium-tert.-butylate as base and sodium iodide to enhance the reactivity of the alkylation agent.

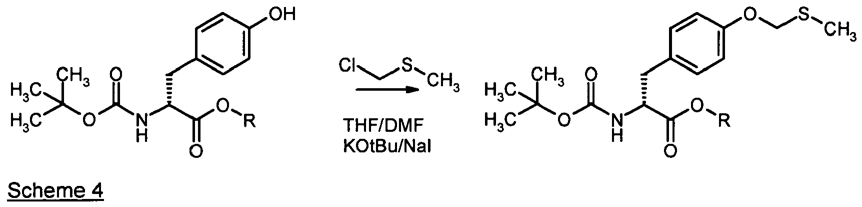

To a person skilled in the art it is obvious, that other chlorothiomethyl ethers can be employed, like for example 1-[(chloromethyl)sulfanyl]-4-methylbenzene or 1-[(chloromethyl)sulfanyl]-4-chlorobenzene.

### 3. Conversion of the methylthiomethylethers into compounds of Formula I

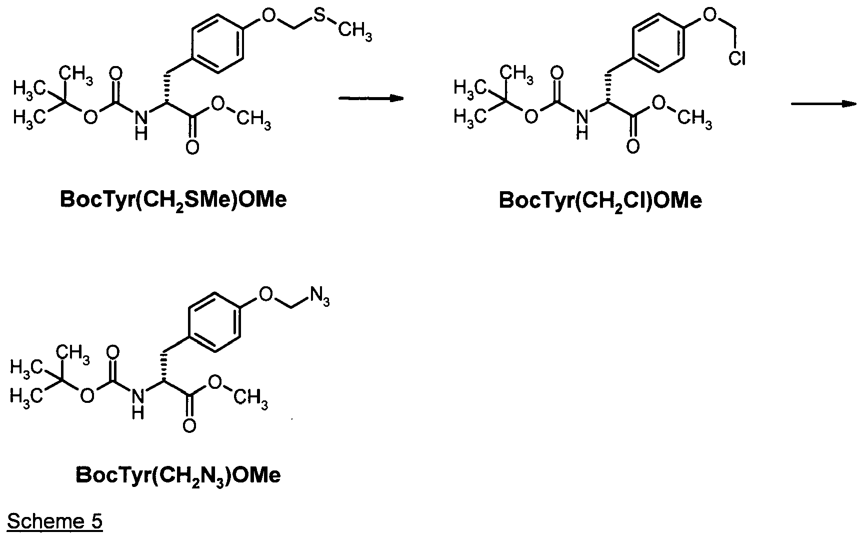

The basic strategy has been described in Angew. Chem. Int. Ed. 2002, 3449 for the synthesis of BocTyr(azidomethyl)OMe. The chloromethylether can be made in 73% using N-chlorosuccinimide and trimethylsilylchloride as activation agent in dichloromethane. The compound could be isolated although some hydrolysis was reported. When this reaction was conducted with the more acid labile tert-butyl ester, the yield dropped to 24% and with the even more labile dicyclopropylmethyl ester, the chloromethylether could not be isolated. It proved advantageous not to isolate this labile intermediate. In an improved protocol, no activation is employed and the reaction mixture is immediately after workup reacted with the ON-nucleophile in an anhydrous environment.

The chloromethyl ether can also be obtained by reaction with sulfuryl chloride in dichloromethane at 0 °C as described in Journal of Medicinal Chemistry, 2005, Vol. 48, No. 10, 3586-3604*.*

For the reaction with the chloro methylether thus generated, it is advantageous to employ the ON-nucleophiles like HOBt in anhydrous form. However, HOBt is commercial supplied only as the hydrate. Anhydrous salts of HOBt are not commercially available as well. It was found however, that tetrabutylammonium OBt can easily be prepared in anhydrous form. Commercial HOBt hydrate was dissolved in anhydrous tetrabutyl-ammoniumhydroxide (Commercial 1M in MeOH) and the solvent evaporated to give a yellow solid. This was stripped twice with toluene to give anhydrous Bu₄NOBt. This compound can safely be dried at 40°C. This method can be used for all O-N-nucleophiles described in this patent. Alternatively, KOBt can be made by reaction of HOBt*H₂O with KOH in Methanol and dried by stripping with toluene and evaporation in the vacuum at 40 °C.

Reaction of the raw mixture from the reaction of the methylthiomethylether with N-chlorosuccinimide with the tetrabutylammonium salt of the O-N-nucleophile, gives the inventive compounds of Formula I.

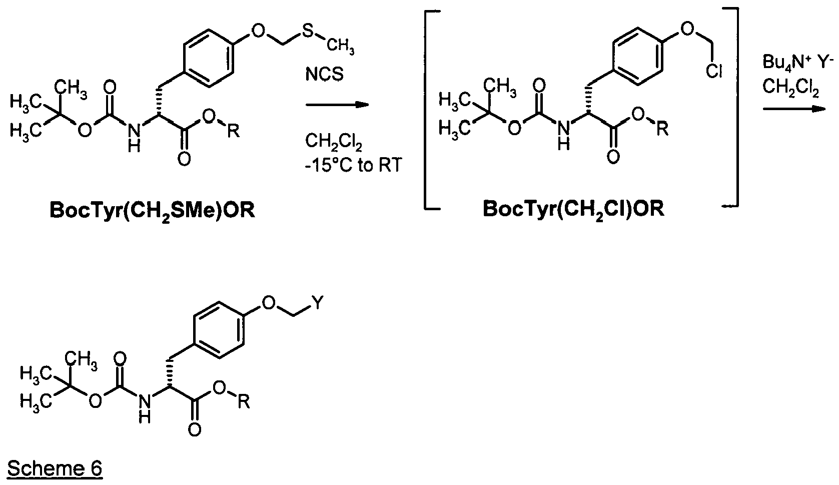

It is understood, that this method is also applicable to tyrosine esters with other N-protection.

### Detailed description of the synthesis of compound of Formula Ib

For the synthesis of deuterated compounds Ib a slightly different synthesis route is employed (Scheme 7) which has the advantage, that perdeuterated dimethylsulfoxide is a readily available reagent. It is reacted according to a published procedure (J. Chem. Soc. Perkin I, 1983, 1141-44) with the protected tyrosine to give the deuterated methylthiomethylether. The last reaction step is the same as described above.

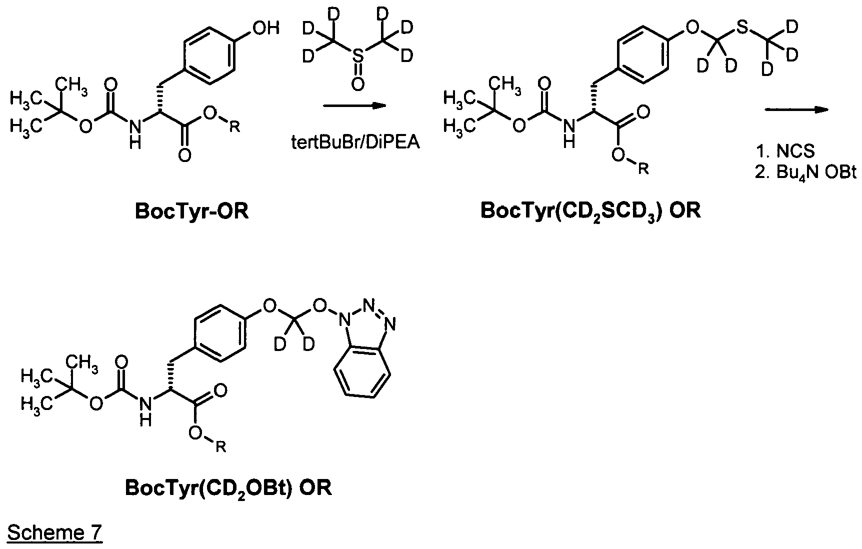

### Detailed description of the synthesis of compound of Formula Ic

Synthesis of the αMethyl tyrosine derivatives Ic and Id is done by combination of the methods descibed for the synthesis of la and Ib respectively. For synthesis of the compounds Ic commercially available racemic α Methyl-tyrosine-methylester is used as starting material (Scheme 8). Bocylation proceeds as described (J. Med. Chem. 2004, 47, 1223-33). Alkylation with CICH₂SCH₃ is done as described above. The benzotriazolyl-methylether can be obtained by the same reaction as described for compounds la above.

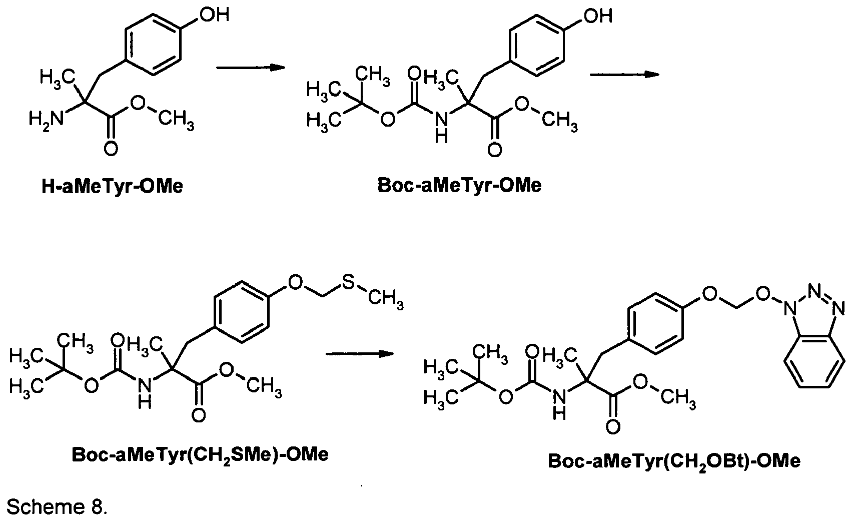

### Detailed description of the synthesis of compound of Formula Id

The compounds of Formula Id can be obtained by combination of the methods depicted in Schemes 7 and 8.

### Detailed description of the synthesis of compound of Formula III

Synthesis of the [¹⁹F]-fluoromethylethers IIIa or IIIc is usually done by reaction of the N-protected tyrosine ester with Bromofluoromethane as shown in Scheme 9 for IIIa.

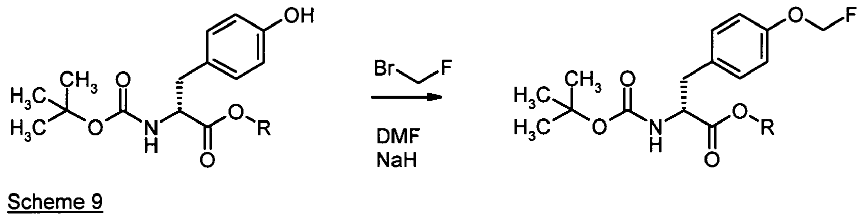

Compounds of Formula IIIb and IIId could be synthesized in analogy using Bromo-fluoro[²_{H2}]methane. In our experimental work, the compounds IIIa and IIIc were used as cold reference in the radiosynthesis of [¹⁸F]-IIIb and [¹⁸F]-IIId.

The radiosynthesis of the [¹⁸F]-fluoromethylethers III can be carried out in a two-step process using a reactive intermediate synthon, e.g. [¹⁸F]Fluoromethyl bromide (Iwata et al., Appl. Radiat. Isot., 2002, 57, 347-352), [¹⁸F]Fluoromethyl iodide (Zhang et al., J. Med. Chem., 2004, 47, 2228-2235, Zhang et al., J. Fluorine Chem., 2004, 125, 1879-1886), [¹⁸F]Fluoromethyl tosylate (Neal et al., J. Label. Compd. Radiopharm., 2005, 48, 557-568), [¹⁸F]Fluoromethyl triflate (Iwata et al., Appl. Radiat. Isot., 2002, 57, 347-352) or [¹⁸F]Fluoromethyl mesylate (Neal et al., J. Label. Compd. Radiopharm., 2005, 48, 557-568), and reacting it with the a hydroxyl functional under basic conditions. These methods are known to those skilled in the art. The reactions can be carried out, for example in a typical reaction vessel (e.g. Wheaton vial) which is known to someone skilled in the art or in a microreactor. The reaction can be heated by typical methods, e.g. oil bath, heating block or microwave. The radiofluorination reactions are carried out in acetonitrile with potassium carbonate as base and "kryptofix" as crown-ether. But also other solvents can be used which are well known to experts. These possible conditions include, but are not limited to: dimethyl sulfoxide and dimethylformamide as solvent and tetraalkyl ammonium and tetraalkyl phosphonium carbonate as base. Water and/or alcohol can be involved in such a reaction as co-solvent. The radiofluorination reactions are conducted for one to 60 minutes. Preferred reaction times are five to 50 minutes. Further preferred reaction times are 10 to 40 min. This and other conditions for such radiofluorination are known to experts *(*Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50*;* Ametamey et al., Chem. Rev., 2008, 108, 1501-1516*).* The radiofluorination can be carried out in a "hot-cell" and/or by use of a module (review: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated or semi-automated synthesis.

### Definitions

### "D" means Deuterinium.

As used hereinafter in the description of the invention and in the claims, the terms "salts of inorganic or organic acids", "inorganic acid" and "organic acid" refer to mineral acids, including, but not being limited to: acids such as carbonic, nitric, phosphoric, hydrochloric, perchloric or sulfuric acid or the acidic salts thereof such as potassium hydrogen sulphate, or to appropriate organic acids which include, but are not limited to: acids such as carboxylic and sulfonic acids, examples of which are trifluoracetic, methansulfonic, ethanesulfonic, benzenesulfonic, toluenesulfonic and trifluormethanesulfonic acid, respectively.

If chiral centers or other forms of isomeric centers are present in a compound according to the present invention, all forms of such stereoisomers, including enantiomers and diastereoisomers, are intended to be covered herein. Compounds containing chiral centers may be used as racemic mixture or as an enantiomerically enriched mixture or as a diastereomeric mixture or as a diastereomerically enriched mixture, or these isomeric mixtures may be separated using well-known techniques, and an individual stereoisomer maybe used alone. In cases wherein compounds may exist in tautomeric forms, such as keto-enol tautomers, each tautomeric form is contemplated as being included within this invention whether existing in equilibrium or predominantly in one form.

The term "amine-protecting group" as employed herein by itself or as part of another group is known or obvious to someone skilled in the art, which is chosen from but not limited to a class of protecting groups namely carbamates, amides, imides, N-alkyl amines, N-aryl amines, enamines, N-sulfonyl and and which is chosen from but not limited to those described in the textbook Greene and Wuts, Protecting groups in Organic Synthesis, third edition, (third edition 1999, page 494-653, which is hereby incorporated herein by reference.

Preferred amine protecting groups are carbamates (e.g. Boc) and Aralkyl (e.g. Trityl)

The term "carboxylic acid-protecting group" as employed herein refers to a protecting group employed to block or protect the carboxylic acid functionality while the reactions involving other functional sites of the compound are carried out. Carboxy protecting groups are disclosed in Greene, Wuts, Protective Groups in Organic Synthesis, third edition, 1999, page 372-453, which is hereby incorporated herein by reference. Such protecting groups are well known to those skilled in the art, having been extensively used in the protection of carboxylic acids. Representative carboxy protecting groups are alkyl (e.g., methyl, ethyl or tertiary butyl and the like); arylalkyl, for example, phenethyl or benzyl and substituted derivatives thereof such as alkoxybenzyl or nitrobenzyl groups and the like; alkylcycloalky (e.g. cyclopropylmethyl or dicyclopropylmethyl); alkoxyalkyl (e.g. methoxymethyl (MOM) or benzyloxymethyl (BOM).

Preferred O-protected compounds of the invention are compounds wherein the protected carboxy group is a lower alkyl (for example, methyl ester, ethyl ester, propyl ester, isopropyl ester, butyl ester, *sec*-butyl ester, isobutyl ester, *tert*. butylester, amyl ester, isoamyl ester), alkyl-cycloalkyl (for example cycloalkylmethyl, dicycloalkylmethyl, 1-cycloalkylethyl) or arylalkyl (for example, benzyl, 4-methoxybenzyl, 2,4-dimethoxybenzyl) ester.

The term "alkyl" as employed herein by itself or as part of another group refers to a C₁-C₆ straight chain or branched chain alkyl group such as, for example methyl, ethyl, propyl, isopropyl, butyl, isobutyl, tert-butyl, pentyl, isopentyl, and neopentyl. Preferably, alkyl is C₁-C₃ straight chain or branched chain alkyl or C₄-C₆ branched chain alkyl.

The term "cycloalkyl" as employed herein by itself or as part of another group refers to a C₃-C₆ cycloalkyl-group, i.e. cyclopropyl, cyclobutyl, cyclopentyl and cyclohexyl.

The term "aryl" as employed herein by itself or as part of another group refers to monocyclic or bicyclic aromatic rings comprising from 6 to 12 carbons, such as phenyl or naphthyl.

The term "heteroaryl" as employed herein by itself or as part of another group refers to monocyclic or bicyclic aromatic groups containing from 5 to 12 carbons in the ring and where up to 4 carbons are replaced by nitrogens in a way, that the resulting heteroaromatic system contains one N-H group. Typical examples are pyrrole, imidazole, triazole; their benzannelated analogs, indol, benzimidazoles, benzotriazoles, pyridyl fused analogs like azabenzotriazole and other fused systems like imidazopyrroles or imidazotriazoles.

The term "halide" (halogen) as employed herein by itself or as part of another group is known or obvious to someone skilled in the art, and means fluoro, chloro, bromo, and iodo.

As used hereinafter in the description of the invention and in the claims, the term "fluorine isotope" (F) refers to all isotopes of the fluorine atomic element unless explicitly otherwise indicated. Fluorine isotope (F) is selected from radioactive or non-radioactive isotope. The radioactive fluorine isotope is [¹⁸F] . The non-radioactive "cold" fluorine isotope is [¹⁹F].

The stereochemistry can be denoted in several ways. For the amino acids often D/L is used for the alpha-position referring to position of the residues when drawn in the Fischer-projection. Stereochemically D corresponds to the stereodescriptor "R" and L corresponds to the stereodescriptor "S" for all of the compounds of the invention.

Without further elaboration, it is believed that one skilled in the art can, using the preceeding description, utilize the present invention to its fullest extent. The following preferred specific embodiments are, therefore, to be construed as merely illustrative, and not limitative of the remainder of the disclosure in any way whatsoever.

The entire disclosure[s] of all applications, patents and publications, cited herein are incorporated by reference herein.

The following examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

### Experimental Part

### Abbreviations

| | |
|---|---|
| 18-c-6 | 1,4,7,10,13,16-hexaoxacyclooctadecane |
| br | broad signal (in NMR data) |
| CI | chemical ionisation |
| d | doublet |
| CH₂Cl₂ | dichloromethane |
| Cs₂CO₃ | cesium carbonate |
| DAD | diode array detector |
| dd | doublet of doublet |
| ddd | doublet of doublet of doublet |
| dt | doublet of triplet |
| DMF | *N,N*-dimethylformamide |
| DMSO | dimethylsulfoxide |
| EI | electron ionisation |
| ELSD | evaporative light scattering detector |
| ESI | electrospray ionisation |
| EtOAc | ethyl acetate |
| EtOH | ethanol |
| Fmoc | fluorenylmethyloxycarbonyl |
| HCOOH | Formic acid |
| HPLC | high performance liquid chromatography |
| GBq | Giga Bequerel |
| h | hour |
| K_{2.2.2} | 4, 7, 13, 16, 21, 24-hexaoxa-1,10-diazabicyclo[8.8.8]-hexacosane |
| K₂CO₃ | potassium carbonate |
| K₂HPO₄ | dipotassium phosphate |
| KOH | potassium hydroxide |
| MBq | Mega Bequerel |
| MeCN | acetonitrile |
| MeOH | methanol |
| MS | mass spectrometry |
| MTB | methyl *tert*-butyl ether |
| m | multiplet |
| mc | centred multiplet |
| min | minute |
| NaH | sodium hydride |
| NMR | nuclear magnetic resonance spectroscopy : chemical shifts (δ) are given in ppm. |
| q | quadruplett (quartet) |
| PMB | para-methoxybenzyl |
| PET | positron Emission Tomography |
| RT | room temperature |
| s | singlet |
| t | triplet |
| TBAOH | tetrabutylammonium hydroxide |
| TBS | *tert*-butyldimethyl silyl |
| THF | tetrahydrofuran |
| THP | tetrahydropyran |
| UPLC | ultra performance liquid chromatography |

The invention is demonstrated, but not limited to, by the following examples.

### 1. Synthesis of non radioactive compounds

First number denotes Precursor (1), that is compounds of Formula (I), cold standard (2), that is compounds of Formula (III) or intermediates (3), second number denotes example, third number to distinguish compounds within the example. (1-3-2 = second compound of Formula (I) in example 3)

### 1.1 Example 1

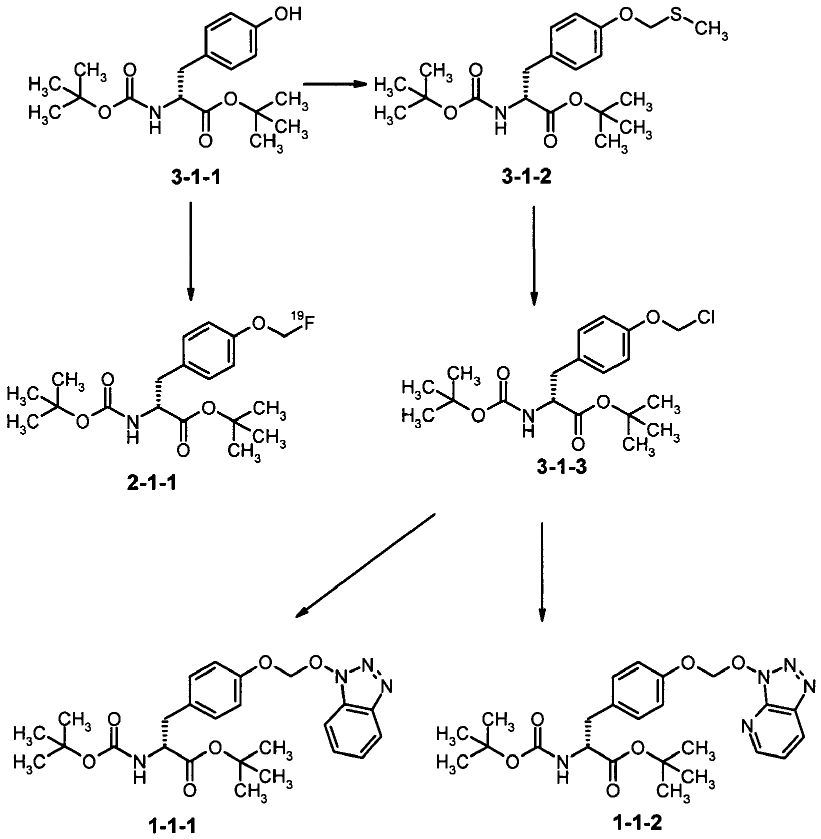

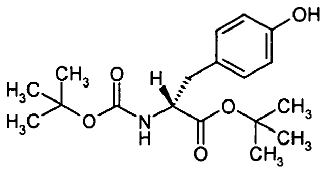

### tert-Butyl N-(tert-butoxycarbonyl)-D-tyrosinate. 3-1-1

To a stirred solution of *tert*-butyl D-tyrosinate (47.46 g, 200 mmol) in dichloromethane (600 ml) and *N,N*-dimethylformamide (60 ml) was added triethylamine (22 g, 220 mmol) and di-*tert*-butyl dicarbonate (43,65 g, 200 mmol). The mixture was stirred at r.t. for 2 h and then subsequently washed with aqueous 1 N hydrochloric acid (3x100 ml), saturated sodium hydrogen carbonate (100 ml), brine (100 ml), dried (magnesium sulfate) and concentrated to give **3-1-1** as a light yellow oil, which solidified on standing. Yield 64 g (95%).

MS (Cl, NH₃): m/z = 355 (M + NH₄), 388 (M + H), 399 (M + NH₄ - C₄H₈), 382 (M + H - C₄H₈), 238 (M + H - C₄H₈ - CO₂).

¹H-NMR (400 MHz, CD₂Cl₂): δ = 7.00 (d, J = 8.3 Hz, 2H, Ar), 6.74 (d, J = 8.1Hz, 2H, Ar), 5.31 (d, J = 8.1Hz, 1H, NH), 4.33 (mc, 1H, 2-H), 2.94 (mc, 2H, 3-H), 1.41 ppm (s, 18H, 1-tBu, 2-*t*Bu).

¹³C-NMR (100 MHz, CD₂Cl₂): δ = 27.40 (q, 1-C2), 27.70 (q, 2-C3), 37.38 (tb, C-3), 55.20 (d, C-2), 79.64 (sb, 2-C2), 81.93 (s, 1-C1), 115.18 (d, Ar-C3), 127.59 (s, Ar-C1), 130.52 (d, Ar-C2), 155.22 (s, Ar-C4), 155.59 (sb, 2-C1), 171.13 ppm (s, C1).

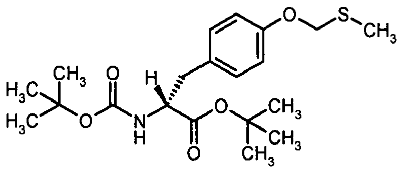

### tert-Butyl N-(tert-butoxycarbonyl)-O-[(methylsulfanyl)methyl]-D-tyrosinate. 3-1-2

A solution of **3-1-1** (4.62 g, 13.7 mmol) and sodium iodide (0.21 g, 1.4 mmol) in *N,N-*dimethylformamide (30 ml) was cooled in an ice water bath. A solution of potassium tert-butoxide (1.73 g , 15.4 mmol) in tetrahydrofuran (15 ml) was added. Then chloromethyl methyl sulfide (1.3 ml, 1.50 g, 15.8 mmol) was added. The mixture was stirred at r.t. for 3 h after which TLC showed complete conversion. Ethyl acetate (60 ml) was added and the mixture was washed with water (50 ml). The water layer was extracted with ethyl acetate (50 ml). The combined organic layers were consecutively washed with 10% citric acid, brine, dried (magnesium sulfate) and concentrated to give a thick yellow oil (smelly), 6.0 g. Purification on column chromatography (SiO₂, heptane/ ethyl acetate 3/1) gave pure **3-1-2** as an oil, 3.3 g (81 %).

The reaction was repeated with 23.1 g of **3-1-1** to give 19.6 g of **3-1-2** (72%)

MS (ES+): m/z = 420 (M + Na), 398 (M + H), 242 (M - 2 C₄H₈ - CO₂).

MS (CI, NH₃): m/z = 415 (M + NH₄), 398 (M + H), 359 (M + NH₄ - C₄H₈), 342 (M - C₄H₈), 303 (M + NH₄ - 2 C₄H₈).

¹H-NMR (400 MHz, CD₂Cl₂): δ = 7.08 (d, J = 8.6 Hz, 2H, Ar), 6.87 (d, J = 8.3Hz, 2H, Ar), 5.13 (s, 2H, O-CH₂-O), 4.98 (db, J = 6.8Hz, 1 H, NH), 4.34 (mc, 1H, 2-H), 2.98 (mc, 2H, 3-H), 2.15 (s, 3H, SCH₃), 1.41, 1.40 ppm (s, 18H, 1-tBu, 2-tBu).

¹³C-NMR (100 MHz, CD₂Cl₂): δ = 14.29 (q, SCH₃), 27.72 (q, 1-C2), 28.05 (q, 2-C3), 37.43 (tb, C-3), 55.08 (d, C-2), 77.48 (t, O-CH₂-S), 79.38 (sb, 2-C2), 81.85 (s, 1-C1), 115.81 (d, Ar-C3), 130.50 (d, Ar-C2), 130.61 (s, Ar-C1), 154.97 (sb, 2-C1), 154.97 (s, Ar-C4), 170.93 ppm (s, C1).

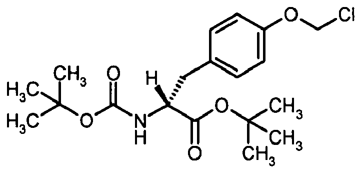

### tert-Butyl N-(tert-butoxycarbonyl)-O-(chloromethyl)-D-tyrosinate. 3-1-3

To a solution of **3-1-2** (18.2 g, 46 mmol) in dichloromethane (200 ml) at r.t. was added *N-*chlorosuccinimide (7.34 g, 55 mmol). After stirring for 10 min was added trimethylsilyl chloride (7.60 g, 70 mmol). The mixture was stirred at r.t. overnight. The mixture was consecutively washed with saturated sodium hydrogen carbonate, water, dried (magnesium sulfate) and concentrated to a yellow oil, 18 g (quantitative). Purification on column chromatography (450 g SiO₂, heptane/ ethyl acetate 3/1) gave **3-1-3** as light yellow oil (4.3 g, 24%).

¹H-NMR (400 MHz, CD₂Cl₂): δ = 7.17 (d, J = 8.6 Hz, 1H, Ar), 7.02 (d, J = 8.6Hz, 1H, Ar), 5.90 (s, 2H, O-CH₂-O), 4.98 (db, J = 6.6Hz, 1H, NH), 4.36 (mc, 1H, 2-H), 3.00 (mc, 2H, 3-H), 1.41, 1.40 ppm (s, 18H, 1-tBu, 2-tBu).

¹³C-NMR (100 MHz, CD₂Cl₂): δ = 27.71 (q, 1-C2), 28.03 (q, 2-C3), 37.53 (tb, C-3), 54.95 (d, C-2), 77.61 (t, O-CH₂-O), 79.41 (sb, 2-C2), 81.96 (s, 1-C1), 115.93 (d, Ar-C3), 130.78 (d, Ar-C2), 131.75 (s, Ar-C1), 154.48 (s, Ar-C4), 154.91 (sb, 2-C1), 170.80 ppm (s, C1).

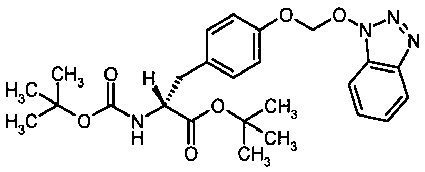

### tert-Butyl O-[(1H-benzotriazol-1-yloxy)methyl]-N-(tert-butoxycarbonyl)-D-tyrosinate. 1-1-1

To a stirred solution of 1*H*-benzotriazol-1-ol hydrate (1.0 g, 8.5 mmol, stripped free of water with toluene) in *N,N*-dimethylformamide (2 ml) and dichloromethane (20 ml) was added at r.t. a solution of **3-1-3** (1.0 g, 2.6 mmol) in dichloromethane (5 ml), followed by 4-(dimethylamino)pyridine (0.4 g, 3.2 mmol). The mixture was stirred at r.t. for 30 min after which time TLC indicated complete consumption of starting material. Water (50 ml) was added and the mixture was extracted with tert-butyl methyl ether (3x50 ml). The organic layers were combined and washed with water (2x30 ml), dried (magnesium sulfate) and concentrated to give 1.3 g of a solid/oil mixture. Purification on column chromatography (30 g SiO₂, heptane/ ethyl acetate 3/1) gave pure **1-1-1** as white solid (0.75 g, 60%).

MS (ES+): m/z = 507 (M + Na), 485 (M + H), 429 (M + H - C₄H₈), 385 (M + H - C₄H₈ - CO₂.

¹H-NMR (300 MHz, CD₂Cl₂): δ = 7.97 (db, J = 8.6 Hz, 1H, Bt), 7.38 (mc, 2H, Bt), 7.25 - 7.06 (m, 5H, 1 Bt, Ar-H), 6.03 (s, 2H, O-CH₂-O), 5.05 (db, J = 7.7Hz, 1H, NH), 4.41 (m, 1H, 2-H), 3.09 (dd, J = 13.8Hz, J = 6.0Hz, 1H, 3-H), 3.00 (dd, J = 13.8Hz, J = 6.0Hz, 1H, 3-H), 1.43, 1.41 ppm (s, 18H, 1-tBu, 2-tBu).

¹³C-NMR (75 MHz, CD₂Cl₂): δ = 27.71 (q, 1-C2), 28.01 (q, 2-C3), 37.52 (tb, C-3), 55.00 (d, C-2), 79.40 (sb, 2-C2), 81.93 (s, 1-C1), 99.06 (t, O-CH₂-O), 108.99 (d, Bt C7), 115.94 (d, Ar-C3), 119.83 (d, Bt C4), 124.55 (d, Bt C5), 128.18 (d, Bt C6), 128.68 (s, Bt C7a), 130.95 (d, Ar-C2), 131.94 (s, Ar-C1), 143.44 (s, Bt C3a), 154.88 (sb, 2-C1), 155.12 (s, Ar-C4), 170.72 ppm (s, C1).

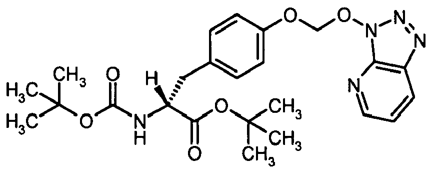

### tert-Butyl N-(tert-butoxycarbonyl)-O-[(1H-1,2,3-triazolo[5,4-b]pyridin-1-yloxy)methyl] -D-tyrosinate. 1-1-2

To a stirred solution of 1*H*-1,2,3-triazolo[5,4-*b*]pyridine-1-ol (0.5 g, 3.7 mmol) in *N,N-*dimethylformamide (1 ml) and dichloromethane (10 ml) was added at r.t. a solution of **3-1-3** (1.0 g, 2.6 mmol) in dichloromethane (5 ml) followed by 4-(dimethylamino)pyridine (0.4 g, 3.2 mmol). The mixture was stirred at r.t. for 3 days after which time TLC indicated only trace of starting material. Water (20 ml) was added and the mixture was extracted with tert-butyl methyl ether (3x25 ml). The organic layers were combined and consecutively washed with water (20 ml), 0.5 N hydrochloric acid (10 ml), brine (10 ml), dried (magnesium sulfate) and concentrated to a sticky solid, 0.85 g. Purification on column chromatography (25 g SiO₂, heptane/ ethyl acetate 1/1) gave pure **1-1-2** as white solid (0.40 g, 32%).

MS (ES+): m/z = 508 (M + Na), 486 (M + H), 430 (M + H - C₄H₈), 374 (M + H - 2 x C₄H₈), 366 (M + H - C₄H₈ - CO₂), 330 (M + H - 2 × C₄H₈ - CO₂).

¹H-NMR (400 MHz, CD₂Cl₂): δ = 8.67 (dd, J = 4.5Hz, J = 1.0Hz, 1H, At 6-H), 8.37 (dd, J = 8.5Hz, J = 1.0Hz, 1H, At 5-H), 7.41 (dd, J = 8.5Hz, J = 4.5Hz, 1H, At 4-H), 7.19 (s, 4H, Ph-H), 6.04 (s, 2H, O-CH₂-O), 5.03 (db, J = 7.6Hz, 1H, NH), 4.38 (m, 1H, 2-H), 3.08 (dd, j 0 13:7Hz, J = 5.9Hz, 1H, 3-H), 3.00 (dd, J = 13.7Hz, J = 6.1Hz, 1H, 3-H). 1.43, 1.41 ppm (s, 18H, 1-*t*Bu, 2-tBu).

¹³C-NMR (100 MHz, CD₂Cl₂): δ = 27.75 (q, 1-C2), 28.06 (q, 2-C3), 37.51 (t, C-3), 55.02 (d, C-2), 79.42 (sb, 2-C2), 81.96 (s, 1-C1), 99.65 (t, O-CH₂-O), 117.13 (d, Ar-C3), 120.76 (d, At C5), 129.12 (d, At C4), 130.82 (d, Ar-C2), 132.21 (s, Ar-C1), 135.14 (s, At C3a), 140.25 (s, At C7a), 151.50 (d, At C6), 154.97 (sb, 2-C1), 155.69 (s, Ar-C4), 170.82 ppm (s, C1).

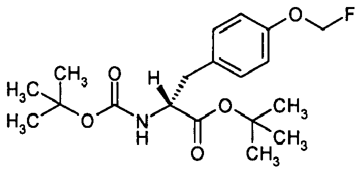

### tert-Butyl N-(tert-butoxycarbonyl)-O-(fluoromethyl)-D-tyrosinate. 2-1-1

A: 1.50 g (4.45 mmol) **3-1-1** were dissolved in 30 ml *N,N*-dimethylformamide, cooled to 10° C and 194 mg (4.45 mmol) sodium hydride (60% in mineral oil) were added in one portion. The mixture was stirred for 30 min.

B: 30 ml *N,N*-dimethylformamide were cooled to 0° C and bromofluoromethane was bubbled into the solution. By weighing of the flask and of the steel container the amount of gas dissolved was determined. 30 ml of *N,N*-dimethylformamide containing 1 g (8.89 mmol) bromofluoromethane were added slowly at 0° C to the solution prepared in A and the reaction was stirred at 0° C for 2 h. The mixture was allowed to warm to r.t. and was stirred another 2 h, after which the reaction mixture was poured in to water and extracted with dichloromethane twice. The combined organic phases were dried over magnesium sulfate, and evaporated to give the raw product as an oil. Chromatography (Silica gel, Gradient hexane to hexane/ ethyl acetate 3:1) gave 1.3 g (79%) of the title compound as clear oil. An analytical sample was purified by preparative HPLC.

HPLC (Chiralpak AD-H 5µ 150x4.6 mm, Hexane / Ethanol 9:1, 1.0 ml/min, (1 mg/ml EtOH, 5 µl injected), DAD 210 nm, 25°C): tr = 4.8 min (96.35%).

MS (Cl, NH₃): m/z = 387 (M + NH₄⁺), 370 (M + H⁺), 331 (M + NH₄⁺ - C₄H₈), 314 (M + H⁺ - C₄H₈).

MS (ES⁺): m/z = 761 (2M + Na⁺), 739 (2M + H⁺), 683 (M + H⁺ - C₄H₈), 639 (2M + H⁺ - C₄H₈ - CO₂). 392 (M + Na⁺), 370 (M + H⁺).

¹H-NMR (400 MHz, CD₂Cl₂): δ = 7.14 (d, J = 8.6 Hz, 2H, Ar), 7.00 (d, J = 8.6Hz, 2H, Ar), 5.69 (d, ²J_{HF} = 54.8Hz, 2H, F-CH₂-O), 4.99 (d, J = 7.6Hz, 1H, NH), 4.36 (mc, 1H, 2-H), 3.00 (mc, 2H, 3-H), 1.41 ppm (s, 9H, 1-tBu), 1.40 (s, 9H, 2-tBu).

¹³C-NMR (100 MHz, CD₂Cl₂): δ = 27.71 (q, 1-C2), 28.03 (qb, 2-C3), 37.48 (t, C-3), 54.99 (d, C-2), 79.38 (sb, 2-C2), 81.92 (s, 1-C1), 100.01 (t, ¹J_{CF} = 217.3Hz, O-CH₂-F), 116.36 (d, Ar-C3), 130.80 (d, Ar-C2), 131.82 (s, Ar-C1), 154.90 (s, 2-C1), 155.72 (s, ³J_{CF} = 3.2Hz, ArC4), 170.79 ppm (s, C1).

### 1.2 Example 2

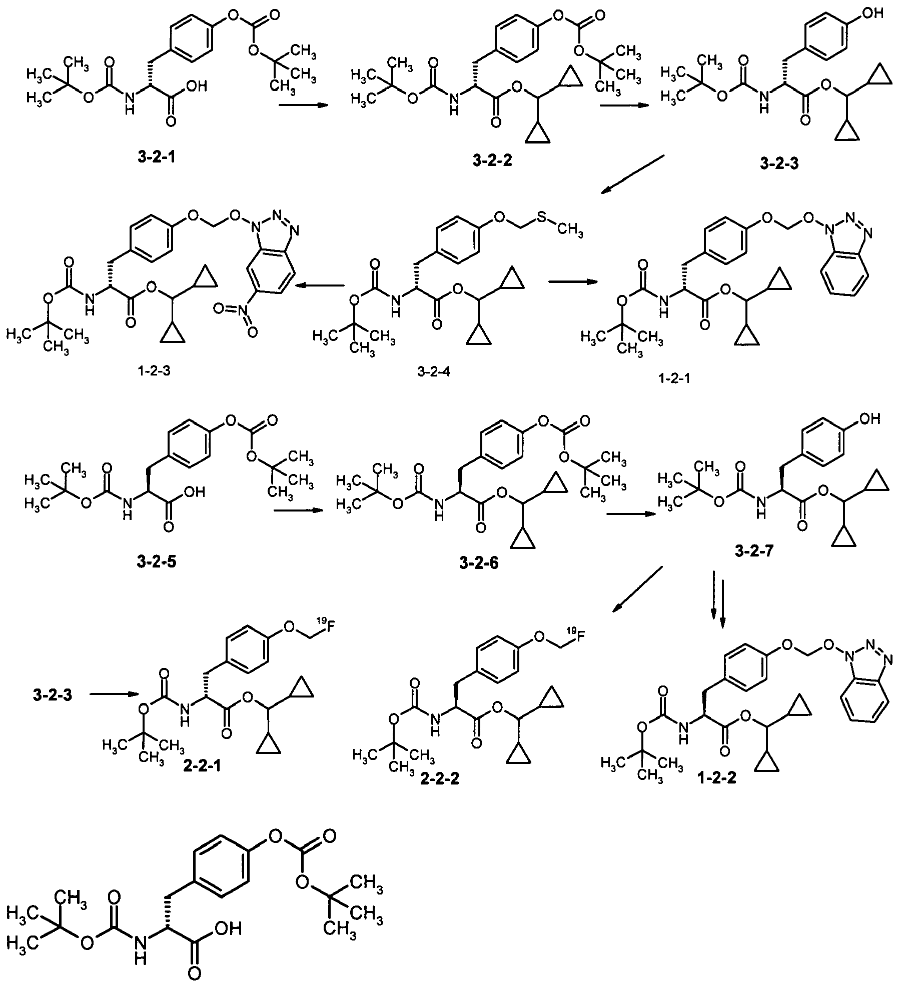

### N,O-Bis(tert-butoxycarbonyl)-D-tyrosine. 3-2-1

18,1g (100.0 mmol) D-tyrosine were suspended in 250 ml water and a solution of 65,4g (300.0 mmol) di-*tert*-butyl dicarbonate in 150 ml 2-propanol was added. The pH was adjusted to 11,5-12 by repeated addition of sodium hydroxide (32% in water). The reaction warmed slightly to about 37°C and was brought to 20°C by cooling. Then 250 ml water were added and the mixture extracted with ether. The combined organic phases were washed with water and dried over sodium sulfate. Evaporation of the solvent gave a gummy residue, which was taken up in ethyl acetate. The solution was filtered and hexane added. Upon evaporation, white crystalls formed, which were dried i. vac. at 30°C. The yield was 39.1 g (>100%).

α_{D} = -27.9 (c = 1, dioxane).

MS (ESI⁺): m/e = 785 (2M + Na⁺), 763 (2M + H⁺), 663 (2M + H⁺ - C₄H₈ - CO₂), 404 (M + Na⁺).

MS (ESI⁻): m/e = 761 (2M - H⁺), 661 (2M - H⁺ - C₄H₈ - CO₂), 380 (M - H⁺).

**¹H** NMR (DMSO-d₆ ,400MHz): δ (ppm) 7.25 (d, *J*=8.6 Hz, 2H, H-2'), 7.06 (d, *J*=8.6 Hz, 2H. H-3'), 6.88 (d, *J*=8.1 Hz, 1H, NH), 4.03 (ddd, *J*=9.3, 8.3, 4.6 Hz, 1H, H-2), 3.03 (dd, *J*=13.6, 4.5 Hz, 1H, H-3), 2.83 (dd, *J*=13.6, 9.9 Hz, 1H, H3), 1.48 (s, 9H), 1.32 (s, 9H).

¹³C NMR (DMSO-d₆, 101MHz): δ (ppm) 173.4 (C-1), 155.3 (2C-1), 151.3 (4'C-1), 149.1 (C-4'), 135.9 (C-1'), 130.1 (C-2'), 120.9 (C-3'), 83.0 (4'C-2), 77.9 (2C-2), 55.3 (C-2), 35.9 (C-3), 28.1 (2C-3), 27.2 (4'C-3).

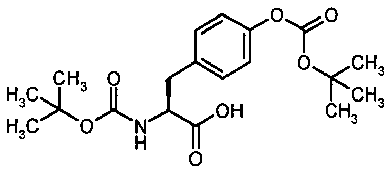

### N,O-Bis(tert-butoxycarbonyl)-L-tyrosine. 3-2-5

In the same way as for **3-2-1,** 18.1 g L-tyrosine were reacted to give 35.9 g (94%) of **3-2-5** as a white solid.

α_{D} = +14.6° (c = 1, dioxane).

MS (ESI⁺): m/e = 785 (2M + Na⁺), 763 (2M + H⁺), 663 (2M + H⁺ - C₄H₈ - CO₂), 404 (M + Na⁺).

MS (ESI⁻): m/e = 761 (2M - H⁺), 661 (2M - H⁺ - C₄H₈ - CO₂), 380 (M - H⁺).

¹H NMR (400 MHz, DMSO-d₆): δ (ppm) 7.09 (d, *J* = 8.3 Hz, 2H), 6.94 (d, *J* = 8.3 Hz, 2H), 5.79 (d, *J* = 6.1 Hz, 1H), 3.69 (dt, *J* = 5.1, 5.8 Hz, 1H), 3.01 (dd, *J* = 5.3, 13.1 Hz, 1H), 2.90 (dd, *J* = 5.6, 13.4 Hz, 1H), 1.44 (s, 9H), 1.31 (s, 9H).

¹³C NMR (DMSO-d₆, 101MHz): δ (ppm) 173.3 (C-1), 155.0 (2C-1), 151.9 (4'C-1), 149.2 (3C-4), 137.5 (3C-1), 130.9 (3C-2), 120.8 (3C-3), 83.3 (4'C-2), 77.6 (2C-2), 56.6 (C-2), 37.2 (C-3), 28.7 (2C-3), 27.7 (4'C-3).

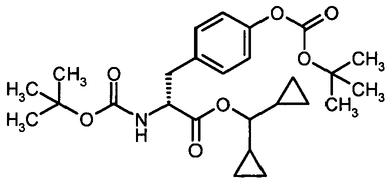

### Dicyclopropylmethyl N,O-bis(tert-butoxycarbonyl)-D-tyrosinate. 3-2-2

10.0g (26.2 mmol) **3-2-1** and 320 mg (2,62 mmol) 4-(dimethylamino)pyridine were dissolved in 30 ml dichloromethane. 3.82 g (34.1 mmol) dicyclopropylmethanol and 653 mg (34.11 mmol) N-ethyl-N'-3-dimethylaminopropyl carbodiimide hydrochloride were added and the mixture stirred at ambient temperature. The reaction was stirred over night. Ethyl acetate was added and the insolubles were filtered off. The filtrate was concentrated i. vac.. Chromatography in two batches on a Biotage system (Flash40+M cartidge, 40 ml/min, n-hexane to n-hexane/ ethyl acetate 1:4 in 30 min) gave 6,99 g (56%) of 3-2-2.

MS (ESI⁺): m/e = 514 (M + K⁺), 498 (M + Na⁺), 476 (M + H⁺), 458 (M + H⁺ - H₂O), 420 (M + H⁺ - C₄H₈), 376 (M + H⁺ - C₄H₈ - CO₂).

¹H NMR (CHLOROFORM-d, 300MHz): ): δ (ppm) 7.21 (d, J=8.7 Hz, 2H, Ar-H), 7.09 (d, J=8.7 Hz, 2H, Ar-H), 5.00 (d, J=7.3 Hz, 1H, NH), 4.52 - 4.64 (m, 1H, 2-H), 3.89 (t, J=8.9 Hz, 1H, OCH), 3.03 - 3.23 (m, 2H, 3H₂), 1.56 (s, 9H, OBoc), 1.43 (s, 9H, NBoc), 1.00 - 1.16 (m, 2H, cyclopropyl CH), 0.41 - 0.64 (m, 4H, cyclopropyl CH₂), 0.25 - 0.41 (m, 4H, cyclopropyl CH₂).

¹³C NMR (CHLOROFORM-d ,75MHz): δ (ppm): 171.4 (C-1), 155.0 (2C-1), 151.8 (4'C-1), 150.0 (3C-4), 133.6 (3C-1), 130.5 (3C-2), 121.1 (3C-3), 83.8 (1C-1), 83.5 (4'C-2), 79.8 (2C-2), 54.4 (C-2), 37.5 (C-3), 28.3 (2C-3), 27.7 (4'C-3), 14.6, 14.6 (cyclopropyl CH), 3.1, 3.0, 2.9, 2.7 (cyclopropyl CH₂).

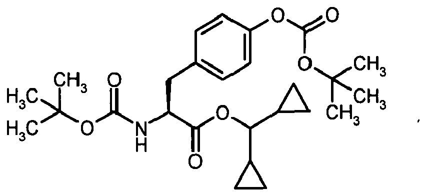

### Dicyclopropylmethyl N,O-bis(tert-butoxycarbonyl)-L-tyrosinate. 3-2-6

In the same way as for **3-2-2,** 10 g **3-2-5** were reacted to give 5.52 g (44%) **3-2-6.**

MS (CI⁺, NH₃): m/e = 493 (M + NH₄⁺), 476 (M + H⁺), 437 (M + NH₄⁺ - C₄H₈), 420 (M + H⁺ - C₄H₈), 376 (M + H⁺ - C₄H₈ - CO₂), 95 (C₇H₁₁⁺).

¹H NMR (CHLOROFORM-d ,300MHz): δ (ppm) 7.20 (d, *J*=8.7 Hz, 2H, Ar-H), 7.08 (d, *J*=8.5 Hz, 2H, Ar-H), 4.99 (d, *J*=7.9 Hz, 1H, NH), 4.49 - 4.65 (m, 1H, 2-H), 3.87 (t, *J*=8.5 Hz, 1H, OCH), 3.03 - 3.20 (m, 2H, 3-H₂), 1.55 (s, 9H, OBoc), 1.42 (s, 9H, NBoc), 0.98 - 1.14 (m, 2H, Cyclopropyl CH), 0.40 - 0.62 (m, 4H, Cyclopropyl CH₂), 0.25 - 0.39 (m, 4H, Cyclopropyl CH₂).

¹³C NMR (CHLOROFORM-d ,75MHz): δ (ppm) 171.4 (C-1), 155.0 (2C-1), 151.8 (OBoc C-1), 150.0 (3C-4), 133.6 (3C-1), 130.5 (3C-2), 121.1 (3C-3), 83.8 (1C-1), 83.5 (OBoc C-2), 79.8 (2C-2), 54.4 (C-2), 37.5 (C-3), 28.3 (3C-3), 27.7 (OBoc C-3), 14.7, 14.6 (cyclopropyl CH), 3.2, 3.0, 2.9, 2.7 (Cyclopropyl CH).

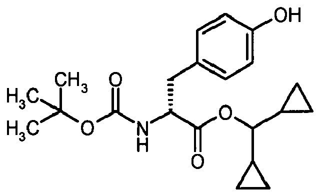

### Dicyclopropylmethyl N-(tert-butoxycarbonyl)-D-tyrosinate. 3-2-3

5.0 g (10.5 mmol) **3-2-2** was dissolved in 150 ml dichloromethane and 150 ml piperidine added. The mixture was stirred at r.t. for 3 h, after which HPLC/MS indicated complete conversion. Ethyl acetate was added the insolubles were filtered off. The filtrate was concentrated i. vac. and taken up in ethyl acetate. Again the insolubles were separated and the solvent removed i. vac.. Chromatography in two batches on a Biotage system (Flash40+M cartidge, 40 ml/min, hexane to ethyl acetate in 30 min) gave 3.74 g (95%) of **3-2-3.**

¹H NMR (CHLOROFORM-d ,400MHz): δ (ppm) 7.05 (d, *J*=8.0 Hz, 2H, Ar-H), 6.73 (d, *J*=8.0 Hz, 2H, Ar-H), 5.71 (br. s., 1H, NH), 5.02 (d, *J*=8.0 Hz, 1H), 4.49 - 4.60 (m, 1H, 2-H), 3.90 (t, *J*=8.3 Hz, 3H, OCH), 2.96 - 3.13 (m, 2H, 3-H₂), 1.44 (s, 9H, Boc), 1.03 - 1.16 (m, 2H, cyclopropyl CH), 0.53 - 0.64 (m, 2H, cyclopropyl CH₂), 0.43 - 0.52 (m, 2H, cyclopropyl CH₂), 0.28 - 0.42 (m, 4H, cyclopropyl CH₂).

¹³C NMR (CHLOROFORM-d, 101 MHz): δ (ppm) 171.8 (C-1), 155.2 (2C-1), 154.9 (3C-4), 130.6 (3C-2), 127.8 (3C-1), 115.3 (3C-3), 83.7 (1C-1), 79.9 (2C-2), 54.7 (C-2), 37.4 (C-3), 28.3 (2C-3), 14.7, 14.6 (cyclopropyl CH), 3.1, 3.0, 2.9, 2.7 (cyclopropyl CH₂).

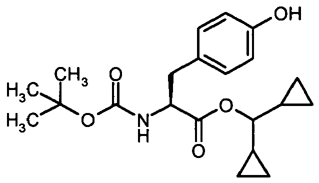

### Dicyclopropylmethyl N-(tert-butoxycarbonyl)-L-tyrosinate. 3-2-7

In the same way as for **3-2-3,** 2.5 g of **3-2-6** were reacted to give 1.53 g (77%) of slightly impure **3-2-7.**

450 mg were purified by preparative HPLC:

Dionex: Pump P 580, Gilson: Liquid Handler 215, Knauer: UV-Detector K-2501, Chiralpak IC 5µm 250x30 mm, hexane / ethanol 95:5, 40 ml/min, r.t., 450 mg / 3.0 ml ethanol, 8 x 0.35 ml, UV 220 nm. 298 mg of **3-2-7** were obtained with 99.6% purity.

MS (ESI+): m/e = 773 (2M + Na+), 751 (2M + H+), 473.

MS (ESI-): m/e = 795 (2M + HCOO-), 749 (2M - H+), 420, (M + HCOO-). ¹H NMR (CHLOROFORM-d ,400MHz): δ (ppm) 7.04 (d, J=8.1 Hz, 2H, Ar-H), 6.72 (d, J=8.3 Hz, 2H, Ar-H), 4.94 - 5.07 (m, 1H, NH), 4.46 - 4.60 (m, 1H, 2-H), 3.88 (t, J=8.3 Hz, 1H, OCH), 2.96 - 3.12 (m, 2H, 3-H2), 1.42 (s, 9H, Boc), 1.02 - 1.15 (m, 2H, cyclopropyl CH), 0.52 - 0.64 (m, 2H, cyclopropyl CH₂), 0.41 - 0.52 (m, 2H, cyclopropyl CH₂), 0.26 - 0.41 (m, 4H, cyclopropyl CH₂).

¹³C NMR (CHLOROFORM-d ,101MHz): δ (ppm) 171.8 (C-1), 155.3 (3C-4), 155.1 (2C-1), 130.7 (3C-2), 127.7 (3C-1), 115.4 (3C-3), 83.8 (1C-1), 79.9 (2C-2), 54.8 (C-2), 37.4 (C-3), 28.4 (2C-3), 14.7, 14.7 (cyclopropyl CH), 3.2, 3.0, 3.0, 2.8, 2.7 (cyclopropyl CH₂).

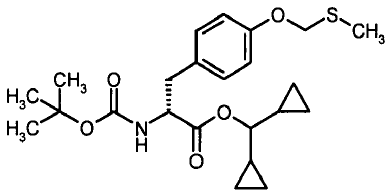

### Dicyclopropylmethyl N-(tert-butoxycarbonyl)-O-[(methylsulfanyl)methyl] -D-tyrosinate. 3-2-4

A solution of 2.5 g (6.66 mmol) **3-2-3,** 100 mg (0.67 mmol) sodium iodide and 17 ml N,N-dimethylformamide was cooled to 0°C in an ice bath. A suspension of 822 mg (7.23 mmol) potassium tert-butylate in 10 ml tetrahydrofuran was added, resulting in a greenish solution. 510 µl chloro dimethyl sulfide were added. The mixture was allowed to come to r.t., after 2 h HPLC/MS indicated complete consumption of the starting material. Ethyl acetate was added and the insolubles were filtered off. The filtrate was concentrated i. vac.. Chromatography on a Biotage system (Flash40+M cartidge, 40 ml/min, n-hexane to n-hexane / ethyl acetate 1:4 in 30 min) gave 1.60 g (55%) of **3-2-4.**

MS (ESI⁺): m/e = 771 (2M + H⁺ - C₄H₈ - CO₂), 590 (771 - Tyr), 530 (M + C₇H₁₁), 474 (M + K⁺), 436 (M + H⁺), 380 (M + H⁺ - C₄H₈), 336 (M + H⁺ - C₄H₈ - CO₂).

¹H NMR (CHLOROFORM-d ,400MHz): δ (ppm) 7.10 - 7.17 (m, 2H, Ar-H), 6.84 - 6.91 (m, 2H, Ar-H), 5.12 (s, 2H, OCH₂S), 4.98 (d, *J*=7.8 Hz, 1H, NH), 4.50 - 4.62 (m, 1H, 2-H), 3.90 (t, *J*=8.3 Hz, 1H, OCH), 2.99 - 3.17 (m, 2H, 3-H₂), 2.25 (s, 3H, S-CH₃), 1.43 (s, 9H, Boc), 1.02 - 1.14 (m, 2H, cyclopropyl CH), 0.52 - 0.63 (m, 2H, cyclopropyl CH₂), 0.42 - 0.53 (m, 2H, cyclopropyl CH₂), 0.27 - 0.42 (m, 4H, cyclopropyl CH₂).

¹³C NMR (CHLOROFORM-d, 101MHz): δ (ppm) 171.5 (C-1), 156.1 (3C-4), 155.0 (2C-1), 130.6 (3C-2), 129.3 (3C-1), 115.9 (3C-3), 83.6 (1C-1), 79.7 (2C-2), 72.5 (OCH₂S), 54.6 (C-2), 37.3 (C-3), 28.3 (2C-3), 14.6, 14.6 (cyclopropyl CH), 14.6 (SCH₃), 3.1, 3,0, 2.9, 2.7 (cyclopropyl CH₂).

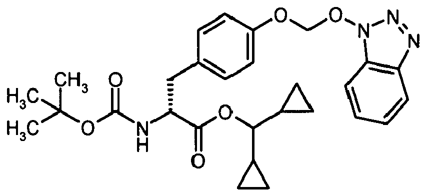

### Dicyclopropylmethyl O-[(1H-benzotriazol-1-yloxy)methyl]-N-(tert-butoxycarbonyl) -D-tyrosinate. 1-2-1

1. A solution of 500 mg (1.15 mmol) Dicyclopropylmethyl *N*-(tert-butoxycarbonyl)-O-[(methylsulfanyl)methyl]-D-tyrosinate **3-2-4** in 4.5 ml dichloromethane was cooled to -15°C and 169 mg (1.26 mmol) N-chlorosuccinimide added. After 4 h during which the mixture was allowed to come to ambient temperature, UPLC-MS indicated formation of about 50% chloromethyl ether.
2. 495,9 mg (3.66 mmol) 1*H*-benzotriazol-1-ol hydrate were stirred with 3,67ml tetrabutyl ammonium hydroxide (anhydrous, 1 M in methanol). After 30 min at ambient temperature, the solution was carefully evaporated i. vac. at max. 40°C. The residue was dissolved twice in anhydrous toluene and evaporated as described above. A yellow solid resulted, which was used without further purification.
3. The tetrabutylammonium 1*H*-benzotriazol-1-olate prepared above was dissolved in 5 ml dichloromethane and molecular sieve (4Ǻ) added. To this solution the chloromethyl ether prepared under 1.) was added at ambient temperature and stirred over night.

The solution was directly chromatographed on a Biotage system (Flash40+M cartidge, 40 ml/min, n-hexane to n-hexane / ethyl acetate 1:4 in 20CV = 2640 ml) gave 71.5 mg (12%) of **1-2-1.**

The compound was further purified by preparative HPLC: Waters Autopurificationsystem: Pump 254, Sample Manager 2767, CFO; DAD 2996, ELSD 2424, SQD 3001; XBrigde C18 5µm 150x19 mm; A = water + 0.1% formic acid; B = acetonitrile; 0-1 min 40% B, 1-8 min 40-100% B, 8-10 min 100% B; 25 ml/min; r.t.; 71 mg / 2 ml dimethyl sulfoxide/ methanol 1:1; 2 x 1 ml, DAD scan range 210-400 nm. Peak at 7.2 - 7.5 min was collected to give 36 mg of the title material (purity: 95.0 % by DAD).

The acid solvent apparently caused some decomposition during evaporation. The compound was repurified: Agilent: Prep 1200, 2xPrep Pump, DLA, MWD, Prep FC, ESA: Corona; Chiralpak IC 5 µm 250x20 mm; hexane / ethanol 80:20; 20 ml/min; r.t.; 36 mg / 1.5 ml ethanol/methanol 1:1; 2 x 0.75 ml; UV 254 nm. The peak at 10.6 - 12.6 min was collected to give 22 mg of the title material (purity: 99.7 % by UV).

¹H NMR (DICHLOROMETHANE-d2 ,300MHz): δ (ppm) 7.93 - 8.05 (m, 1H), 7.31 - 7.47 (m, 2H), 7.24 (d, J=8.7 Hz, 2H) 7,17 - 7,25 (m, 1H), 7.08 (d, J=8.7 Hz, 2H), 6.03 (s, 2H, OCH₂O), 5.05 (d, J=7.7 Hz, 1H, NH), 4.46 - 4.63 (m, 1H, 2-H), 3.90 (t, J=8.7 Hz, 1H, OCH), 2.95 - 3.30 (m, 2H, 3-H₂), 1.41 (s, 9H, tBu), 1.02 - 1.19 (m, 2H, CH,cyclopropyl), 0.41 - 0.66 (m, 4H, CH₂,cyclopropyl), 0.26 - 0.41 (m, 4H, CH₂,cyclopropyl).

¹³C NMR (DICHLOROMETHANE-d₂ ,75MHz): δ (ppm) 171.4 (C-1), 155.2 (2C-1), 154.9 (3C-4), 143.5 (BtC-4), 131.8 (3C-1), 131.0 (3C-2), 128.7 (BtC-7a), 128.2 (BtC-6), 124.6 (BtC-5), 119.9 (BtC-4), 116.1 (3C-3), 109.0 (BtC-7), 99.1 (OCH₂O), 83.6 (1C-1), 79.5 (2C-2), 54.7 (C-2), 37.3 (C-3), 28.1 (2C-3), 14.6, 14.6 (cyclopropyl CH), 3.0, 2.7, 2,7, 2.5 (Cyclopropyl CH₂).

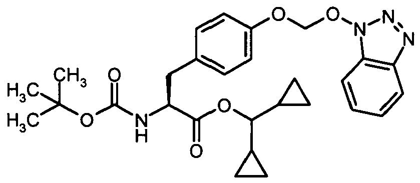

### Dicyclopropylmethyl O-[(1H-benzotriazol-1-yloxy)methyl]-N-(tert-butoxycarbonyl) -L-tyrosinate. 1-2-2

The compound can be prepared in analogy to **1-2-1** from **3-2-7.** In the fact, it was isolated from a preparation of **1-2-1,** where a stereochemically impure tyrosine derivative had been inadvertedly used as starting material.

307 mg of a mixture were purified by preparative HPLC: Dionex: Pump P 580, Gilson: Liquid Handler 215, Knauer: UV-Detector K-2501; Chiralpak IC 5 µm 250x30 mm; hexane / ethanol 80:20; 30 ml/min; r.t.; 307 mg / 1.5 ml ethanol; 6 x 0.25 ml; UV 254 nm. The peak at 15.7 to 17.5 min was collected to give 128 mg of **1-2-1** with 98% purity. The peak at 20.0 to 21.3 min was collected to give 30 mg of **1-2-2** with 98% purity.

MS (ESI⁺): m/e = 524 (M + H⁺).

¹H NMR (DICHLOROMETHANE-d₂ ,300MHz): δ (ppm) 7.97 (dt, J=7.5, 0.9 Hz, 1H, Bt-H), 7.31 - 7.47 (m, 2H, Bt-H), 7.24 (d, J=8.7 Hz, 2H, Ar-H), 7.21 (d, J=7.5 Hz, 1H, Bt-H), 7.08 (d, J=8.7 Hz, 2H, Ar-H), 6.03 (s, 2H, OCH₂O), 5.06 (d, J=7.5 Hz, 1H, NH), 4.53 (dt, J=7.5, 5.8 Hz, 1H, 2-H), 3.90 (t, J=8.5 Hz, 1H, CHO), 3.17 (dd, J=13.8, 5.8 Hz, 1H, 3-H), 3.06 (dd, J=13.8, 5.7 Hz, 1H, 3-H), 1.41 (s, 9H, Boc), 1.04 - 1.18 (m, 2H, cyclopropyl CH), 0.42 - 0.65 (m, 4H, cyclopropyl CH₂), 0.30 - 0.41 (m, 4H, cyclopropyl CH₂).

¹³C NMR (DICHLOROMETHANE-d₂ ,75MHz): δ (ppm) 171.3 (C-1), 155.1 (3C-4), 154.9 (2C-1), 143.4 (Bt C-3a), 131.7 (3C-1), 131.0 (3C-2), 128.6 (Bt C-7a), 128.2 (Bt C-6), 124.6 (Bt C-5), 119.8 (Bt C-4), 116.0 (3C-3), 109.0 (Bt C-7), 99.0 (OCH₂O), 83.6 (1C-1), 79.5 (2C-2), 54.7 (C-2), 37.3 (C-3), 28.0 (2C-3), 14.6, 14.5 (cyclopropyl CH), 3.0, 2.7, 2.4 (cyclopropyl CH₂).

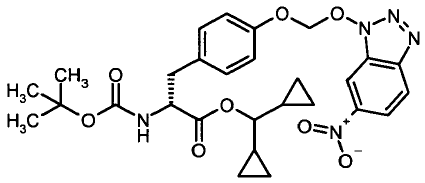

### Dicyclopropylmethyl O-[(6-nitro-1H-benzotriazol-1-yloxy)methyl]-N-(tert-butoxy carbonyl)-D-tyrosinate. 1-2-3

In the same way as described for **1-2-1,** 150 mg (0.30 mmol) **3-2-4** in 2.5 ml dichloromethane were reacted to give after chromatography (Biotage system SNAP 25 cartidge, **25** ml/min, n-hexane to n-hexane / ethyl acetate 6:4 in 10CV, then isokratic 4 CV) 74 mg (43%) of **1-2-3,** which was further purified by preparative HPLC: (Dionex: Pump P 580, Gilson: Liquid Handler 215, Knauer: UV-Detektor K-2501; Chiralpak IC 5µm 250x20 mm; Hexan / Ethanol 50:50; 30 ml/min; RT; 74mg / 2.0 ml EtOH; 2 x 1.0 ml: UV 210 nm). The fraction eluting at 10.6 -11.8 min were collected to give 45 mg (26%) of **1-2-3** with a purity of 99.5 %.

MS (ESI⁺): m/e = 662 (M + C₇H₁₁⁺), 590 (M + Na⁺), 568 (M + H⁺), 512 (M + H⁺ -C₄H₈), 468 (M + H⁺- CO₂ - C₄H₈).

¹H NMR (DICHLOROMETHANE-d₂ ,500MHz): δ (ppm) 8.25 (dd, *J*=9.1, 1.3 Hz, 1H, Bt H-5), 8.18 (d, *J*=9.1 Hz, 1H, Bt H-4), 8.19 (d, *J*=1.3 Hz, 1H, Bt H-7), 7.31 (d, *J*=8.2 Hz, 2H, Ar-H), 7.11 (d, *J*=8.2 Hz, 2H, Ar-H), 6.14 (s, 2H, OCH₂O), 5.12 (d, *J*=7.6 Hz, 1H, NH), 4.57 (dt, *J*=7.6, 5.4 Hz, 1H, 2-H), 3.95 (t, *J*=8.2 Hz, 1H, OCH), 3.24 (dd, *J*=13.9, 5.4 Hz, 1H, 3-H), 3.14 (dd, *J*=13.9, 5.4 Hz, 1H, 3-H), 1.46 (s, 9H, Boc), 1.10 - 1.21 (m, 2H, cyclopropyl CH), 0.47 - 0.67 (m, 4H, cyclopropyl CH₂), 0.35 - 0.45 (m, 4H, cyclopropyl CH₂).

¹³C NMR (DICHLOROMETHANE-d₂ ,126MHz): δ (ppm) 171.3 (C-1), 154.9 (2C-1), 154.6 (3C-4), 147.4 (Bt C-6), 145.4 (Bt C-3a), 132.3 (3C-1), 131.3 (3C-2), 128.2 (Bt C-7a), 121.2 (Bt C-4), 119.5 (Bt C-5), 115.9 (3C-3), 106.7 (Bt C-7), 99.4 (OCH₂O), 83.6 (1C-1), 79.5 (2C-2), 54.7 (C-2), 37.2 (C-3), 28.0 (2C-3), 14.6, 14.5 (1 C-2), 3.0, 2.7, 2.5 (1C-3/4).

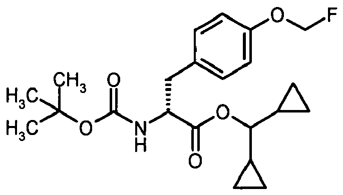

### Dicyclopropylmethyl N-(tert-butoxycarbonyl)-O-(fluoromethyl)-D-tyrosinate. 2-2-1

As described in the preparation of **2-1-1,** 250 mg (0,67 mmol) **3-2-3** were reacted to give 290 mg of raw product, which was purified by preparative HPLC.

Dionex: Pump P 580, Gilson: Liquid Handler 215, Knauer: UV-Detector K-2501, Chiralpak IC 5 µm 250x30 mm, hexane / ethanol 95:5, 40 ml/min, r.t., 290 mg / 3 ml ethanol, 10 x 0.3 ml, UV 220 nm. The peak at 7.7 - 8.2 min was collected to give 85 mg (33%) **2-2-1** with 99.8 % purity.

¹H NMR (CHLOROFORM-d ,400MHz): δ (ppm) 7.16 (d, J=8.5 Hz, 2H, Ar-H), 6.99 (d, *J*=8.5 Hz, 2H, Ar-H), 5.68 (d, ²J_{HF}=54.7 Hz, 2H, OCH₂F), 4.98 (d, *J*=7.8 Hz, 1H, NH), 4.50 - 4.62 (m, 1H, 2-H), 3.89 (t, *J*=8.5 Hz, 2H, CHO), 3.13 (dd, *J*=14.1, 6.0 Hz, 1H, 3-H), 3.05 (dd, *J*=13.8, 5.5 Hz, 1H, 3-H), 1.42 (s, 9H, Boc), 0.99 - 1.16 (m, 2H, Cyclopropyl CH), 0.52 - 0.63 (m, 2H, cyclopropyl CH₂), 0.41 - 0.52 (m, 2H, cyclopropyl CH₂), 0.27 - 0.40 (m, 4H, cyclopropyl CH₂).

¹³C NMR (101 MHz, CHLOROFORM-*d*): δ ppm 171.5 (C-1) 155.8 (d, ³J_{CF}=3,1 Hz, 3C-4) 155.0 (2C-1) 131.31(3C-1) 130.8 (3C-2) 116.6 (3C-3) 100.8 (d, ¹J_{CF}=218.5 Hz, OCH₂F) 83.7 (1C-1) 79.76 (2C-2) 54.56 (C-2) 37.41 (C-3) 28.31 (2C-3) 14.66, 14.62 (cyclopropylCH) 3.13, 2.96, 2.92, 2.71 (cyclopropyl CH₂).

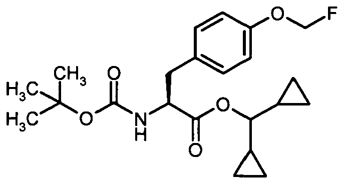

### Dicyclopropylmethyl N-(tert-butoxycarbonyl)-O-(fluoromethyl)-L-tyrosinate. 2-2-2

As described in the preparation of **2-1-1,** 250 mg (0,67 mmol) **3-2-7** were reacted to give 244 mg of raw product, which was purified by preparative HPLC.

Dionex: Pump P 580, Gilson: Liquid Handler 215, Knauer: UV-Detector K-2501, Chiralpak IC 5 µm 250x30 mm, hexane / ethanol 95:5, 40 ml/min, r.t., 244 mg / 1.8 ml ethanol/methanol 1:1, 3 x 0.6 ml, UV 220 nm. The peaks at 8.3 - 9.2 min were collected to give 83 mg (38%) of **2-2-2** in >99 % purity.

¹⁹F NMR (DMSO-d₆ ,376MHz): δ (ppm) -149.8 (t, ¹J_{HF} =55.1 Hz).

¹H NMR (DMSO-d₆ ,300MHz): δ (ppm) 7.21 (d, *J*=8.5 Hz, 2H, Ar-H), 6.98 (d, *J*=8.5 Hz, 2H, Ar-H), 5.78 (d, ¹J_{HF} =54.3 Hz, 2H, OCH₂O), 3.97 - 4.12 (m, 1H, NH), 3.82 (t, *J*=81 Hz, 1H, OCH), 2.90 (dd, *J*=13.8, 5.7 Hz, 1H, 3-H), 2.80 (dd, *J*=13.6, 10.0 Hz, 1H, 3-H), 1.30 (s, 9H, Boc), 0.92 - 1.12 (m, 2H, cyclopropyl CH), 0.09 - 0.53 (m, 8H, cyclopropyl CH₂).

¹³C NMR (DMSO-d₆, 75MHz): δ (ppm) 172.1 (C-1), 155.7 (3C-4), 155.2 (2C-1), 132.8 (3C-1), 130.9 (3C-2), 116.4 (3C-3), 101.0 (d, ¹J_{CF} = 215.8 Hz, OCH₂O), 81.8 (1C-1), 78.6 (2C-2), 56.2 (C-2), 36.1 (C-3), 28.6 (2C-2), 15.0 (cyclopropyl CH), 3.1, 2.9, 2.8, 2.8 (cyclopropyl CH₂).

### 1.3 Example 3

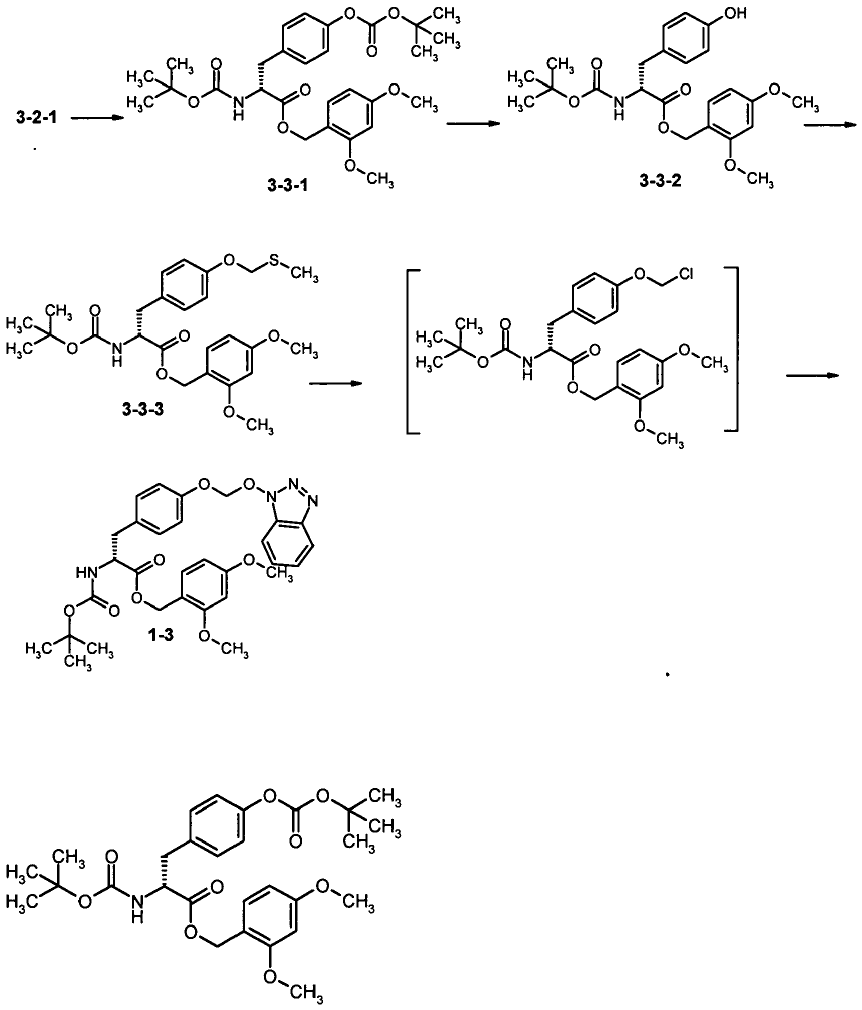

### 2,4-Dimethoxybenzyl N,O-bis(tert-butoxycarbonyl)-D-tyrosinate . 3-3-1

5.0 g (13.1 mmol) **3-2-1** and 160 mg (1,31 mmol) 4-(dimethylamino)pyridine were dissolved in 30 ml dichloromethane (previously dried over 4Ǻ molecular sieve). 2,87 g (17.0 mmol) 2,4-Dimethoxybenzyl alcohol and 3.27 g (17.0 mmol) *N*-ethyl-*N'*-3-dimethylaminopropyl carbodiimide hydrochloride were added and the mixture stirred at ambient temperature over night. Ethyl acetate was added and the insolubles were filtered off. The filtrate was concentrated i. vac.. Column chromatography over 500 g silicagel with stepwise gradient (1L hexane, hexane/ethyl acetate 9:1, hexane/ethyl acetate 8:2, hexane/ethyl acetate 7:3, hexane/ethyl acetate 6:4, respectively) gave 2.15 g (31 %) of **3-3-1.** (smaller scale reactions gave 49 - 55% yield).

MS (ESI⁺): m/e = 549 (M + H⁺ + OH), 532 (M + H⁺), 151 (C₉H₁₁O₂⁺).

¹H NMR (CHLOROFORM-d ,400MHz): δ (ppm) 7.19 (d, J=9.1 Hz, 2H, Dmb H-7), 6.98 - 7.10 (m, 4H, Dmb H6, H-4, Tyr H-4/8), 6.42 - 6.51 (m, 2H, Tyr H-5/7), 5.19 (d, J=11.1 Hz, 1H, Dmb H-1), 5.07 (d, J=11.1 Hz, 1H, Dmb H-1), 4.99 (d, J=8.1 Hz, 1H, NH), 4.54 - 4.64 (m, 1H, Tyr H-2), 3.83 (s, 3H, Dmb OMe), 3.82 (s, 3H, Dmb OMe), 3.00 - 3.15 (m, 2H, Tyr H-3), 1.56 (s, 9H, tBu), 1.41 (s, 9H, t-Bu).

¹³C NMR (CHLOROFORM-d ,101MHz): δ (ppm) 171.7 (C-1), 161.6 (Dmb C-5), 159.2 (Dmb C-3), 155.1 (2C-1), 151.9 (OBoc C-1), 150.0 (3C-4), 133.6 (3C-1), 132.0 (Dmb C-7), 130.4 (3C-2), 121.2 (3C-3), 116.0 (Dmb C-2), 104.1 (Dmb C-6), 98.6 (Dmb C-4), 83.5 (OBoc C-2), 79.9 (2C-2), 62.8 (Dmb C-1), 55.5 (Dmb-OMe), 55.5 (Dmb-OMe), 54.3 (C-2), 37.5 (C-3), 28.4 (2C-3), 27.7 (OBoc C-3).

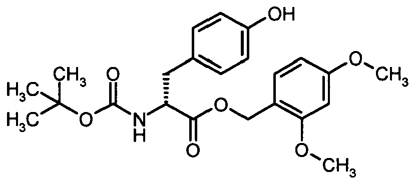

### 2,4-Dimethoxybenzyl N-(tert-butoxycarbonyl)-D-tyrosinate. 3-3-2

2.10 g (3,95 mmol) **3-3-1** was dissolved in 40 ml dichloromethane (dried over 4Ǻ molecular sieve) and 40 ml piperidine added. The mixture was stirred at r.t. for 2 h, after which HPLC/MS over 80% conversion. The reaction mixture was partitioned between ethyl-acetate and water, The organic phase was separated and dried over sodium sulfate. The residue obtained upon evaporation i. vac. (3.8g) was purified by chromatography on a Biotage system (Flash 40+M, n-hexane to ethyl acetate in 15CV =1980 ml) to give 1.10 g (64.5%) **3-3-2.**

¹H NMR (400 MHz, DICHLOROMETHANE-d₂): δ (ppm) 7.19 (d, *J* = 7.8 Hz, 1H, DMB 6-H), 6.92 (m, d, *J* = 8.6 Hz, 2H, Ar-H), 6.68 (d, *J* = 8.1 Hz, 2H, Ar-H), 6.43 - 6.51 (m, 2H, DMB 3-H, 5-H), 5.14 (d, *J* = 11.9 Hz, 1H, DMB 1-H), 5.00 (d, *J* = 8.3 Hz, 1H, NH), 5.05 (d, *J* = 11.6 Hz, 1H, DMB 1-H), 4.41 - 4.54 (m, 1H, 2-H), 3.82 (s, 3H, DMB OMe), 3.81 (s, 3H, DMB OMe), 2.83 - 3.08 (m, 2H, 3-H), 1.39 (s, 9H, Boc).

¹³C NMR (101 MHz, DICHLOROMETHANE-d₂): δ (ppm) 171.9 (C-1), 161.6 (DMB C-5), 159.2 (DMB C-3), 155.1 (3C-4), 155.1 (2C-1), 131.7 (DMB C-7), 130.5 (3C-2), 127.8 (3C-1), 116.0 (DMB C-2), 115.2 (3C-3), 104.1 (DMB C-6), 98.4 (DMB C-4), 79.6 (2C-2), 62.6 (DMB C-1), 55.5 (DMB 5-OMe), 55.4 (DMB 3-OMe), 54.7 (C-2), 37.2 (C-3), 28.0 (2C-3).

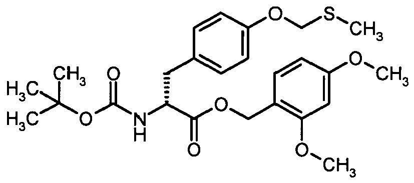

### 2,4-Dimethoxybenzyl N-(tert-butoxycarbonyl)-O-[(methylsulfanyl)methyl] -D-tyrosinate. 3-3-3

1.1 g (2.556 mmol) **3-3-2** were reacted as described for **3-2-4.** Chromatography of the raw product on a Biotage system (Flash 40+M, gradient hexane to ethyl acetate/hexane 1:3, 15CV =1980 ml) gave 490 mg (39%) of **3-3-3.**

MS (ESI⁺): m/e = 514 (M + Na⁺), 301 (C₁₈H₂₁O₄⁺).151 (C₉H₁₁O₂⁺).

MS (ESI⁻): m/e = 536 (M + HCOO⁻).

¹H NMR (400 MHz, DICHLOROMETHANE-d₂) δ (ppm) 7.20 (d, *J* = 8.1 Hz, 1H, Dmb 7-H), 7.00 (d, *J* = 8.3 Hz, 2H, Ar-H), 6.82 (d, *J* = 8.6 Hz, 2H, Ar-H), 6.49 (d, *J* = 2.3 Hz, 1H, Dmb 4-H), 6.47 (dd, *J* = 2.3, 8.3 Hz, 1H, Dmb 6-H), 5.12 (s, 2H, S-CH₂), 5.14 (d, *J* = 11.9 Hz, 1H), 5.06 (d, *J* = 11.9 Hz, 1H), 4.98 (d, *J* = 8.1 Hz, 1H, NH), 4.43 - 4.55 (m, 1H. 2-H), 3.82 (s, 3H, Dmb-OCH₃), 3.81 (s, 3H, Dmb-OCH₃), 3.03 (dd, *J* = 5.3, 13.9 Hz, 1H, 3-H), 2.96 (dd, *J* = 5.8, 13.9 Hz, 1H, 3-H), 2.23 (s, 3H, SCH₃), 1.39 (s, 9H, Boc).

¹³C NMR (101 MHz, DICHLOROMETHANE-d₂): δ (ppm) 171.8 (C-1), 161.6 (Dmb C-5), 159.2 (Dmb C-3), 156.1 (3C-4), 154.9 (2C-1), 131.8 (Dmb C-7), 130.4 (3C-2), 129.3 (3C-1), 116.0 (Dmb C-2), 115.8 (3C-3), 104.1 (Dmb C-6), 98.4 (Dmb C-4), 79.5 (2C-2), 72.4 (OCH₂S), 62.6 (Dmb C-1), 55.5 (Dmb OCH₃), 55.4 (Dmb OCH₃), 54.6 (C-2), 37.2 (C-3), 28.0 (3C-3), 14.3 (SCH₃).

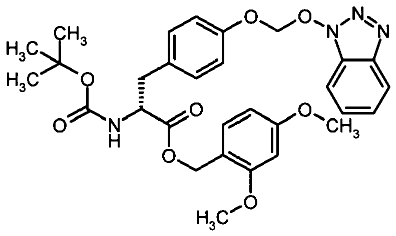

### 2,4-Dimethoxybenzyl O-[(1H-benzotriazol-1-yloxy)methyl]-N-(tert-butoxycarbonyl) -D-tyrosinate 1-3

240 mg (0.49 mmol) **3-3-3** were reacted as described for **1-2-1.** The raw product was directly chromatographed on a Biotage system (Flash40+M cartidge, 40 ml/min, n-hexane to n-hexane / ethyl acetate 65:35 in 18CV = 2376 ml) gave 75 mg (27%) of **1-3.**

The compound was further purified by preparative HPLC: Dionex: Pump P 580, Gilson: Liquid Handler 215, Knauer: UV-Detector K-2501; Chiralpak IC 5 µm 250x20 mm; hexane / ethanol 80:20; 20 ml/min; r.t.; 71 mg / 1 ml ethanol/methanol 1:1; 2 x 0.5 ml, UV 254 nm. The peak at 20.0 - 22.5 min was collected to give 43 mg (15%) of the title material (purity: 98,5%) as white solid.

MS (Cl⁺_{'} NH₃): m/e = 578 (M⁺), 151 (C₉H₁₁O⁺).

¹H NMR (DICHLOROMETHANE-d₂ ,400MHz): δ (ppm) 8.03 (d, *J*=8.3 Hz, 1 H, Bt), 7.38 - 7.49 (m, 2 H, Bt), 7.26 (d, *J*=8.3 Hz, 1 H, Dmb 7-H), 7.24 (d, *J*=8.5 Hz, 1 H, Bt), 7.15 (d, *J*=8.5 Hz, 2 H, Ar-H), 7.07 (d, *J*=8.5 Hz, 2 H, Ar-H), 6.54 (d, *J*=2.3 Hz, 1 H, Dmb 4-H), 6.51 (dd, *J*=8.3, 2.3 Hz, 1 H, Dmb 6-H), 6.07 (s, 2 H, OCH₂O), 5.22 (d, *J*=11.8 Hz, 1 H, Dmb-1-H), 5.14 (d, *J*=11.5 Hz, 1 H, Dmb 1-H), 5.09 (d, *J*=7.3 Hz, 1 H, NH), 4.52 - 4.65 (m, 1 H, 2-H), 3.88 (s, 3 H, Dmb OMe), 3.84 (s, 3 H, Dmb OMe), 3.16 (dd, *J*=13.8, 5.5 Hz, 1 H, 3-H), 3.07 (dd, *J*=13.8, 5.3 Hz, 1 H, 3-H), 1.46 (s, 9 H, Boc).

¹³C NMR (101 MHz, DICHLOROMETHANE-d₂): δ (ppm) 172.0 (C-1), 162.1 (Dmb C-5), 159.6 (Dmb C-3), 155.5 (3C-4), 155.3 (2C-1), 143.9 (Bt C-3a), 132.2 (Dmb C-7), 132.0 (3C-1), 131.3 (3C-2), 129.1 (Bt C-7a), 128.6 (Bt C-6), 125.0 (Bt C-5), 120.3 (Bt C-4), 116.5 (3C-3), 116.4 (Dmb C-2), 109.5 (Bt C-7), 104.6 (Dmb C-6), 99.4 (OCH₂O), 98.8 (Dmb C-4), 80.0 (2C-2), 63.1 (Dmb C-1), 55.9 (Dmb OMe), 55.8 (Dmb OMe), 55.0 (C-2), 37.8 (C-3), 28.4 (2C-3).

### 1.4 Example 4

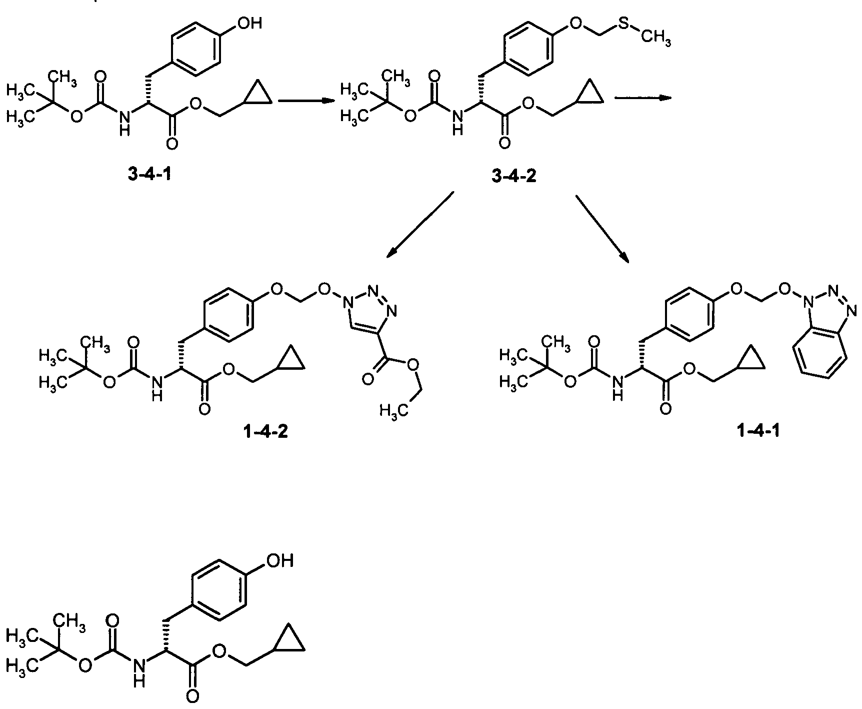

### Cyclopropylmethyl N-(tert-butoxycarbonyl)-D-tyrosinate. 3-4-1

5.00 g (17.8 mmol) Boc-D-tyrosine and 2.90 g (8.89 mmol) caesium carbonate were stirred in 150 ml water for 30 min at r.t. and then lyophilized. The resulting white powder was dissolved in 100 ml *N,N*-dimethylformamide (dried over 4Ǻ molecular sieve), 1,724 ml (17.8 mmol) (Bromomethyl)cyclopropane were added and the mixture stirred at r.t. over night. The mixture was partitioned between ethyl acetate and water, the aqueous phase was extracted with ethyl acetate, the combined organic phases dried over sodium sulfate and the solvent evaporated i. vac.. The residue was dissolved in ethyl acetate and extracted twice with water. After drying and evaporation 5,28 g (89%) **3-4-1** were obtained as a white solid.

¹H NMR (400 MHz, CHLOROFORM-*d*) d ppm 7.00 (d, *J*=8.1 Hz, 2 H, Ar-H) 6.73 (d, *J*=7.8 Hz, 2 H, Ar-H) 5.54 (br. s, 1 H, OH) 5.02 (d, *J*=7.6 Hz, 1 H, NH) 4.48 - 4.61 (m, 1 H, 2-H) 3.88 - 3.99 (m, 2 H, OCH₂) 2.95 - 3.10 (m, 2 H, 3-H₂) 1.42 (s, 9 H, Boc) 1.03 - 1.18 (m, 1 H, cyclopropyl CH) 0.52 - 0.63 (m, 2 H, cyclopropyl CH₂) 0.22 - 0.31 (m, 2 H, cyclopropyl CH₂). Spectrum is identical with an earlier preparation via a different route.

MS (ESI⁺): m/e = 693 (2M + Na⁺), 671 (2M + H⁺), 336 (M + H⁺), 280 (M + H⁺ - C₄H₈), 236 (M + H⁺ - C₄H₈ - CO₂).

¹³C NMR (CHLOROFORM-d ,101 MHz): δ (ppm) 172.2 (C-1), 155.7 (3C-4), 155.2 (2C-1), 130.4 (3C-2), 127.1 (3C-1), 115.5 (3C-3), 79.9 (2C-2), 70.2 (1C-1), 54.6 (C-2), 37.5 (C-3), 28.4 (2C-3), 9.7 (cyclopropyl CH), 3.5, 3.4 (cyclopropyl CH₂).

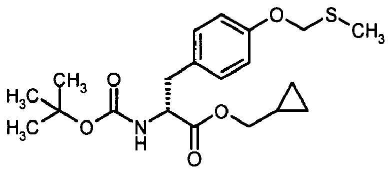

### Cyclopropylmethyl N-(tert-butoxycarbonyl)-O-[(methylsulfanyl)methyl]-D-tyrosinate. 3-4-2

A solution of 1.19 g (3.55 mmol) **3-4-1,** 53 mg (0.36 mmol) sodium iodide and 8 ml *N,N-*dimethylformamide was cooled to 0°C in an ice bath. A suspension of 358 mg (3.19 mmol) potassium *tert*-butylate in 3 ml tetrahydrofuran was added, resulting in a greenish solution. 337 µl (4.08 mmol) chloro dimethyl sulfide were added. The mixture was allowed to come to r.t., stirred for 2 h and stored at 5°C over night. Ethyl acetate was added and the insolubles were filtered off. The filtrate was concentrated i. vac.. Chromatography on a Biotage system (Flash40+M cartridge, 40 ml/min, n-hexane to n-hexane / ethyl acetate 1:4, 15 CV=1980 ml) gave 660 mg (47%) of **3-2-4** and 200 mg (17%) starting material. MS (ESI⁺): m/e = 791 (2M + H⁺), 396 (M + H⁺), 340 (M + H⁺ - C₄H₈), 296 (M + H⁺ - C₄H₈ - CO₂).

¹H NMR (DICHLOROMETHANE-d₂, 400MHz): δ (ppm) 7.09 (d, *J*=8.6 Hz, 2H, Ar-H), 6.87 (d, *J*=8.6 Hz, 2H, Ar-H), 5.13 (s, 2H, OCH₂S), 4.99 (d, *J*=7.8 Hz, 1H, NH), 4.42 - 4.54 (m, 1H, 2-H), 3.87 - 3.99 (m, 2H, OCH₂), 2.95 - 3.12 (mc, 2H, 3-H), 2.22 (s, 3H, SCH₃), 1.40 (s, 9H, Boc), 1.04 - 1.18 (m, 1H, cyclopropyl CH), 0.53 - 0.61 (m, 2H, cyclopropyl CH₂), 0.22 - 0.31 (m, 2H, cyclopropyl CH₂).

¹³C NMR (101 MHz, DICHLOROMETHANE-d₂): δ (ppm) 171.9 (C-2), 156.1 (3C-4), 154.9 (2C-1), 130.4 (3C-2), 129.4 (3C-1), 115.9 (3C-3), 79.5 (2C-2), 72.4 (OCH₂S), 70.1 (1C-1), 54.6 (C-2), 37.4 (C-3), 28.0 (3C-3), 14.3 (SCH₃), 9.6 (1C-2), 3.2 (1 C-3), 3.1 (1C-4).

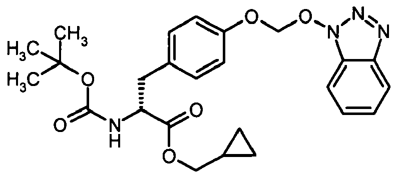

### Cyclopropylmethyl O-[(1H-benzotriazol-1-yloxy)methyl]-N-(tert-butoxycarbonyl) -D-tyrosinate. 1-4-1

650 mg (1.64 mmol) **3-4-2** were reacted as described for **1-2-1.** The reaction mixture was directly applied to Isolute and chromatographed on a Biotage system (Flash40+M cartridge, 40 ml/min, n-hexane to n-hexane / ethyl acetate 1:2 in 15CV = 1980 ml) gave 400 mg (50%) of **1-4-1.** The whole reaction including purification was done in one day. Storage of the raw product adversely affects the yield.

The compound was further purified by preparative HPLC: Dionex: Pump P 580, Gilson: Liquid Handler 215, Knauer: UV-Detector K-2501; Chiralpak IC 5µm 250x20 mm; hexane / ethanol 80:20; 40 ml/min; r.t.; 400 mg / 3.2 ml ethanol; 8 x 0.4 ml, UV 254 nm. The peak at 15.6 - 18.1 min was collected to give 308 mg (39%) of **1-4-1** as white solid with 99,9% purity.

MS (ESI⁺): m/e = 505 (M + Na⁺), 483 (M + H⁺), 427 (M + H⁺ - C₄H₈), 383 (M + H⁺ - C₄H₈ - CO₂).

¹H NMR (DICHLOROMETHANE-d₂ ,400MHz): δ (ppm) 8.03 (dt, J=8.3, 1.0 Hz, 1H, Bt H-7), 7.47 (ddd, J=8.0, 7.0, 1.0 Hz, 1H, Bt 5-H*), 7.41 (ddd, J=8.0, 7.0, 1.0 Hz, 1H, Bt 6-H*), 7.27 (d, br., J=8.0 Hz, 1H, Bt 4-H), 7.25 (d, J=8.5 Hz, 2H, Ar-H), 7.13 (d, J=8.5 Hz, 2H, Ar-H), 6.08 (s, 2H, OCH₂O), 5.11 (d, J=7.5 Hz, 1H, NH), 4.53 - 4.64 (m, 1H, 2-H), 3.94 - 4.07 (mc, 2H, OCH₂), 3.20 (dd, J=14.1, 5.5 Hz, 1H, 3-H), 3.11 (dd, J=13.6, 5.5 Hz, 1H, 3-H), 1.47 (s, 9H, Boc), 1.13 - 1.22 (m, 1H, cyclopropyl CH), 0.59 - 0.66 (m, 2H, cyclopropyl CH₂), 0.31 - 0.37 (m, 2H, cyclopropyl CH₂).

¹³C NMR (DICHLOROMETHANE-d₂ ,101MHz): δ (ppm) 172.2 (C-1), 155.6 (3C-4), 155.3 (2C-1), 143.9 (Bt C-3a), 132.1 (3C-1), 131.3 (3C-2), 129.1 (Bt C-7a), 128.6 (Bt C-6), 125.0 (Bt C-5), 120.3 (Bt C-4), 116.6 (3C-3), 109.4 (Bt C-7), 99.5 (OCH₂O), 80.0 (2C-2), 70.6 (1C-1), 55.0 (C-2), 37.9 (C-3), 28.4 (2C-3), 10.1 (1C-2), 3.6 (1C-3), 3.5 (1 C-4).

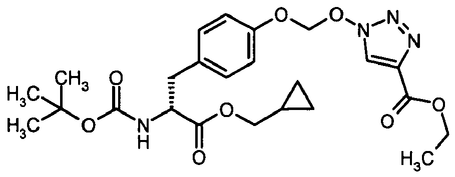

### Cyclopropylmethyl N-(tert-butoxycarbonyl)-O-({[4-(ethoxycarbonyl)-1H-1,2,3-triazol-1-yl]oxy}methyl)-D-tyrosinate. 1-4-2

360 mg (0,91 mmol) **3-4-2** were reacted as described for **1-4-1.** The reaction mixture was directly applied to Isolute and chromatographed on a Biotage system (Flash40+M cartridge, 40 ml/min, n-hexane to n-hexane / ethyl acetate 1:2 in 15CV = 1980ml) gave 230 mg (50%) of **1-4-2.** The compound was further purified by preparative HPLC. Agilent: Prep 1200, 2xPrep Pump, DLA, MWD, Prep FC, ESA: Corona, Chiralpak IC 5 µm 250x20 mm, hexane / ethanol 50:50, 15 ml/min, r.t., 230 mg / 3.5 ml ethanol/methanol 1:1, 7 x 0.5 ml, UV 210 nm. The peak at 7.0 - 8.9 min was collected to give 190 mg (41%) of **1-4-2** with 98,5% purity.

MS (ESI⁺): m/e = 527 (M + Na⁺), 505 (M + H⁺), 449 (M + H⁺ - C₄H₈), 405 (M + H⁺ - C₄H₈ - CO₂).

MS (ESI⁻): m/e = 549 (M + HCOO).

¹H NMR (DICHLOROMETHANE-d₂, 400MHz): δ (ppm) 8.00 (s, 1H, T H-5), 7.19 (d, *J*=8.6 Hz, 2H, Ar-H), 7.06 (d, *J*=8.6 Hz, 2H, Ar-H), 5.90 (s, 2H, OCH₂O), 5.04 (d, *J*=7.8 Hz, 1H, NH), 4.52 (ddd, *J*=7.8, 6.1, 5.6 Hz, 1H, 2-H), 4.36 (q, *J*=7.3 Hz, 2H, T OCH₂), 3.92 (dd, *J*=11.4, 7.6 Hz, 1H, OCH₂), 3.96 (dd, *J*=11.4, 7.3 Hz, 2H, OCH₂), 3.13 (dd, *J*=13.6, 5.6 Hz, 1H, 3-H), 3.04 (dd, *J*=13.6, 6.1 Hz, 1H, 3-H), 1.36 (t, *J*=7.1 Hz, 3H), 1.40 (s, 9H, Boc), 1.08 - 1.16 (m, 1H, cyclopropyl CH), 0.53 - 0.62 (m, 2H, cyclopropyl CH₂), 0.24 - 0.32 (m, 2H, cyclopropyl CH₂).

¹³C NMR (101 MHz, DICHLOROMETHANE-d₂): δ (ppm) 171.7 (C-), 159.9 (T COOEt), 155.0 (3C-4), 154.9 (2C-1), 138.2 (T C-4), 132.2 (3C-1), 131.0 (3C-2), 123.3 (T C-5), 116.3 (3C-3), 99.2 (OCH₂O), 79.6 (2C-2), 70.2 (1C-1), 61.4 (T OCH₂), 54.6 (C-2), 37.4 (C-3), 28.0 (2C-3), 14.0 (T CH₃), 9.7 (cyclopropyl CH), 3.2 (cyclopropyl CH₂), 3.1 (cyclopropyl CH₂).

### Example 5

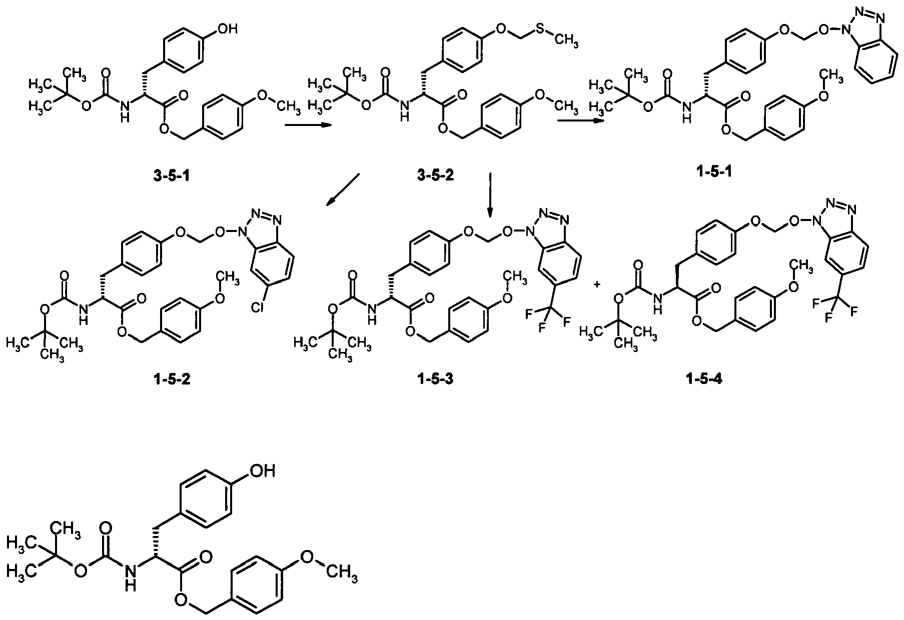

### 4-Methoxybenzyl N-(tert-butoxycarbonyl)-D-tyrosinate. 3-5-1

To 1,763 g (6,27 mmol) Boc-D-Tyr-OH in 52 ml *N,N*-dimethylformamide were added 1,041 g (3,2 mmol) caesium carbonate and the mixture stirred at r.t. for 1,5 h. 1,260 g (6,27 mmol) 4-methoxybenzyl bromide were added and the mixture stirred at r.t. over night. It was diluted with ethyl acetate and water. The pH was adjusted to 5 with 250 µl of 5 % acetic acid. The aqueous phase was separated and extracted with ethyl acetate. The combined extracts were dried and evaporated in the vacuum at 50 °C to yield 2,79 g (100 %) of **3-5-1.**

MS (ES⁺): m/e = 402,53 (M M + H⁺), 803,72 (2M + H⁺).

¹H NMR (CHLOROFORM-d ,400MHz): δ (ppm) 7.24 (d, *J*=8.5 Hz, 2H, Mbn-H), 6.82 - 6.92 (m, 4H, Mbn-H, Ar-H), 6.67 (d, *J*=8.3 Hz, 2H, Ar-H), 5.11 (d, *J*=12.0 Hz, 1H, Mbn 1-H), 5.02 (d, *J*=12.5 Hz, 1H, Mbn 1-H), 4.99 (d, *J*=8.5 Hz, 1H, NH), 4.49 - 4.59 (m, 1H, 2-H), 3.81 (s, 3H, Mbn OCH₃), 2.93 - 3.03 (m, 2H, 3-H), 1.41 (s, 9H, Boc).

¹³C NMR (CHLOROFORM-d ,101 MHz): δ (ppm) 171.9 (C-1), 159.8 (Mbn C-5), 155.2 (3C-4), 155.1 (2C-1), 130.4 (3C-2), 127.4 (3C-1), 127.4 (Mbn C-2), 115.4 (3C-3), 114.0 (Mbn C-4), 80.0 (2C-1), 66.9 (Mbn C-1), 55.3 (Mbn OCH₃), 54.6 (C-3), 37.4 (C-3), 28.3 (2C-3).

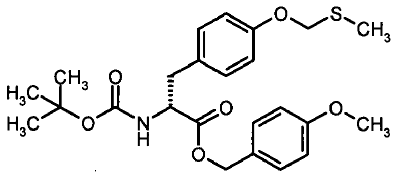

### 4-Methoxybenzyl N-(tert-butoxycarbonyl)-O-[(methylsulfanyl)methyl]-D-tyrosinate. 3-5-2

1,60 g (3,99 mmol) **3-5-1** were dissolved in 32 ml *N,N*-dimethylformamide. 2,60 g (7,97 mmol) caesium carbonate were added and the mixture stirred for 30 min. 0,4 ml (4,78 mmol) Chloromethyl methyl sulfide were added and the mixture stirred at r.t. for 48 h. Further 0,1 ml (1,20 mmol) Chloromethyl methyl sulfide were added and the mixture stirred for 24 h. The solvent was distilled off and the residue distributed between water and ethyl acetate. The organic solvent was extracted with sodium chloride solution, dried and evaporated. The residue was chromatographed on a Biotage system (isolera Four, SNAP 25g, 25 ml/min, n-hexane to n-hexane / ethyl acetate 1:5) to give 682 mg (33 %) **3-5-2.**

MS (ES⁺): m/e = 462,55 (M + H⁺), 923,69 (2M + H⁺).

¹H NMR (CHLOROFORM-d ,300MHz): δ (ppm) 7.25 (d, *J*=8.3 Hz, 2H, Mbn-H), 6.93 (d, *J*=8.3 Hz, 2H, Ar-H), 6.89 (d, *J*=8.7 Hz, 2H, Ar-H), 6.80 (d, *J*=8.5 Hz, 2H, Mbn-H), 5.10 (s, 2H, OCH₂S), 5.12 (d, *J*=12.1 Hz, 1H, Mbn 1-H), 5.03 (d, *J*=11.9 Hz, 1H, Mbn 1-H), 4.96 (d, *J*=8.5 Hz, 1H, NH), 4.48 - 4.63 (m, 1H, 2-H), 3.82 (s, 3H, OCH₃), 2.92 - 3.10 (m, 2H, 3-H), 2.25 (s, 3H, SCH₃), 1.41 (s, 9H, Boc).

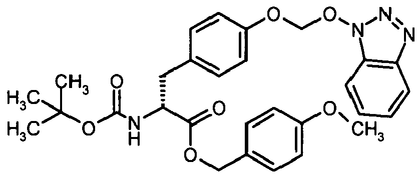

### 4-Methoxybenzyl O-[(1H-benzotriazol-1-yloxy)methyl]-N-(tert-butoxycarbonyl)

### -D-tyrosinate. 1-5-1

520 mg (1,13 mmol) **3-5-2** were reacted as described for **1-2-1.** The reaction mixture was directly applied to Isolute and chromatographed on a Biotage system (Isolera Four, SNAP 50 g, 40 ml/min, n-hexane to n-hexane / ethyl acetate 1:4) to give 492 mg.

The compound was further purified by preparative HPLC. Waters Autopurificationsystem: Pump 254, Sample Manager 2767, CFO, DAD 2996, ELSD 2424, SQD 3001, XBrigde C18 5 µm 150x19 mm, A = water + 0.2% ammonia, B = acetonitrile, 0-1 min 40% B, 1-8 min 40-100% B, 25 ml/min, r.t., 500 mg / 7 ml dimethyl sufoxide/methanol 1:1, 7 x 1 ml, DAD scan range 210-400 nm, MS ESI+, ESI-, scan range 160-1000 m/z, ELSD. The peak at 6.6 - 7.0 min was collected to give 213 mg (33 %) of **1-5-1** as white solid.with >99% purity.

MS (ES⁺): m/e = 549,62 (M + H⁺).

¹H NMR (CHLOROFORM-d ,400MHz): δ (ppm) 8.00 (d, *J*=7.6 Hz, 1H), 7.32 - 7.43 (m, 2H), 7.25 - 7.31 (m, 2H), 7.12 (d, *J*=8.1 Hz, 1H), 6.97 (d, *J*=8.6 Hz, 1H), 6.93 - 7.07 (d, *J*=8.6 Hz, 2H), 6.88 (d, *J*=8.6 Hz, 2H, Bn), 6.01 (s, 2H, OCH₂O) 5.16 (d, *J*=11.4 Hz, 1H), and 5.05 (d, *J*=11.4 Hz, 2H, OCH₂Ar), 5.02 (d, *J*=8.8 Hz, 1H, NH), 4.55 - 4.66 (m, 1H, 2-H), 3.79 (s, 3H. OCH₃), 3.11 (dd, *J*=13.9, 5.8 Hz, 1H, 3-H), 3.03 (dd, *J*=14.1, 5.6 Hz, 1H, 3-H), 1.43 (s, 9H, Boc).

¹³C NMR (CHLOROFORM-d ,101 MHz): δ (ppm) 171.6 (C-1), 159.9 (Mbn C-5), 155.2 (3C-4), 155.1 (2C-1), 143.5 (Bt C-3a), 131.2 (3C-1), 130.9 (3C2), 130.7 (Mbn C-3), 128.8 (Bt C-7a), 128.3 (Bt C-6), 127.3 (Mbn C-2), 124.7 (Bt C-5), 120.1 (Bt C-4), 116.1 (3C-3), 114.0 (Mbn C-4), 109.1 (Bt C-7), 99.0 (OCH₂O), 80.1 (2C-1), 67.1 (Mbn C-1), 55.4 (Mbn C-6), 54.5 (C-2), 37.5 (C-3), 28.4 (2C-3).

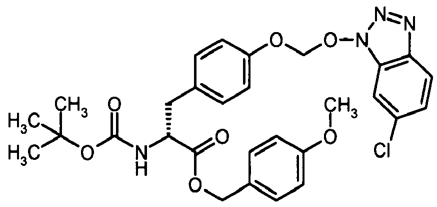

### 4-Methoxybenzyl N-(tert-butoxycarbonyl)-O-{[(6-chloro-1H-benzotriazol-1-yl) oxy]methyl}-D-tyrosinate. 1-5-2

100 mg (0,22 mmol) **3-5-2** were reacted as described for **1-2-1,** where the 1*H-*benzotriazol-1-ol hydrate was replaced by 6-chloro-1*H*-benzotriazol-1-ol. The reaction mixture was directly applied to Isolute and chromatographed on a Biotage system (Isolera Four, SNAP 10 g, 12 ml/min, n-hexane to n-hexane / ethyl acetate 4:1) to give 75 mg.

MS (ESI⁺): m/e = 583,17 (M + H⁺).

MS (ESI⁻): m/e = 627,10 (M + HCOO).

¹H NMR (CHLOROFORM-d ,300MHz): δ (ppm) 7.92 (dd, *J*=8.9, 0.4 Hz, 1H Bt 4-h), 7.31 (dd, *J*=8.9, 1.9 Hz, 1H, Bt 5-H), 7.28 (d, *J*=8.5 Hz, 2H, PMB 2-H), 7.03 (d, *J*=8.7 Hz, 2H, Ar 2-H), 7.06 (br. s., 1H, Bt 7-H), 6.94 (d, *J*=8.5 Hz, 2H, PMB 3-H), 6.87 (d, *J*=8.7 Hz, 2H, Ar 3-H), 5.96 - 6.04 (m, 2H, OCH₂O), 5.15 (d, *J*=11.7 Hz, 1H, PMB CH₂), 5.05 (d, *J*=12.1 Hz, 1H, PMB CH₂), 5.01 (br. s., 1H, NH), 4.53 - 4.66 (m, 1H, 2-H), 3.79 (s, 3H, PMB OMe), 3.08 (br. s., 2H, 3-H), 1.43 (s, 9H, Boc).

¹³C NMR (CHLOROFORM-d ,75MHz): δ (ppm) 171.6 (C-1), 159.8 (1C-5), 155.0 (2C-1), 154.8 (3C-4), 142.0 (Bt C-7a), 134.8 (Bt C-3a), 131.4 (3C-1), 131.0 (1C-2), 130.6 (3C-2), 129.3 (1C-3), 127.3 (Bt C-6), 126.0 (Bt C-5), 121.1 (Bt C-7), 116.0 (3C-3), 113.9 (1C-4), 108.9 (Bt C-4), 98.8 (OCH₂O), 80.0 (2C-2), 67.1 (1C-1), 55.3 (OCH₃), 54.4 (C-2), 37.4 (C-3), 28.3 (2C-3).

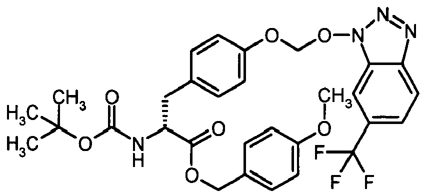

### 4-Methoxybenzyl O-[(6-trifluoromethyl-1H-benzotriazol-1-yloxy)methyl]-N-(tert-butoxycarbonyl)-D-tyrosinate. 1-5-3

150 mg (0,33 mmol) **3-5-2** were reacted as described for **1-2-1,** where the 1*H-*benzotriazol-1-ol hydrate was replaced by 6-trifluoromethyl-1*H*-benzotriazol-1-ol. The reaction mixture was directly applied to Isolute and chromatographed on a Biotage system (Isolera Four, SNAP 25 g, 25 ml/min, n-hexane 1 CV, n-hexane to n-hexane / ethyl acetate 6:4 10CV, then isokratic 4CV) to give 221 mg (>100%). The material was further purified by HPLC: (Dionex: Pump P 580, Gilson: Liquid Handler 215, Knauer: UV-Detector K-2501; Chiralpak IC 5µm 250x30 mm; Hexan / Ethanol 80:20; 40 ml/min; RT; 221 mg / 3 ml EtOH / Dichlormethan 1:1; 6 x 500 ml; UV 210 nm). Two peaks, 13.1 - 14.1 min (77mg (38%), 99.5 % purity, **1-5-3)** and 14.1 - 15.5 min (48 mg (22%), 93.2 % purity, **1-5-4)** were collected.

Both peaks had the same mass. The stereochemistry was putatively assigned in comparison with **1-2-1** and **1-2-2.**

MS (ESI⁺): m/e = 639 (M + Na⁺), 617 (M + H⁺), 561 (M + H⁺ -C₄H₈), 121 (C₈H₉O⁺).

¹H NMR (DICHLOROMETHANE-d₂, 400MHz): δ (ppm) 8.13 (d, *J*=8.6 Hz, 1H, Bt 4-H), 7.59 (dd, *J*=8.8, 1.3 Hz, 1H, Bt 5-H), 7.48 (s, 1H, Bt 7-H), 7.24 - 7.32 (m, 2H, Mbn 3-H), 7.07 (d, *J*=8.3 Hz, 2H, Ar-H), 6.98 (d, *J*=8.6 Hz, 2H, Ar-H), 6.83 - 6.92 (m, 2H, Mbn 4-H), 6.01 - 6.09 (m, 2H, OCH₂O), 5.12 (d, *J*=11.9 Hz, 1H, Mbn 1-H), 5.06 (d, *J*=11.9 Hz, 1H, Mbn 1-H), 5.02 (d, *J*=7.8 Hz, 1H, NH), 4.54 (dt, *J*=7.8, 5.8 Hz, 1H, 2-H), 3.77 (s, 1H, OMe), 3.11 (dd, *J*=13.9, 5.8 Hz, 1H, 3-H), 3.03 (dd, *J*=13.9, 5.8 Hz, 1H, 3H), 1.40 (s, 9H, Boc).

¹⁹F NMR (DICHLOROMETHANE-d₂ ,376MHz): δ (ppm) -62,3.

¹³C NMR (101 MHz, DICHLOROMETHANE-*d*₂) δ ppm 171.6 (C-1), 159.9 (Mbn C-5), 155.0 (br., 2C1), 154.6 (3C-4), 144.6 (Bt C-3a), 131.8 (3C-1), 131.0 (3C-2), 130.5 (Mbn C-3) 130.1 (q, ²J_{CF}=32.8 Hz, Bt C-6), 128.2 (Bt C-7a), 127.5 (Mbn C-2), 123.7 (q, ¹J_{CF} =272.4 Hz, CF₃), 121.2 (Bt C-4, Bt C-5), 115.9 (3C-3) 113.8 (Mbn C-4), 107.8 (q, ³J_{CF} =4.8 Hz, Bt C-7), 99.0 (OCH₂O), 79.6 (2C-2) ,66.9 (Mbn C-1) ,55.2 (Mbn OMe), 54.6 (C-2), 37.2 (C-3), 28.0 (2C-3).

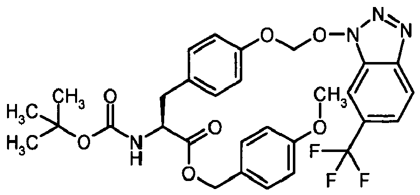

### 4-Methoxybenzyl O-[(6-trifluoromethyl-1H-benzotriazol-1-yloxy)methyl]-N-(tert-butoxycarbonyl)-L-tyrosinate. 1-5-4

The compound can be prepared in analogy to **1-5-3** from L-tyrosine. In the event it was isolated from the preparation of **1-5-4,** where a partial racemisation had occurred during the synthesis or stereochemically impure tyrosine had been used.

MS (ESI⁺): m/e = 639 (M + Na⁺), 617 (M + H⁺), 561 (M + H⁺ -C₄H₈), 121 (C₈H₉O⁺).

¹H NMR (DICHLOROMETHANE-d₂ ,400MHz): δ (ppm) 8.13 (d, *J*=8.8 Hz, 1H, Bt 4-H), 7.59 (dd, *J*=8.8, 1.3 Hz, 1H, Bt 5-H), 7.48 (s, 1H, Bt 7-H), 7.28 (d, *J*=8.6 Hz, 2H, Mbn 3-H), 7.07 (d, *J*=8.3 Hz, 2H, Ar-H), 6.98 (d, *J*=8.6 Hz, 2H, Ar-H), 6.84 - 6.90 (m, 2H, Mbn 4-H), 6.02 - 6.08 (m, 2H, OCH₂O), 5.12 (d, *J*=11.6 Hz, 1H, Mbn 1-H), 5.06 (d, *J*=11.6 Hz, 1H, Mbn 1-H), 5.02 (d, *J*=8.1 Hz, 1H, NH), 4.54 (dt, *J*=8.1, 5.8 Hz, 1H, 2-H), 3.77 (s, 3H, Mbn OMe), 3.11 (dd, *J*=13.9, 5.8 Hz, 1H, 3-H), 3.03 (dd, *J*=13.6, 5.8 Hz, 1H, 3-H), 1.40 (s, 9H, Boc).

¹⁹F NMR (DICHLOROMETHANE-d₂ ,376MHz): δ (ppm) -62.3.

¹³C NMR (DICHLOROMETHANE-d2, 101MHz): δ ppm 171.6 (C-1), 159.9 (Mbn C-5), 155.0 (2C1), 154.6 (3C-4), 144.6 (Bt C-3a), 131.8 (3C-1), 131.0 (3C-2), 130.5 (Mbn C-3), 130.1 (q, ²J_{CF} =32.8 Hz, Bt C-6), 128.2 (Bt C-7a), 127.5 (Mbn C5), 123.7 (q, ¹J_{CF} =273.2 Hz, CF₃), 121.2 (Bt C-4, Bt C-5), 115.9 (3C-3), 113.8 (MBn C-4), 107.8 (q, ³J_{CF} =4.8 Hz, Bt C-7), 99.0 (OCH₂O) 79.7 (2C-2), 66.9 (Mbn C-1), 55.2 (Mbn OMe), 54.6 (C-2), 37.2 (C-3) 28.0 (2C-3).

### Example 6

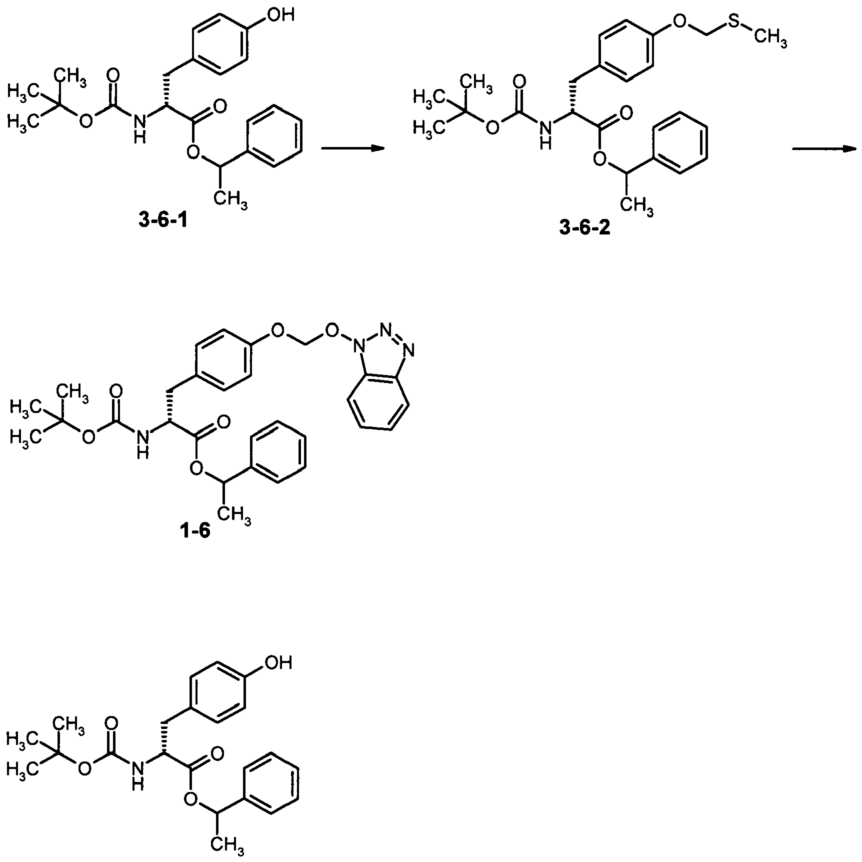

### alpha-Methylbenzyl N-(tert-butoxycarbonyl)-D-tyrosinate. 3-6-1

To 500 mg (1,78 mmol) Boc-D-Tyr-OH in 15 ml *N,N*-dimethylformamide were added 295,4 mg (0,91 mmol) caesium carbonate and the mixture stirred at r.t. for 0,5 h. 328,9 mg (1,78 mmol) 1-phenylethyl bromide were added and the mixture stirred at r.t. over night. The mixture was evaporated in the vacuum at 50°C. The residue was dissolved in ethyl acetate and water. The aqueous phase was separated and extracted with ethyl acetate. The combined extracts were dried and evaporated in the vacuum at 50 °C to yield 725 mg (106 %) of **3-6-1** as mixture of diastereomers.

MS (ES+): m/e = 386,55 (M + H⁺), 771,71 (2M + H⁺).

¹H-NMR (400MHz, CHLOROFORM-d): δ [ppm]= 1.37 - 1.47 (m, 9H), 1.51 (d, 1.5H), 1.57 (d, 1.5H), 3.02 - 3.13 (m, 2H), 4.48 - 4.63 (m, 1H), 4.84 - 5.05 (m, 1H), 5.82 - 5.99 (m, 1H), 6.55 - 6.66 (m, 1H), 6.68 - 6.84 (m, 2H), 7.00 (m, 1H), 7.29 - 7.41 (m, 5H).

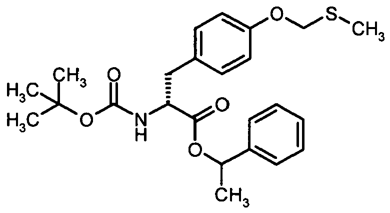

### alpha-Methylbenzyl N-(tert-butoxycarbonyl)-O-[(methylsulfanyl)methyl]-D-tyrosinate. 3-6-2

101 mg (0,26 mmol) **3-6-1** and 4,05 mg (0,03 mmol) sodium iodide were dissolved in 2 ml N,N-dimethylformamide and cooled for 10 minutes with ice. 0,30 ml (0,30 mmol) potassium tert-butoxide, 1,0M in tetrahydrofuran were added and the mixture stirred for 60 min. 0,03 ml (0,30 mmol) chloromethyl methyl sulfide were added and the mixture stirred at r.t. for 48 h and at 66 °C for 5 h. The mixture was diluted with ethyl acetate, extracted with sodium chloride solution, dried and evaporated. The residue was chromatographed ( SNAP 5g, n-hexane / ethyl acetate 85:15) to give 45 mg (27 %) **3-6-2** as mixture of diastereomers.

MS (ES⁺): m/e = 446,54 (M + H⁺), 891,69 (2M + H⁺).

¹H-NMR (400MHz, CHLOROFORM-d): δ [ppm]= 1.38 - 1.47 (m, 9H), 1.51 (m, 1.5H), 1.55 - 1.60 (m, 1.5H), 2.24 - 2.26 (m, 3H), 3.02 - 3.13 (m, 2H), 4.58 (m, 1H), 4.90 - 5.03 (m, 1H), 5.13 (s, 2H), 5.84 - 5.97 (m, 1H), 6.70 - 6.83 (m, 1H), 6.84 - 6.93 (m, 1H), 7.04 - 7.16 (m, 1 H), 7.28 - 7.42 (m, 6H).

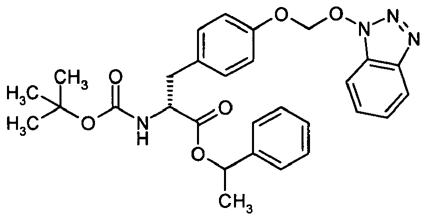

### alpha-Methylbenzyl O-[(1H-benzotriazol-1-yloxy)methyl]-N-(tert-butoxycarbonyl) -D-tyrosinate. 1-6

120 mg (0,27 mmol) **3-6-2** were reacted as described for **1-2-1.** The reaction mixture was directly applied to Isolute and chromatographed on a Biotage system (Isolera Four, SNAP 10 g, n-hexane to n-hexane / ethyl acetate 1:4) to give 55 mg (34,5 %) **1-6** as mixture of diastereomers.

MS (ES⁺): m/e = 533,64 (M + H⁺).

¹H NMR (CHLOROFORM-d ,300MHz): δ (ppm) 7.96 - 8.04 (m, 1H), 7.28 - 7.43 (m, 9H), 7.00 - 7.21 (m, 4H), 6.81 - 6.93 (m, 2H, Ar-H), 5.83 - 6.09 (m, 3H, OCH₂O, OCH), 4.91 - 5.09 (m, 1H, NH), 4.51 - 4.68 (m, 1H, 2-H), 2.89 - 3.23 (m, 2H, 3-H), 1.53 und 1.59 (d, *J*=6.8 Hz, CH₃), 1.43 (s, 9H).

¹³C NMR (CHLOROFORM-d ,75MHz): δ (ppm) 171.0, 171.0 (C-1), 1551, 155.1 (3C-4), 155.0 (2C-1), 143.5 (Bt C-3a), 140.8, 140.6 (1 ipso), 131.4, 131.0 (3C-1), 130.9, 130.9 (3C-2), 128.8 (Bt C-3a), 128.6, 128.6 (1 meta), 128.3, 128.3 (1 para), 128.2 (Bt C-6), 126.6,126.1 (1 ortho), 124.7 (Bt C-5), 120.1, 120.1 (Bt C-4), 116.1, 116.0 (3C-3), 109.1, 109.0 (Bt C-7), 99.0, 98.9 (OCH₂O), 80.0 (2C-2), 73.7, 73.7 (1C-1), 54.6, 54.3 (C-2), 37.7, 37.3 (C-3), 28.3 (2C-3), 22.1, 21.9 (1 CH₃).

### Example 7

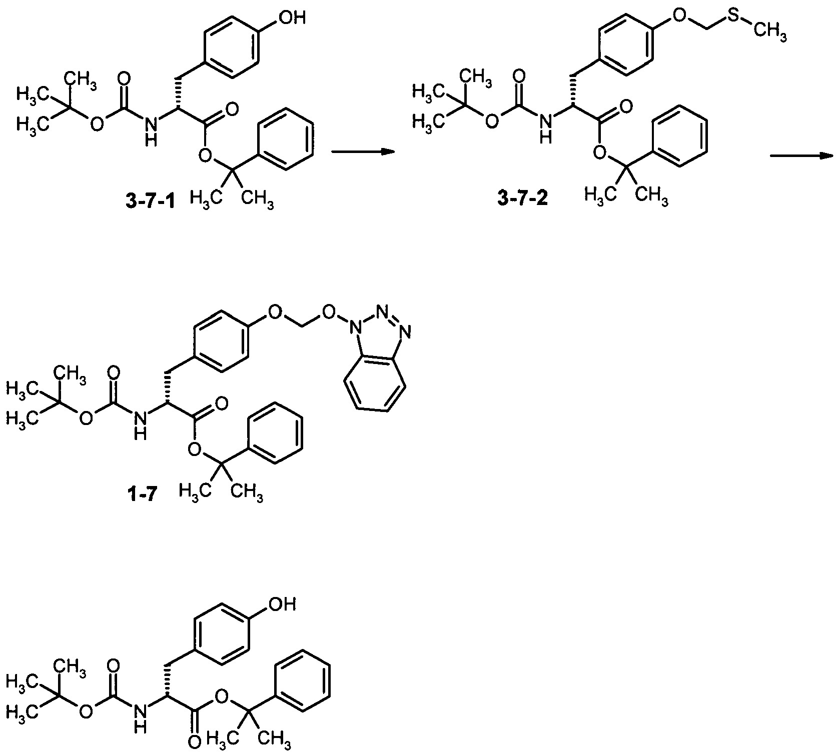

### alpha,alpha-Dimethylbenzyl N-(tert-butoxycarbonyl)-D-tyrosinate. 3-7-1

To 511,3 mg (1,82 mmol) Boc-D-Tyr-OH in 51 ml dichloromethane were added 1020,0 mg (3,6 mmol) 2-phenylisopropyltrichloroacetamidate (Tetrahedron Lett. 1993, 34, 323-326; WO 2008/048970*,* 66) in 10 ml cyclohexane. After stirring for 20 h, the mixture was concentrated and separated by chromatography (10 g, n-hexane to n-hexane / ethyl acetate 2:3) to give 772 mg (106 %) **3-7-1.**

MS (ES⁺): m/e = 400,54 (M + H⁺).

¹H-NMR (300MHz, CHLOROFORM-d): δ [ppm]= 1.42 (s, 9H), 1.60 (s, 3H), 1.70 (s, 3H), 2.92 - 3.05 (m, 2H), 4.42 - 4.55 (m, 1H), 4.85 - 4.97 (m, 1H), 6.75 (d, 2H), 7.04 (d, 2H), 7.28 - 7.34 (m, 5H).

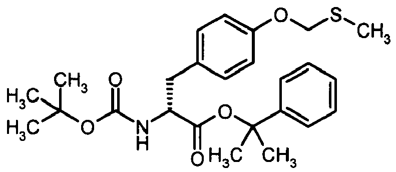

### alpha,alpha-Dimethylbenzyl N-(tert-butoxycarbonyl)-O-[(methylsulfanyl)methyl)] -D-tyrosinate. 3-7-2

A solution of 1,228 g (3,07 mmol) **3-7-1,** 47 mg (0.32 mmol) sodium iodide and 7 ml *N,N-*dimethylformamide was cooled to 0°C in an ice bath. A suspension of 388 mg (3,46 mmol) potassium tert-butylate in 3 ml tetrahydrofuran was added. 293 µl (3,54 mmol) chloro dimethyl sulfide were added. The mixture was allowed to come to r.t. and stirred for 3 h. ethyl acetate was added. The mixture was extracted with water and sodium chloride solution, dried and concentrated in the vacuum. Chromatography over 10 g basic silicagel (n-hexane to n-hexane / ethyl acetate 2:3) gave 139 mg (8%) of **3-7-2** and 208 mg (14%) starting material.

MS (Cl⁺): m/e = 477,61 (M + NH₄⁺), 936,71 (2M + NH₄⁺).

¹H-NMR (300MHz, CHLOROFORM-d): δ [ppm]= 1.42 (s, 9H), 1.73 (m, 3H), 1.76 (m, 3H), 2.26 (s, 3H), 2.98 - 3.12 (m, 2H), 4.49 (d, 1H), 5.13 (s, 2H), 6.88 (d, 2H), 7.11 (d, 2H), 7.30 - 7.37 (m, 5H).

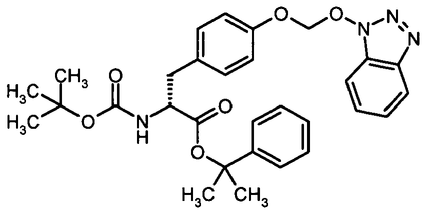

### alpha,alpha-Dimethylbenzyl O-[(1H-benzothazol-1-yloxy)methyl]-N-(tert-butoxy-carbonyl)-D-tyrosinate. 1-7

120 mg (0,26 mmol) **3-7-2** were reacted as described for 1-2-1. The reaction mixture was directly applied to Isolute and chromatographed on a Biotage system (Isolera Four, SNAP 10 g, n-hexane to n-hexane / ethyl acetate 1:4) to give 35 mg (23,3 %) **1-7.**

MS (ES⁺): m/e = 547,36 (M + H⁺).

¹H NMR (CHLOROFORM-d, 300MHz): δ (ppm) 7.95 - 8.04 (m, 1H), 7.11 - 7.41 (m, 11H), 7.06 (d, *J*=8.7 Hz, 2H, Ar-H), 6.03 (s, 2H, OCH₂O) 4.98 (d, *J*=8.1 Hz, 2H. NH), 4.47 - 4.63 (m, 1H, 2-H), 3.15 (dd, *J*=13.9, 6.4 Hz, 1H, 3-H), 3.04 (dd, *J*=13.9, 6.0 Hz, 1H), 3-H, 1.78 (s, 3H, CH₃), 1.75 (s, 3H, CH₃), 1.42 (s, 9H, Boc).

¹³C NMR (CHLOROFORM-d ,75MHz): δ (ppm) 170.4 (C-1), 155.2 (3C-4), 155.1 (2C-1), 144.9 (Cu ipso), 143.5 (Bt C-3a), 131.6 (3C-1), 131.0 (3C-2), 128.8 (Bt C-7a), 128.3 (Cu meta), 128.3 (Bt C-6), 127.3 (Cu para), 124.7 (Bt C-5), 124.4 (Cu ortho), 120.1 (Bt C-4), 116.0 (3C-3), 109.0 (Bt C-7), 99.0 (OCH₂O) 83.4 (Cu C), 79.9 (2C-2), 54.8 (C-2), 37.6 (C-3), 28.8 (Cu CH₃), 28.3 (2C-3), 27.9 (Cu CH₃).

### 1.8 Example 8

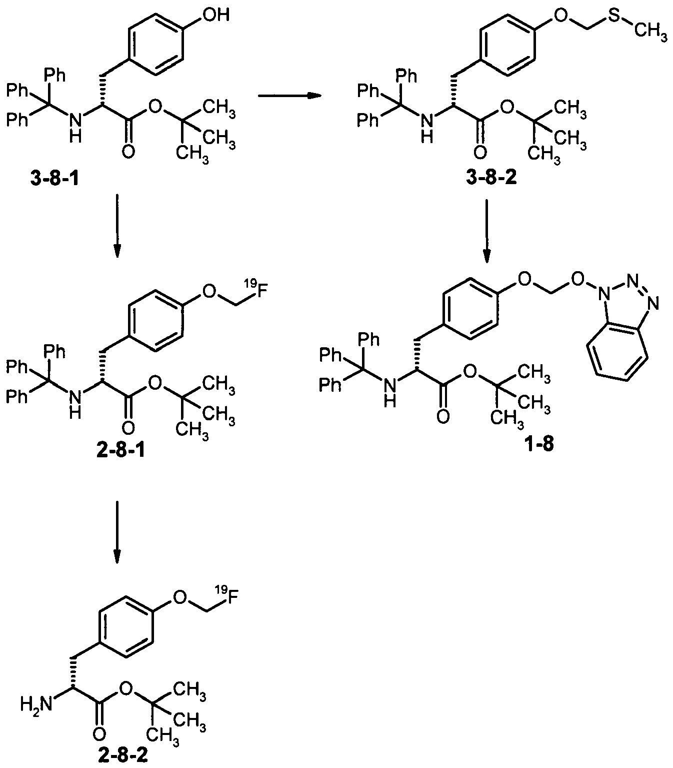

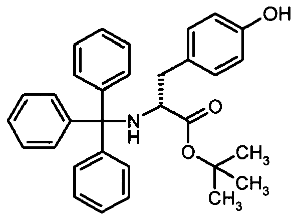

### tert-Butyl N-trityl-D-tyrosinate. 3-8-1

This compound was synthesized as described (Journal of Labelled Compounds and Radiopharmaceuticals 2004; 47, 477-483) using D-Tyrosine.

MS (ES⁺): m/e = 243,47 (Ph₃C⁺).

MS (ES-): m/e = 524,66 (M + HCOO⁻), 957,71 (2M - H⁺).

¹H NMR (DMSO-d6 ,300MHz): δ (ppm) 9.16 (s, 1H, OH), 7.29 - 7.37 (m, 6H), 7.18 - 7.27 (m, 6H), 7.10 - 7.18 (m, 3H), 6.83 (d, J=8.3 Hz, 2H), 6.60 (d, J=8.5 Hz, 2H), 3.09 - 3.21 (m, 1H, 2-H), 2.64 (d, J=9.2 Hz, 1H, NH), 2.56 (dd, J=13.6, 7.7 Hz, 1H, 3-H), 2.36 (dd, J=13.6, 6.0 Hz, 1H, 3-H), 1.01 (s, 9H, OtBu).

¹³C NMR (101 MHz, CHLOROFORM-d): δ (ppm) 173.9 (C-1), 154.4 (3C-4), 146.4 (Tr C-2), 131.2 (3C-2), 129.8 (3C-1), 128.9 (Tr C-3), 127.8 (Tr C-4), 126.4 (Tr C-5), 114.9 (3C-3), 80.5 (1C-1), 71.2 (Tr C-1), 58.2 (C-2), 41.3 (C-3), 27.9 (1 C-2).

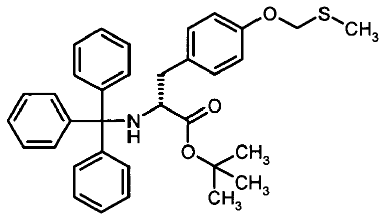

### tert-Butyl O-[(methylsulfanyl)methyl]-N-trityl-D-tyrosinate. 3-8-2

A solution of 1,20 g (2,50 mmol) **3-8-1**, 39 mg (0.26 mmol) sodium iodide and 5,5 ml *N,N-*dimethylformamide was cooled to 0°C in an ice bath. A suspension of 365 mg (3,25 mmol) potassium *tert*-butylate in 3 ml tetrahydrofuran was added. After 10 min 238 µl (2,89 mmol) chloro dimethyl sulfide were added. The mixture was allowed to come to r.t. and stirred for 20 h. Ethyl acetate was added. The mixture was extracted with water, dried and concentrated in the vacuum. This gave 1,46 g (92%) of **3-8-2** with 85% purity.

MS (ES⁺): m/e = 243,47 (Ph₃C⁺), 540,66 (M + H⁺).

¹H NMR (CHLOROFORM-d ,400MHz): δ (ppm) 7.41 - 7.48 (m, 6 H), 7.18 - 7.25 (m, 6 H), 7.11 - 7.18 (m, 5 H), 6.88 (d, *J*=8.6 Hz, 2 H), 5.13 (s, 2 H, OCH₂S), 3.42 - 3.52 (m, 1 H, 2-H), 2.83 (dd, *J*=13.4, 6.8 Hz, 1 H, 3-H), 2.76 (dd, *J*=13.4, 6.1 Hz, 1 H, 3-H), 2.58 (d, *J*=7.6 Hz, 1 H, NH), 2.24 (s, 3 H, SCH₃), 1.06 ppm (s, 9 H, tBu).

¹³C NMR (CHLOROFORM-d, 75 MHz,): δ (ppm) 173.7 (C-1), 155.7 (3C-4), 146.3 (Tr C-2), 131.1 (3C-1), 131.0 (3C-2), 128.8 (Tr C-3), 127.8 (Tr C-4), 126.3 (Tr C-5), 115.6 (3C-3), 80.4 (1C-1), 72.5 (SCH₂O) 71.2 (Tr C-1), 58.1 (C-2), 41.3 (C-3), 27.8 (1C-2), 14.5 (SCH₃).

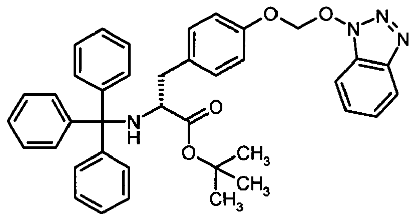

### tert-Butyl O-[(1H-benzotriazol-1-yloxy)methyl]-N-trityl-D-tyrosinate. 1-8

240 mg (0,45 mmol) **3-8-2** were reacted as described for **1-2-1.** The reaction mixture was directly applied to Isolute and chromatographed on a Biotage system (Isolera Four, SNAP 25 g, n-hexane to n-hexane / ethyl acetate 1:4) to give 65 mg (23,3 %) **1-8.**

Waters Autopurificationsystem: Pump 254, Sample Manager 2767, CFO, DAD 2996, ELSD 2424, SQD 3001, Luna C18(2) 5 µm 150x21.2 mm, A = water + 0.1 % formic acid, B = acetonitrile, 0-1 min 70% B, 1-12 min 70-100% B, 25 ml/min, r.t., 54 mg / 1 ml dimethyl sulfoxide/methanol 1:1, 1 x 1 ml, DAD scan range 210-400 nm, MS ESI+, ESI-, scan range 160-1000 m/z, ELSD. The peak at 13.0 - 13.2 min was collected to give 12 mg (3,9%) of **1-8** with 97.4 % purity.

MS (ES⁺): m/e = 243,47 (Ph₃C⁺), 627,63 (M + H⁺).

¹H NMR (CHLOROFORM-d ,400MHz): δ (ppm) 7.99 (d, *J*=8.1 Hz, 1H, Bt-H), 7.47 (d, *J*=7.1 Hz, 6H, Tr-H), 7.28 - 7.34 (m, 2H, Bt-H), 7.21 - 7.24 (m, 6H, Tr-H), 7.13 - 7.20 (m, 4H, Bt-H, Ar-H), 7.09 (d, *J*=8.6 Hz, 2H, Ar-H), 6.01 - 6.08 (m, 2H, OCH₂O), 3.54 (dd, *J*=7.1, 5.8 Hz, 1H. 2-H), 2.90 (dd, *J*=13.4, 7.1 Hz, 1H, 3-H), 2.82 (dd, *J*=13.9, 5.8 Hz, 1H, 3-H), 2.62 (br. s, 1H, NH), 1.11 (s, 9H, *t*Bu-H).

¹³C NMR (DICHLOROMETHANE-d2 ,151MHz): δ = 173.6 (C-1), 155.5 (3C-4), 146.8 (Tr C-2), 143.9 (Bt C-3a), 133.6 (3C-1), 131.9 (3C-2), 129.2 (Tr C-3), 129.1 (Bt C-7a), 128.6 (Bt C-6), 128.2 (Tr C-4, 126.8 (Tr C-5), 125.0 (Bt C-5), 120.2 (Bt C-4), 116.1 (3C-3), 109.5 (Bt C-7), 99.7 (OCH₂O) 80.8 (1C-1), 71.7 (Tr C-1), 58.4 (C-2), 41.4 (C-3), 28.1 (1C-2).

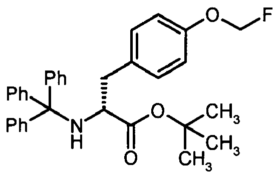

### tert-Butyl O-(fluoromethyl)-N-trityl-D-tyrosinate. 2-8-1

To 200,0 mg (0,42 mmol) **3-8-1** in 4 ml *N,N*-dimethylformamide cooled to 5 °C were added 16,7 mg (0,42 mmol) sodium hydride (60%). The mixture was stirred for 30 min at 5-10 °C. A solution of 167 mg (1,48 mmol) bromofluoromethane in 4 ml *N,N*-dimethylformamide was added and the mixture stirred for 2 h at 5-10 °C and 2 h at r.t.. The mixture was partitioned between dichloromethane and water, the aqueous phase was extracted with dichloromethane, the combined organic phases dried and the solvent evaporated to give 214 mg (90%) **2-8-1.**

MS (ES⁺): m/e = 243,47 (Ph₃C⁺) only.

¹⁹F NMR (376 MHz, CHLOROFORM-d): δ (ppm) -148.02 (t, *J*=55.1 Hz).

¹H NMR (CHLOROFORM-d ,300MHz): δ (ppm) 7.44 (d, *J*=7.2 Hz, 6H), 7.11 - 7.25 (m, 11H), 7.00 (d, *J*=8.5 Hz, 2H), 5.69 (d, ¹*J_{HF}=55.0* Hz, 2H), 3.42 - 3.55 (m, 1H, 2-H), 2.85 (dd, *J*=13.9, 6.4 Hz, 1H, 3-H), 2.78 (dd, *J*=13.6, 5.8 Hz, 1H, 3-H), 2.52 - 2.64 (m, 1H, NH), 1.07 (s, 9H, tBu).

¹³C NMR (101 MHz, CHLOROFORM-*_{d}*) δ ppm 173.6 (C-1), 162.6 (s, 1 C), 155.6 (d, ³*J_{CF}*=2.4 Hz, 3C-4), 146.3 (Tr C2), 133.0 (3C-1), 131.3 (3 C-2), 128.8 (Tr C-3), 127.9 (Tr C4), 126.4 (Tr C-5), 116.4 (3C-3), 101.0 (d, *¹J_{CF}*=218.9 Hz, OCH₂F), 80.6 (1C-1), 71.3 (Tr C-1), 58.0 (C-2), 41.3 (C-3), 36.5 (s, 1 C), 29.8 (s, 1 C), 27.9 (1 C-2).

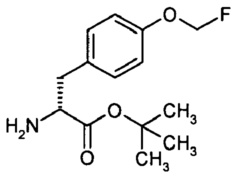

### tert-Butyl O-(fluoromethyl)-D-tyrosinate. 2-8-2

To a solution of 80 mg (0,16 mmol) **2-8-1** in 0,8 ml acetic acid were added 0,2 ml water and the solution stirred for 2 h at r.t.. Water was added and the precipitate was filtered off. The filtrate was neutralized with sodium hydrogen-carbonate solution and extracted with ethyl acetate. The combined organic phases were dried over sodium sulfate and the solvent evaporated to give 30 mg (71%) **2-8-2.**

Agilent: Prep 1200, 2 x Prep Pump, DLA, MWD, Prep FC, XBrigde C18 5 µm 150x19 mm, A = water + 0.2% ammonia, B = methanol, 0-1 min 10% B, 1-8 min 10-80% B, 8-8.1 min 80-100% B, 8.1-10 min 100% B, 25 ml/min, r.t., 30 mg / 1 ml dimethyl sulfoxide/methanol 1:1, 1 x 1 ml, UV 219 nm. The Peak at 5 - 1.33 min was collected to give 17 mg (36%) of **2-8-2** with 99,3 % purity.

MS (ES+): m/e = 214,42 (M + H⁺ - C₄H₈), 270,51 (M + H⁺), 539,62 (2M + H⁺).

¹⁹F NMR (376 MHz, CHLOROFORM-d): δ (ppm) -148.29 (t, J=53.9 Hz).

¹H NMR (CHLOROFORM-d ,400MHz): δ (ppm) 7.10 (d, J=8.5 Hz, 2H, Ar-H), 6.94 (d, J=8.5 Hz, 2H, Ar-H), 5.61 (d, ²J_{HF}=54.7 Hz, 2H), 3.50 (dd, J=7.5, 5.5 Hz, 1H, 2-H), 2.92 (dd, J=13.6, 5.8 Hz, 1H, 3-H), 2.74 (dd, J=13.8, 7.5 Hz, 1H, 3-H), 1.65 (br. s, 2H, NH), 1.36 (s, 9H, tBu-H).

¹³C NMR (101 MHz, CHLOROFORM-d) δ (ppm) 174.25 (C-1) 155.67 (3 C-4) 155.64 (2C-1) 132.71 (3 -1 C) 130.60 (3 C-2) 116.70 (d, ⁴J_{CF}=1.3 Hz, 3 C-3),100.88 (d, ¹J_{CF}=218.9 Hz, OCH₂O 81.21 (1C-1) 56.32 (C-2) 40.32 (C-3) 28.02 (1C-2).

### 1.9 Example 9

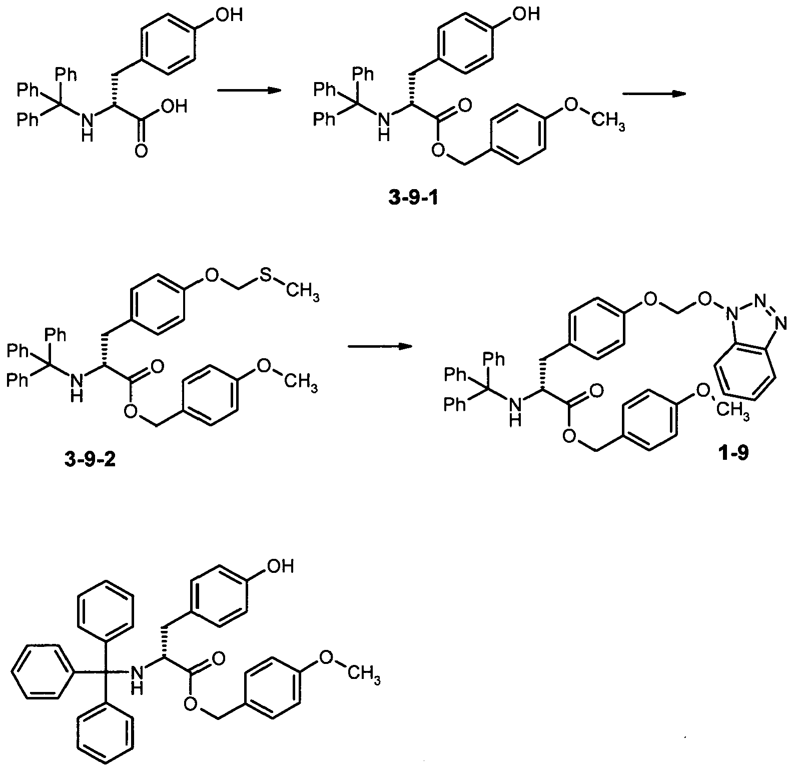

### 4-Methoxybenzyl N-trityl-D-tyrosinate. 3-9-1

265 mg (0,53 mmol) *N*-trityl-D-tyrosine (Liebigs Ann. Chem. 1988, 1083-1084) were dissolved in 4,4 ml *N,N*-dimethylformamide. 89 mg (0,27 mmol) caesium carbonate were added and the mixture stirred for 30 min. 107 mg (0,54 mmol) 4-methoxybenzyl bromide were added and the mixture stirred for 16 h. Further 54 mg (0,27 mmol) 4-methoxybenzyl bromide were added and the mixture stirred at 40 °C for 4 h. The mixture was diluted with ethyl acetate and extracted with water. The aqueous phase was neutralized with acetic acid to pH 5 and extracted with ethyl acetate. The organic solutions were combined, dried over sodium sulfate and concentrated. The residue was purified by chromatography over 10 g silica gel with hexane/ethyl acetate 100 - 80/20 - 60/40 to give 215 mg.

MS (ESI⁺): m/e = 544,33 (M + H⁺).

MS (ESI⁻): m/e = 588,18 (M + HCOO).

¹H-NMR (400MHz, CHLOROFORM-d): δ [ppm]= 2.59 - 2.69 (m, 1H), 2.84 (dd, 1H), 2.94 (dd, 1H), 3.51 - 3.61 (m, 1H), 3.81 (s, 3H), 4.20 (d, 1H), 4.42 (d, 1H), 4.92 (br. s., 1H), 6.68 - 6.73 (m, 2H), 6.79 - 6.84 (m, 2H), 6.91 - 7.02 (m, 4H), 7.13 - 7.26 (m, 9H), 7.40 - 7.49 (m, 6H).

¹³C-NMR (101MHz, CHLOROFORM-d): δ [ppm]= 41.4, 55.3, 58.3, 66.1, 71.0, 113.7, 115.0, 126.3, 127.5, 127.9, 128.8, 129.4, 130.0, 130.9, 145.9, 154.4, 159.5, 174.4.

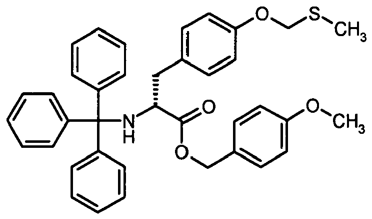

### 4-Methoxybenzyl O-[(methylsulfanyl)methyl]-N-trityl-D-tyrosinate. 3-9-2

298 mg (0,55 mmol) 4-Methoxybenzyl *N*-trityl-D-tyrosinate **3-9-1** were dissolved in 4,5 ml *N,N*-dimethylformamide. 357 mg (1,1 mmol) caesium carbonate were added and the mixture stirred for 16 h. 64 mg (0,66 mmol) chloro dimethyl sulfide were added and the mixture stirred for 16 h. Further 23 mg (0,24 mmol) chloro dimethyl sulfide were added and the mixture stirred for 2 h. Further 23 mg (0,24 mmol) chloro dimethyl sulfide and 200 mg caesium carbonate were added and the mixture stirred for 20 h. The mixture was concentrated, diluted with ethyl acetate and extracted with water. The organic solutions were washed with saturated sodium chloride solution, dried over sodium sulfate and concentrated to give 335 mg.

MS (ESI⁺): m/e = 604,23 (M + H⁺).

MS (ESI⁻): m/e = 648,00 (M + HCOO).

¹H-NMR (300MHz, CHLOROFORM-d): δ [ppm]= 2.23 - 2.30 (m, 3H), 2.68 (d, 1H), 2.76 (dd, 1 H), 2.86 (dd, 1 H), 3.52 - 3.61 (m, 1H), 3.78 - 3.83 (m, 3H), 4.20 (d, 1H), 4.41 (d, 1H), 5.12 (s, 2H), 6.77 - 6.88 (m, 4H), 6.94 (d, 2H), 7.06 (d, 2H), 7.12 - 7.26 (m, 9H), 7.38 - 7.50 (m, 6H).

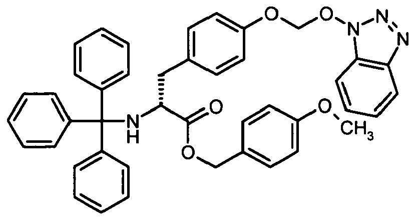

### 4-Methoxybenzyl O-[(1H-benzotriazol-1-yloxy)methyl]-N-trityl-D-tyrosinate. 1-9

160 mg (0,27 mmol) **3-9-2** were reacted as described for **1-2-1.** The reaction mixture was directly applied to Isolute and chromatographed on a Biotage system (Isolera Four, SNAP 10 g, n-hexane to n-hexane / ethyl acetate 4:1) to give 73 mg (40 %) **1-9.**

The material was further purified by HPLC (Waters Autopurificationsystem: Pump 254, Sample Manager 2767, CFO, DAD 2996, ELSD 2424, SQD 3001, XBrigde C18 5µm 100x30 mm, A = water + 0.1% formic acid, B = acetonitrile, 0-1 min 50% B, 1-8 min 50-100% B, 50 ml/min, r.t., 69 mg / 2.1 ml dimethyl sulfoxide/methanol 1:1, 3 x 0.7 ml, DAD scan range 210-400 nm, MS ESI+, ESI-, scan range 160-1000 m/z, ELSD). The peak at 7.8 - 8.1 min was collected to give 16 mg (9%) of **1-9** with >99 % purity.

MS (ESI⁺): m/e = 691,26 (M + H⁺).

MS (ESI⁻): m/e = 736,15 (M + HCOO).

¹H NMR (CHLOROFORM-d ,300MHz): δ (ppm) 7.98 (d, *J*=8.3 Hz, 1H, Bt 7-H), 7.44 (d, *J*=7.0 Hz, 6H, Tr o-H), 7.09 - 7.33 (m, 14H, Tr m-H, p-H, Ar 2-H, Bt H-4,5,6), 7.02 (d, *J*=8.3 Hz, 2H, Mbn 2-H), 6.99 (d, *J*=8.3 Hz, 2H, Ar 3-H), 6.78 (d, *J*=8.7 Hz, 2H, Mbn 3-H), 6.01 (br. s, 2H, OCH₂O) 4.46 (d, *J*=12.1 Hz, 1H, Mbn 1-H), 4.26 (d, *J*=11.9 Hz, 1H, Mbn 1-H), 3.75 (s, 3H, Mbn OMe), 3.55 - 3.65 (m, 1H, 2-H), 2.95 (s, 2H).

¹³C NMR (CHLOROFORM-d ,101MHz): δ (ppm) 174.2 (C-1), 159.6 (Mbn C-5), 155.1 (3C-4), 145.9 (Tr C-2), 143.5 (Bt C-3a), 132.6 (3C-1), 131.4 (3C-2), 130.2 (Mbn C-3), 128.8 (Tr C-3), 128.4 (Bt C-6), 128.0 (Bt C-7a), 127.9 (Tr C-4), 127.5 (Mbn C-2), 126.5 (Tr C-5), 124.7 (Bt C-5), 120.0 (Bt C-4), 115.9 (3C-3), 113.8 (Mbn C-4), 109.1 (Bt C-7), 99.2 (OCH₂O), 71.2 (Tr C-1), 66.3 (Mbn C-1), 58.1 (C-2), 55.3 (Mbn OMe), 41.4 (C-3).

### 1.10 Example 10

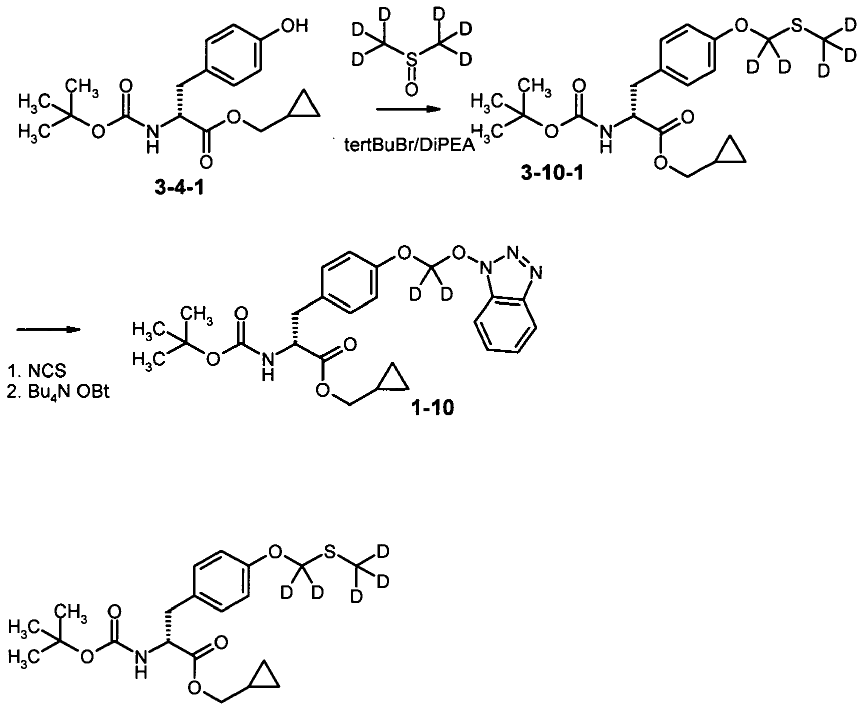

### Cyclopropylmethyl N-(tert-butoxycarbonyl)-O-[([²H₃]methylsulfanyl)[²H₂]methyl] -D-tyrosinate. 3-10-1

A solution of 1.00g (2.98 mmol) **3-4-1** was dissolved in 10 ml [²H₆]dimethyl sulfoxide and 5,1 ml (29,8 mmol) ethyl-diisopropyl amine added. The mixture was heated to 45° C under Argon atmosphere and the reaction started by addition of 3,46 ml 4.09 mmol) tert-butyl bromide. It was kept at this temperature for 72 h and then filtered. The filtrate was diluted with dichloromethane and washed with sat. sodium hydrogen carbonate. The organic phase was evaporated and the residue chromatographed on a Biotage system: (Flash40+M cartidge, 40 ml/min, 3CV dichloromethane, dichloromethane to dichloromethane/methanol 4:1 in 12CV, 15CV = 1980 ml) gave 1,08 g which was further purified on an Autopurification System (Waters: 2525 Binary Gradient Module, Detector: MS Micromass ZQ, UV Photo Diode Array 2996, 210-350 nm; X-Bridge Prep 50x50mm, C18 5 µm; Gradient: acetonitrile from 50% acetonitrile to 80%, water 0,1% formic acid; 9Min, 60 ml/min) to give 153 mg (12%) of **3-10-1** as a clear oil. Re-chromatography of an impure fraction gave another 8 mg of **3-10-1.** MS (ESI⁺): m/e = 423 (M + Na⁺), 401 (M + H⁺), 345 (M + H⁺ - C₄H₈), 301 (M + H⁺ - C₄H₈ - CO₂),

¹H NMR (DICHLOROMETHANE-d₂ ,600MHz): δ (ppm) 7.14 (d, J=8.7 Hz, 2H, Ar-H), 6.92 (d, J=8.7 Hz, 2H, Ar-H), 5.15 - 5.17 (m, 0,09H, OCHDS), 4.99 - 5.09 (m, 1H, NH), 4.48 - 4.57 (m, 1H, 2-H), 3.97 (mc, 2H, OCH₂), 2.97 - 3.15 (m, 2H, 3-H), 2.22 - 2.25 (m, 0.09H, SCHD₂), 1.45 (s, 9H, Boc), 1.11 - 1.20 (m, 1H, cyclopropyl CH), 0.58 - 0.65 (m, 2H, cyclopropyl CH₂), 0.27 - 0.37 (m, 2H, cyclopropyl CH₂) [>90% deuteration in both positions].

¹³C NMR (DICHLOROMETHANE-d₂ ,151MHz): δ (ppm) 172.3 (C-1), 156.5 (3C-4), 155.3 (2C-1), 130.8 (3C-2), 129.8 (3C-1), 116.3 (3C-3), 79.9 (2C-2), 72.3 (quint., ¹J_{CD} = 24.2 Hz, OCD₂S), 70.4 (1C-1), 55.0 (C-2), 37.7 (C-), 28.4 (2C-3), 14.0 (sept., ¹J_{CD} = 21.6 Hz, SCD₃), 14.1 (quint, ¹J_{CD} = 22.3 Hz, SCHD₂), 10.0 (1C-2), 3.6 (1 C-3), 3.5 (1 C-4).

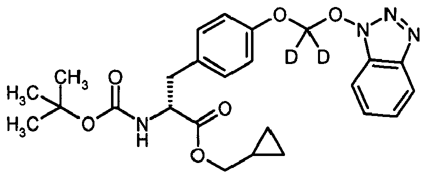

### Cyclopropylmethyl O-[(1H-benzotriazol-1-yloxy)[²H₂]methyl]-N-(tert-butoxy-carbonyl)-D-tyrosinate. 1-10

160 mg (0.40 mmol) of **3-10-1** were reacted as described for **1-2-1** with the exception that the reaction time in step 3 is shortened to 10 min. The solution was directly chromatographed on a Biotage system (Flash25+M cartidge, 25 ml/min, n-hexane to n-hexane / ethyl acetate 1:1 in 15CV = 780 ml) gave 88 mg of **1-10.** The compound was further purified by preparative HPLC (Dionex: Pump P 580, Gilson: Liquid Handler 215, Knauer: UV-Detector K-2501, Chiralpak IC 5µm 250x30 mm, hexane / ethanol 80:20, 40 ml/min, r.t., 88 mg / 1.6 ml ethanol 2 x 0.8 ml, UV 254 nm). Collection of the eluate from 16.0 to 17.2 min gave after evaporation 43 mg of **1-10** with a purity of 97.3%. After thorough drying under high vakuum, 15.4 mg (8%) **1-10** was obtained.

MS (ESI⁺): m/e = 485 (M + H⁺), 429 (M + H⁺ - C₄H₈), 385 (M + H⁺ - C₄H₈ - CO₂).

¹H NMR (DICHLOROMETHANE-d₂ ,600MHz): δ (ppm) 7.95 (d, J=8.3 Hz, 1H, Bt-H), 7.39 (ddd, J=8.3, 6.8, 0.8 Hz, 1H, Bt-H), 7.34 (ddd, J=8.3, 6.8, 1.1 Hz, 1H, Bt-H), 7.16 - 7.23 (m, 3H, Bt-H, Ar-H), 7.06 (d, J=8.3 Hz, 2H, Ar-H), 5.38 (d, J=7.9 Hz, 1H, NH), 4.48 (ddd, J=7.9, 6.8, 5.6 Hz, 1H, 2-H), 3.98 - 3,88 (m, 2H, COOCH₂), 3.12 (dd, J=13.9, 5.6 Hz, 1H, 3-H), 3.02 (dd, J=13.9, 6.8 Hz, 1H. 3-H), 1.39 (s, 9H, Boc), 1.07 - 1.13 (m, 1H, Cyclopropyl CH), 0.51 - 0.59 (m, 2H, cyclopropyl CH₂), 0.23 - 0.29 (m, 2H, cyclopropyl CH₂). 6.02 (s, 0.08H, OCH₂O corresponds to 4 mol% undeuterated compound, 6.01 (d, J=1.1 Hz, 0.12H, OCDHO) corresponds to 12 mol% monodeuterated compound.

¹³C NMR (DICHLOROMETHANE-d₂ ,151MHz): δ (ppm) 172.2 (C-1), 155.4 (3C-4, 2C-1), 143.7 (Bt C-3a), 132.1 (3C-1), 131.1 (3C-2), 128.9 (Bt C-7a), 128.5 (Bt C-6), 124.9 (Bt C-5), 120.1 (Bt C-4), 116.4 (3C-3), 109.3 (Bt C-7), 98.8 (p, ¹J_{CD} = 25Hz, OCD2O), 79.7 (2C-2), 70.3 (1C-1), 55.0 (C-2), 37.5 (C-3), 28.3 (2C-3), 9.9 (1C-2). 3.5 (1C-3), 3.4 (1C-4).

### 1.11 Example 11

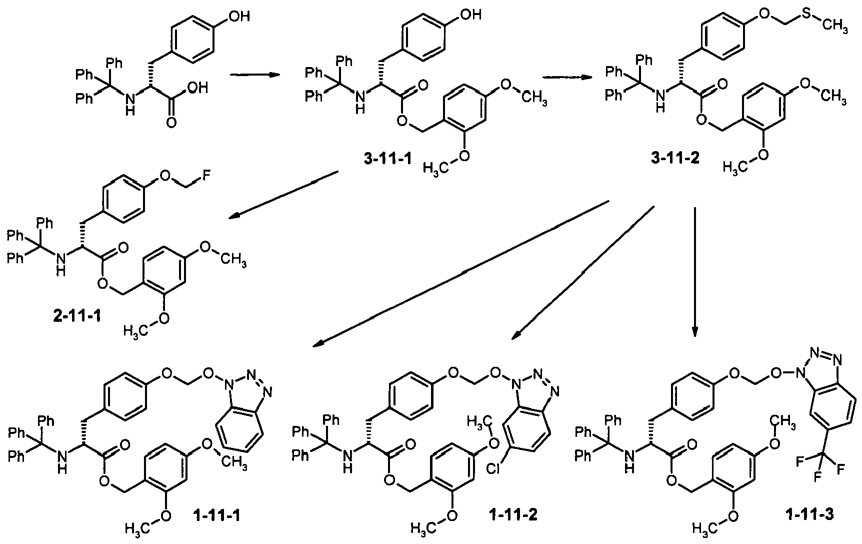

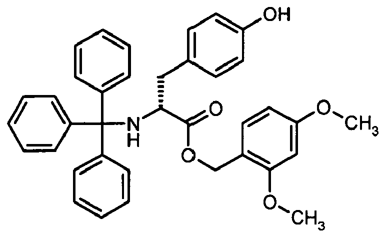

### 2,4-Dimethoxybenzyl N-trityl-D-tyrosinate. 3-11-1

5,00 g (11,81 mmol) *N*-trityl-D-tyrosine (Liebigs Ann. Chem. 1988, 1083-1084) were dissolved in 97,7 ml *N,N*-dimethylformamide. 2,31 g (7,08 mmol) caesium carbonate were added and the mixture stirred for 15 min. 3,14 g (13,58 mmol) 2,4-dimethoxybenzyl bromide in toluene (US5663200, 1997*, Example 49a*) were added and the mixture stirred for 16 h. The mixture was concentrated, diluted with ethyl acetate and extracted with water. The organic solutions were washed with saturated sodium chloride solution, dried over sodium sulfate and concentrated. The residue was purified by chromatography over a 55 g SNAP KP-NH cartridge (Biotage) with dichloromethane/ethanol 100/0 - 97/3 - 94/6 -91/9 to give 4,218 g.

MS (ESI⁺): m/e = 574,42 (M + H⁺).

MS (ESI⁻): m/e = 572,29 (M - H), 618,42 (M + HCOO).

¹H NMR (CHLOROFORM-d ,300 MHz): δ (ppm) 7.40 - 7.45 (m, 6H, Tr-H), 7.12 - 7.24 (m, 9H, Tr-H), 6.99 (d, 2H, Ar-H), 6.92 (d, 1H, Dmb H-6), 6.68 (d, 2H, Ar-H), 6.49 (d, 1H, Dmb H-3), 6.40 (dd, 1H, Dmb H-5), 4.94 (br. s, 1H, OH), 4.58 (d, 1H, OCH₂Ar), 4.34 (d, 1H), 3.81 (s, 3H, Dmb OMe), 3.76 (s, 3H, Dmb OMe), 3.52 - 3.61 (m, 1H, 2-H), 2.85 (br. s, 1H, 3-H), 2.84 (br. s, 1H, 3-H), 2.59 (d, 1H, NH).

¹³C NMR (CHLOROFORM-d ,101MHz): δ (ppm) 174.4 (C-1), 161.1 (Dmb C-5), 158.8 (Dmb C-3), 154.3 (3C-4), 146.0 (Tr C-2), 131.6 (Dmb C-7), 131.0 (3C-2), 129.5 (3C-1), 128.8 (Tr C-3), 127.8 (Tr C-4), 126.3 (Tr C-5), 116.4 (Dmb C-2), 115.0 (3C-3), 103.9 (Dmb C-6), 98.4 Dmb C-4), 71.1 (Tr C-1), 61.8 (Dmb C-1), 58.2 (C-2), 55.5 (Dmb OMe), 55.3 (Dmb OMe), 41.2 (C-3).

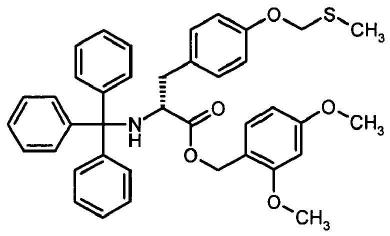

### 2,4-Dimethoxybenzyl O-[(methylsulfanyl)methyl]-N-trityl-D-tyrosinate. 3-11-2

8,791 g (15,32 mmol) 2,4-Dimethoxybenzyl *N*-trityl-D-tyrosinate **3-11-1** were dissolved in 123 ml *N,N*-dimethylformamide. 9,99 g (30,65 mmol) caesium carbonate were added and the mixture stirred for 30 min. 1,78 g (18,39 mmol) chloro dimethyl sulfide were added and the mixture stirred for 20 h. The mixture was concentrated, diluted with ethyl acetate and extracted with water. The organic solutions were washed with saturated sodium chloride solution, dried over sodium sulfate and concentrated. The residue was purified by chromatography over a 110 g SNAP KP-NH cartridge (Biotage) with n-hexane/ethyl acetate 100/0 - 80/20 - 60/40 to give 5,59 g.

¹H NMR (CHLOROFORM-d ,300MHz): δ (ppm) 7.37 - 7.46 (m, 6H, Tr-H), 7.11 - 7.25 (m, 9H, Tr-H), 7.07 (d, *J*=8.7 Hz, 2H, Ar-H), 6.92 (d, *J*=8.9 Hz, 1H, Dmb H-6), 6.83 (d, *J*=8.7 Hz, 2H, Ar-H), 6.40 (d, *J*=2.1 Hz, 1H, Dmb H-3), 6.39 (dd, *J*=7.0, 2.3 Hz, 1H, Dmb H-5), 5.11 (s, 2H, OCH₂S), 4.58 (d, *J*=12.1 Hz, 1H, OCH₂Ar), 4.34 (d, *J*=12.1 Hz, 1H, OCH₂Ar), 3.80 (s, 3H, Dmb OMe), 3.76 (s, 3H, Dmb OMe), 3.51 - 3.63 (m, 1H, 2-H), 2.86 (br. s, 1H, 3-H), 2.84 (br. s, 1H, 3-H), 2.59 (d, *J*=10.5 Hz, 1H, NH), 2.25 (s, 3H, SCH₃).

¹³C NMR (CHLOROFORM-d ,75MHz): δ (ppm) 174.2 (C-1), 161.1 (Dmb C-5), 158.8 (Dmb C-3), 155.8 (3C-4), 146.0 (Tr C-2), 131.5 (Dmb C-7), 130.9 (3C-2), 130.7 (3C-1), 128.8 (Tr C-3), 127.8 (Tr C-4), 126.3 (Tr C-5), 116.3 (Dmb C-2), 115.6 (3C-3), 103.8 (Dmb C-6), 98.3 (Dmb C-4), 72.4 (OCH₂S, 71.0 (Tr C-1), 61.6 (Dmb C-1), 58.1 (C-2), 55.4 (Dmb OMe), 55.3 (Dmb OMe), 41.2 (C-3), 14.6 (SCH₃).

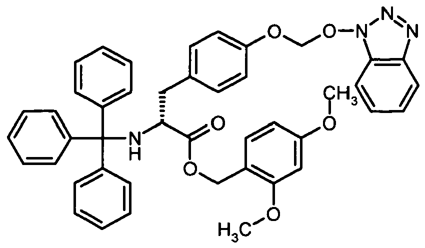

### 2,4-Dimethoxybenzyl O-[(1H-benzotriazol-1-yloxy)methyl]-N-trityl-D-tyrosinate. 1-11-1

To a solution of **3-11-2** (356 mg, 0,56 mmol) in dichloromethane (5 ml) at -15 °C was added *N*-chlorosuccinimide (82,5 mg, 0,62 mmol). The cooling bath was removed and the solution stirred for 4 h. A solution of tetrabutylammonium 1-hydroxybenzotriazolat (253,8 mg, 0,67 mmol) in dichloromethane (2 + 0,5 ml) was added. The solution was stirred for 1 h. The reaction mixture was directly applied to Isolute and chromatographed on a Biotage system (Isolera Four, SNAP 10 g, dichloromethane/ethyl acetate 100/0 - 95/5) to give 248 mg. The compound was purified by preparative HPLC (Dionex: Pump P 580, Gilson: Liquid Handler 215, Knauer: UV-Detektor K-2501,Chiralpak IB 5µm 250x30 mm,Hexan / Ethanol 80:20,40 ml/min,RT,248 mg / 3.5 ml Ethanol/Dichloromethane,5 x 0.7 ml,UV 254 nm, 8.5 - 12.2 min,94.2 % ,120 mg,Peak 6 - 5.17 min) to give 116 mg of **1-11-1** with 94 % purity.

MS (ESI⁺): m/e = 721,39 (M + H⁺), 243,11 (C₁₉H₁₅⁺)_{.}

¹H NMR (DICHLOROMETHANE-d₂ ,300MHz): δ (ppm) 7.96 (d, *J*=8.3 Hz, 1H, Bt H-4), 7.37 - 7.51 (m, 6H, Tr H), 7.10 - 7.36 (m, 17H, Tr-H, Ar-H), 7.04 (d, *J*=8.7 Hz, 2H, Ar-H), 6.96 (d, *J*=8.1 Hz, 1H, Dmb 6-H), 6.40 (d, *J*=2.4 Hz, 1H, Dmb 3-H), 6.38 (dd, *J*=8.1, 2.4 Hz, 1H, Dmb 5-H), 5.98 - 6.07 (mc, 2H, OCH₂O), 4.59 (d, *J*=11.9 Hz, 1H, Dmb 1-H), 4.34 (d, *J*=11.9 Hz, 1H, Dmb 1-H), 3.75 (s, 3H, Dmb OMe), 3.75 (s, 3H, Dmb OMe), 2.80 - 2.99 (m, 2H, 3-H).

¹³C NMR (CHLOROFORM-d ,75MHz): δ (ppm) 173.7 (C-1), 161.2 (Dmb C-5), 158.8 (Dmb C-3), 154.9 (3C-4), 146.0 (2C-2), 143.4 (Bt C-3a), 132.8 (3C-1), 131.4 (Dmb C-7), 131.3 (3C-3), 128.7 (Tr C-3), 128.7 (Bt C-7a), 128.2 (Bt C-6), 127.8 (Tr C-4), 126.3 (Tr C-5), 124.5 (Bt C-5), 119.7 (Bt C-4), 116.2 (Dmb C-2), 115.7 (3C-3), 109.0 (Bt C-7), 103.9 (Dmb C-6), 99.1 (OCH₂O) 98.1 (Dmb C-4), 71.1 (Tr C-1), 61.6 (Dmb C-1), 57.9 (C-2), 55.3 (Dmb OMe), 55.3 (Dmb OMe), 41.0 (C-3).

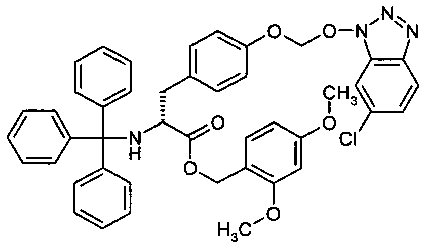

### 2,4-Dimethoxybenzyl O-{[(6-chloro-1H-benzotriazol-1-yl)oxy]methyl}-N-trityl-D-tyrosinate. 1-11-2

To a solution of **3-11-2** (80 mg, 0,13 mmol) in dichloromethane (1,1 ml) at -15 °C was added *N*-chlorosuccinimide (18,54 mg, 0,14 mmol). The cooling bath was removed and the solution stirred for 4 h. A solution of tetrabutylammonium 6-chloro-1-hydroxybenzotriazolat (62,3 mg, 0,15 mmol) in dichloromethane (0,6 ml) was added. The solution was stirred for 1 h. The reaction mixture was directly applied to Isolute and chromatographed (SNAP 5 g, dichloromethane to dichloromethane / ethyl acetate 95:5). The compound was purified by preparative HPLC (Waters Autopurificationsystem: Pump 254, Sample Manager 2767, CFO, DAD 2996, ELSD 2424, SQD 3001, XBrigde C18 5 µm 100x30 mm, A = water + 0.2% ammonia, B = acetonitrile, 0-1 min 70% B, 1-8 min 70-100% B, 50 ml/min, r.t. 14 mg / 1.5 ml dimethyl sulfoxide/methanol 1:1, 1 x 1.5 ml, DAD scan range 210-400 nm, MS ESI+, ESI-, scan range 160-1000 m/z, ELSD). The fractions eluting at 6.6 - 7.0 min were collected to give 6 mg (6%) of **1-11-2** with >99 % purity (DAD).

¹H NMR (DICHLOROMETHANE-d₂ ,400MHz): (ppm) 7.90 (d, *J*=8.8 Hz, 1H, Bt 4-H), 7.41 - 7.46 (m, 6H, Tr-H), 7.27 - 7.33 (m, 2H, Bt 5-H, H-7), 7.14 - 7.26 (m, 11H, Tr-H, Ar-H), 7.01 (d, *J*=8.8 Hz, 2H, Ar-H), 6.94 (d, *J*=8.1 Hz, 1H, Dmb 6-H), 6.38 (d, *J*=2.3 Hz, 1H, Dmb 3-H), 6.36 (dd, *J*=8.1, 2.5 Hz, 1H, Dmb 5-H), 6.00 (s, 2H, OCH₂O), 4.59 (d, *J*=11.9 Hz, 1H, Dmb 1-H), 4.33 (d, *J*=11.9 Hz, 1H, Dmb 1-H), 3.75 (s, 3H, Dmb OMe), 3.74 (s, 3H, Dmb OMe), 3.53 - 3.60 (m, 1H, 2-H), 2.85 - 2.97 (m, 2H, 3-H).

¹³C NMR (CHLOROFORM-d ,101MHz): δ (ppm) 173.8 (C-1), 161.2 (Dmb C-5), 158.9 (Dmb C-3), 154.7 (3C-4), 146.0 (2C-2), 142.1 (Bt C-3a), 134.6 (Bt C-6), 133.0 (3C-1), 131.5 (Dmb C-7), 131.4 (3C-2), 129.2 (Bt C-7a), 128.8 (Tr C-3), 127.9 (Tr C-4), 126.4 (Tr C-5), 125.9 (Bt C-5), 121.0 (Bt C-4), 116.2 (Dmb C-2), 115.8 (3C-3), 109.0 (Bt C-7), 103.9 (Dmb C-6), 99.2 (OCH₂O), 98.1 (Dmb C-4), 71.2 (Tr C-1), 61.6 (Dmb C-1), 58.0 (C-2), 55.3 (Dmb OMe), 55.3 (Dmb OMe), 41.0 (C-3).

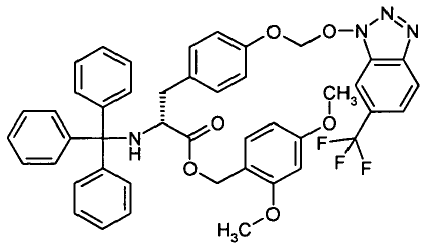

### 2,4-Dimethoxybenzyl O-{[(6-trifluoromethyl-1H-benzotriazol-1-yl)oxy]methyl}-N-trityl-D-tyrosinate. 1-11-3

To a solution of **3-11-2** (824,6 mg, 1,30 mmol) in dichloromethane (12 ml) at -15 °C was added *N*-chlorosuccinimide (191,1 mg, 1,43 mmol). The cooling bath was removed and the solution stirred for 5 h. A solution of tetrabutylammonium 6-trifluoromethyl-1-hydroxybenzotriazolat (694,1 mg, 1,56 mmol) in dichloromethane (6 ml) was added. The solution was stirred for 1 h. The reaction mixture was directly applied to Isolute and chromatographed (SNAP 25 g, n-hexane/ethyl acetate 100/0 - 85/15 - 60/40) to give 213 mg of **1-11-3** with >95 % purity.

MS (ESI⁺): m/e = 789,37 (M + H⁺).

MS (ESI⁻): m/e = 833,07 (M + HCOO).

¹H NMR (CHLOROFORM-d ,400MHz): δ (ppm) 8.12 (d, *J*=8.8 Hz, 1H, Bt 4-H), 7.57 (d, *J*=8.8 Hz, 1H, Bt 5-H), 7.40 - 7.50 (m, 6H, Tr o-H), 7.11 - 7.25 (m, 12H, Tr m-H, p-H, Bt 7-H, Ar-H), 6.95 (m, 3H, Ar-H, Dmb 6-H), 6.32 - 6.42 (m, 2H, Dmb 3-H, 5-H), 6.04 (s, 2H, OCH₂O), 4.62 (d, *J*=11.9 Hz, 1H, Dmb 1-H), 4.35 (d, *J*=11.9 Hz, 1H, Dmb 1-H), 3.76 (s, 3H, Dmb OMe), 3.75 (s, 3H, Dmb OMe), 3.62 (br. s., 1H, 2-H), 2.93 (m, 2H, 3-H), 2.62 (br. s, 1H, NH).

¹³C NMR (101 MHz, CHLOROFORM-d) δ ppm 174.0 (C-1), 161.2 (Dmb C-5), 158.9 (Dmb C-3), 154.3 (3C-4), 146.0 (2C-2), 144.5 (Bt C-3a), 133.1 (3C-1), 131.6 (Dmb C-7), 131.5 (3C-2), 130.4 (q, ²J_{CF}=32.0 Hz, Bt C-6), 128.8 (Tr C-3), 128.2 (Bt C-7a), 127.8 (Tr C-4), 126.4 (Tr C-5), 123.6 (q, ¹J_{CF}=273.2 Hz, CF₃), 121.4 (q, ³J_{CF}=3.2 Hz, Bt C-5), 121.2 (Bt C-4), 116.3 (Dmb C-2), 115.6 (3C-3), 107.9 (q, J=4.8 Hz, Bt C-7), 103.9 (Dmb C-6), 99.0 (OCH₂O) 98.4 (Dmb C-4), 71.2 (Tr C-1), 61.8 (Dmb C-1), 57.9 (C-2), 55.4 (Dmb OMe), 55.3 (Dmb OMe), 41.1 (C-3).

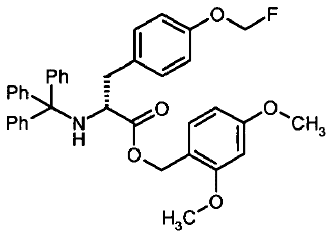

### 2,4-Dimethoxybenzyl O-(fluoromethyl)-N-trityl-D-tyrosinate. 2-11-1

84 mg (0,15 mmol) 2,4-dimethoxybenzyl *N*-trityl-D-tyrosinate **3-11-1** were dissolved in 1 ml tetrahydrofuran. The solution was cooled to 0 °C. 16,5 mg (0,41 mmol) sodium hydride (60% in mineral oil) were added and the mixture stirred for 1 hour. 1,05 ml of tetrahydrofuran containing bromofluoromethane were added slowly at 0 °C and the mixture was stirred at 0 °C for 12 h. 1 ml methanol was added and the mixture diluted with ethyl acetate and extracted with water. The organic solutions were dried over sodium sulfate and concentrated. The residue was applied to Isolute and chromatographed (SNAP 10 g, n-hexane to n-hexane/ethyl acetate 6:4) to give 60 mg of **2-11-1** with 90 % purity.

MS (ESI⁺): m/e = 606,24 (M + H⁺).

¹⁹F NMR (376 MHz, CHLOROFORM-*d*): δ (ppm) -147.9 (t, *J*=55.1 Hz).

¹H NMR (CHLOROFORM-d ,300MHz): δ (ppm) 7.39 - 7.47 (m, 6H, Tr-H), 7.11 - 7.25 (m, 9H, Tr-H), 7.07 (d, *J*=8.5 Hz, 2H, Ar-H), 6.93 (d, *J*=8.5 Hz, 2H, Ar-H), 6.89 (d, *J*=8.9 Hz, 1H, Dmb 6-H), 6.35 - 6.43 (m, 2H, Dmb 5-H, 3-H), 5.67 (d, ²J_{HF}=55.0 Hz, 2H, OCH₂F), 4.58 (d, *J*=11.9 Hz, 1H, Dmb 1-H), 4.34 (d, *J*=11.9 Hz, 1H, Dmb 1-H), 3.80 (s, 3H, Dmb OMe), 3.75 (s, 3H, Dmb OMe), 3.52 - 3.64 (m, 1H, 2-H), 2.84 - 2.92 (m, 2H, 3-H, NH), 2.60 (d, br., *J*=9.6 Hz, 1H, 3-H).

¹³C NMR (75 MHz, CHLOROFORM-*d*) δ (ppm) 174.1 (C-1), 161.1 (Dmb C-5), 158.8 (Dmb C-3), 155.6 (d, ³J_{CF} =3.0 Hz, 3C-4), 145.9 (Tr C-2), 132.5 (Dmb C-7), 131.5 (3C-1), 131.0 (3C-2), 128.8 (Tr C-3), 127.8 (Tr C-4), 126.3 (Tr C-5), 116.3 (d, ⁴J_{CF}=1.2 Hz, 3C-3), 116.3 (Dmb C-2), 103.9 (Dmb C-6), 100.9 (d, ¹J_{CF} =218.4 Hz, OCH₂F), 98.3 (Dmb C-4), 71.1 (Tr C-1), 61.6 (Dmb C-1), 58.0 (C-2), 55.4 (Dmb OMe), 55.3 (Dmb OMe), 41.3 (C-3).

### 1.12 Example 12

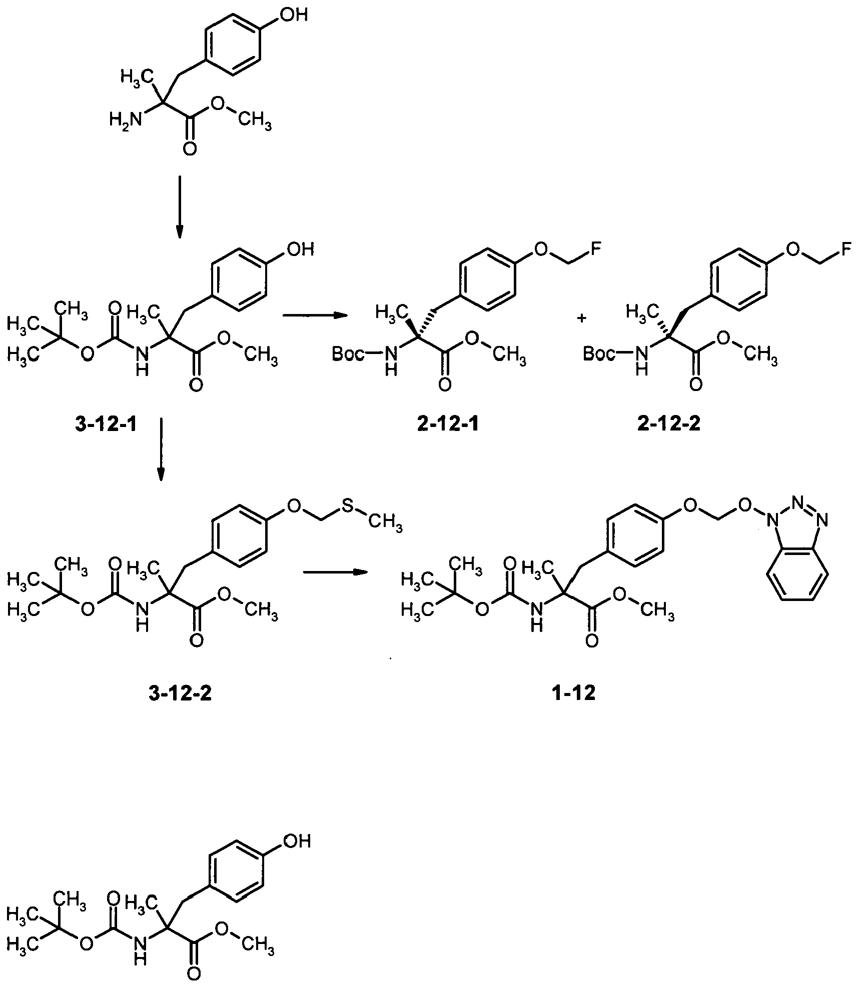

### Methyl N-(tert-butoxycarbonyl)-alpha-methyltyrosinate 3-12-1

9.25 g (37.6 mmol) Methyl alpha-methyltyrosinate hydrochloride were suspended in 100 ml dioxane and 100 ml 1N sodium hydrogen carbonate The pH of the reaction mixture was adjusted to 8-9 with 1N sodium hydroxide. 28.8 g (131 mmol) di-tert-butyl dicarbonate were added in portions and the mixture stirred for 3 d at r.t., while the pH was controlled and kept between 8 and 9. The reaction mixture was brought to pH 2 with 1 N sodium hydrogen sulfate and extracted with ethyl acetate. The organic phase was washed with water and brine, after evaporation of the solvent 14.8 g of raw material were obtained.

Chromatography on a Biotage Isolera system (SNAP 340 cartridge, 100 ml/min, n-hexane to n-hexane/ethyl acetate 59:41 in 12 CV = 4080 ml, Fractions 83-100) gave 10 g (86%) **3-12-1** as white solid.

MS (ESI⁻): m/e = 618 (2M - H⁺), 354 (M + HCOO⁻), 308 (M - H⁺).

MS (ESI⁺): m/e = 641 (2M + Na⁺), 619 (2M + H⁺), 332 (M + Na⁺), 310 (M + H⁺), 254 (M + H⁺ -C₄H₈), 210 (M + H⁺ - CO₂ - C₄H₈).

¹H NMR (CHLOROFORM-d ,500MHz): δ (ppm) 6.96 (d, *J*=8.5 Hz, 2H, Ar-H), 6.77 (d, *J*=8.2 Hz, 2H, Ar-H), 5.91 (br. s., 1H, OH), 5.20 (br. s., 1H, NH), 3.79 (s, 3H, OMe), 3.30 (br. s., 1H, 3-H), 3.15 (d, *J*=13.6 Hz, 1H, 3-H), 1.59 (br. s., 3H, 2-CH₃), 1.51 (s, 9H, Boc). ¹³C NMR (CHLOROFORM-d, 126MHz): δ (ppm) 174.6 (C-1), 155.1 (3C-4), 154.5 (2C-1), 131.2 (3C-2), 128.0 (br. 3C-1), 115.2 (3C-3), 79.6 (br. 2C-2), 60.5 (br. C-2), 52.5 (1C-1), 41.2 (br. C-3), 28.4 (3C-3), 23.6 (2-CH₃).

In fraction 62 - 68, 1.48 g (10%) of the bisbocylated compound were isolated. Methyl *N,O*-bis(*tert*-butoxycarbonyl)-alpha-methyltyrosinate

MS (ESI⁺): m/e = 432 (M + Na⁺), 427 (M⁺ + H₂O), 410 (M + H⁺), 354 (M + H⁺ - C₄H₈), 310 (M + H⁺- CO₂ - C₄H₈), 254 (M + H⁺- CO₂ - 2C₄H₈).

¹H NMR (CHLOROFORM-d ,500MHz): δ (ppm) 7.08 (s, 4H, Ar-H), 5.11 (br. s., 1H, NH), 3.76 (s, 3H, OCH₃), 3.36 (br. d, *J*=12.3 Hz, 1H, 3-H), 3.23 (d, *J*=13.6 Hz, 1H, 3-H), 1.56 (s, 9H, OBoc), 1.54 (br. s., 3H, 2-CH₃), 1.47 (s, 9H, NBoc).

¹³C NMR (CHLOROFORM-d, 126MHz): δ (ppm) 174.3 (C-1), 154.3 (2C-1), 151.9 (OBoc C-1), 150.1 (3C-4), 134.0 (br., 3C-1), 131.0 (3C-2), 120.9 (3C-3), 83.5 (OBoc C-2), 79.6 (br., 2C-2), 60.2 (br., C-2), 52.6 (OCH₃), 40.8 (br., C-3), 28.4 (2C-3), 27.8 (OBoc C-3), 23.7 (br., 2-CH₃).

### Methyl (R) and (S)-2-[(tert-Butoxycarbonyl)amino]-3-(fluoromethoxy)phenyl-2-methylpropionate

As described in the preparation of **2-1-1,** 250 mg (0,81 mmol) **3-12-1** were reacted to give 221 mg of raw product, which was purified by preparative HPLC (Dionex: Pump P 580, Gilson: Liquid Handler 215, Knauer: UV-Detector K-2501, Chiralpak AD-H 5 µm 250x20 mm, hexan / ethanol 80:20, 20 ml/min, r.t., 221 mg / 4 ml ethanol, 10 x 0.4 ml, UV 210 nm) The peaks at 4.8 - 5.5 min (75 mg 99.5 %) and 5.7 - 6.3 min (76 mg 98.6 %) were collected. Combined yield 27%.

The stereochemistry of the first peak was putatively assigned "R" (comparison of the retention behaviour on the chiral HPLC with 2-2-1 and 2-2-2).

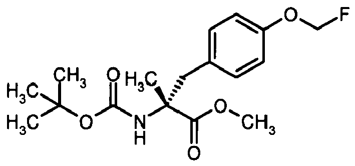

### Methyl N-(tert-butoxycarbonyl)-O-(fluoromethyl)-alpha-methyl-D-tyrosinate 2-12-1 α_{D} +44.1° (MeOH, c=1, 589nm).

MS (ESI⁺): m/e = 364 (M + Na⁺), 342 (M + H⁺), 286 (M + H⁺ - C₄H₈), 242 (M + H⁺- CO₂ - **C₄H₈).**

¹⁹F NMR (376 MHz, DICHLOROMETHANE-*d*₂) δ ppm -149.0 (t, ²J_{HF} =55.1 Hz). ¹H NMR (DICHLOROMETHANE-d₂, 400MHz): δ (ppm) 7.07 (d, J=8.5 Hz, 2H, Ar-H), 7.01 (d, J=8.5 Hz, 2H, Ar-H), 5.72 (d, **²**J_{HF} =54.7 Hz, 2H, OCH₂F), 5.14 (br. s., 1H, NH), 3.75 (s, 3H, OCH₃), 3.34 (br. d, J=13.1 Hz, 1H, 3-H), 3.17 (d, J=13.8 Hz, 1H, 3-H), 1.54 (s, 3H, 2-CH₃), 1.48 (s, 9H, Boc).

¹³C NMR (101 MHz, DICHLOROMETHANE-d₂) δ ppm 174.3 (C-1), 155.7 (d, ³J_{CF}=2.7 Hz, 3C-4), 154.2 (2C-1), 131.8 (3C-1), 131.3 (3C-2)), 116.2 (d, ⁴J_{CF} =1.2 Hz, 3C-3), 101.0 (d, ¹J_{CF}=217.4 Hz, OCH₂F), 79.3 (br., 2C-2), 60.3 (C-2), 52.3 (1C-1), 40.7 (br., C-3), 28.1 (2C-3), 23.4 (2-CH₃).

The stereochemistry of the second peak was putatively assigned "S" (comparison of the retention behaviour on the chiral HPLC with **2-2-1** and **2-2-2**).

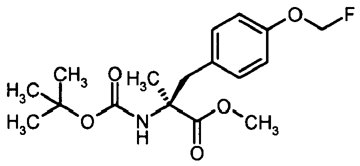

### Methyl N-(tert-butoxycarbonyl)-O-(fluoromethyl)-alpha-methyl-L-tyrosinate 2-12-2 α_{D} -45.8° (MeOH, c=1, 589nm).

MS (ESI⁺): m/e = 364 (M + Na⁺), 342 (M + H⁺), 286 (M + H⁺ - C₄H₈), 242 (M + H⁺ - CO₂ - C₄H₈).

¹⁹F NMR (376 MHz, DICHLOROMETHANE-d₂) δ ppm -149.0 (t, ²J_{HF} =55.1 Hz). ¹H NMR (DICHLOROMETHANE-d₂ ,400MHz): δ (ppm) 7.04 (d, J=8.8 Hz, 2H, Ar-H), 6.98 (d, J=8.6 Hz, 2H, Ar-H), 5.70 (d, J=54.8 Hz, 2H, OCH₂F), 5.13 (br. s, 1H, NH), 3.73 (s, 3H, OCH₃), 3.33 (br. d, J=13.6 Hz, 1H, 3-H), 3.14 (d, J=13.6 Hz, 1H, 3-H), 1.51 (s, 3H, 2-CH₃), 1.45 (s, 9H, Boc).

¹³C NMR (101 MHz, DICHLOROMETHANE-d₂) δ ppm 174.3 (C-1), 155.7 (d, ³J_{CF}=2.4 Hz, 3C-4), 154.2 (2C-1), 131.7 (3C-1), 131.3 (3C-2)), 116.2 (3C-3), 100.9 (d, ¹J_{CF}=217.3 Hz, OCH₂F), 79.3 (br., 2C-2), 60.3 (C-2), 52.3 (1C-1), 40.7 (br., C-3), 28.1 (2C-3), 23.4 (2-CH₃).

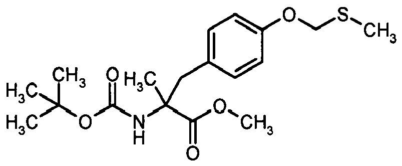

### Methyl N-(tert-butoxycarbonyl)-alpha-methyl-O-[(methylsulfanyl)methyl]tyrosinate 3-12-2

A solution of 2.00 g (6.67 mmol) **3-12-1**, 239 mg (0.67 mmol) tetrabutyl ammonium iodide in 20ml *N,N*-dimethylformamide were cooled in an ice bath and a solution of 798 mg (7.11 mmol) potassium tert-butoxide in 7 ml tetrahydrofurane added. Subsequently 614 µl (7.44 mmol) chloromethyl methyl sulfide were added, whereupon the solution turned yellow. The ice bath was removed and the reaction stirred for 2 h at r.t. For work-up, ethyl acetate was added and the resulting solution washed with water. After phase separation, the aquaeus phase was re-extracted with ethyl acetate. The combined organic phases were washed with 1 N sodium hydrogen carbonate₃ and brine and then dried over sodium sulfate. Evaporation gave 2.56 g raw product. Chromatography on a Biotage Isolera system (SNAP 50 cartridge, 50 ml/min, n-hexane to n-hexane/ethyl acetate 6:4 in 12 CV) did not return pure product. Rechromatography of the product containing fractions on a Biotage system (C18HS 40+M cartidge, 40 ml/min, water to water / acetonitrile 1:1 in 12CV = 1584 ml, water / acetonitrile 1:1 3CV = 396 ml) gave 1.39 g (58%) **3-12-2.**

MS (ESI*): m/e = 392 (M + Na⁺), 370 (M + H⁺), 314 (M + H⁺ - C₄H₈), 270 (M + H⁺ - CO₂ - C₄H₈).

¹H NMR (CHLOROFORM-d ,300MHz): δ (ppm) 7.00 (d, *J*=8.5 Hz, 2H, Ar-H), 6.85 (d, *J*=8.7 Hz, 2H, Ar-H), 5.11 (s, 2H, OCH₂S), 3.75 (s, 3H, OCH₃), 3.31 (br. d, *J*=13.4 Hz, 1H, 3-H), 3.14 (d, *J*=13.8 Hz, 1H, 3-H), 2.25 (s, 3H, SCH₃), 1.54 (br. s., 3H, 2-CH₃), 1.46 (s, 9H, Boc).

¹³C NMR (CHLOROFORM-d ,75MHz): δ (ppm) 174.4 (C-1), 156.0 (3C-4), 154.3 (2C-1), 131.0 (3C-2), 129.6 (br., 3C-1), 115.6 (3C-3), 79.5 (br., 2C-2), 72.4 (OCH₂S), 60.4 (C-2), 52.5 (O CH₃), 40.9 (br., C-3), 28.4 (2C-3), 23.6 (2-CH₃), 14.6 (SCH₃).

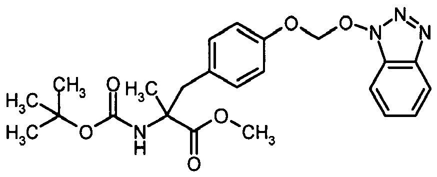

### Methyl O-[(1H-benzotriazol-1-yloxy)methyl]-N-(tert-butoxycarbonyl)-alpha-methyltyrosinate 1-12

300 mg (0.81 mmol) of **3-12-2** were reacted as described for **1-2-1.** The raw product was purified by chromatography on a Biotage Isolera system (SNAP 50 cartridge, 50 ml/min, n-Hexane, 1 CV, n-Hexane to n-hexane/ethyl acetate 6:4 in 10 CV, n-hexane/ethyl acetate 6:4 4 CV) gave slightly impure material. Further purification was done by preparative HPLC (Dionex: Pump P 580, Gilson: Liquid Handler 215, Knauer: UV-Detector K-2501; Chiralpak IA 5 µm 250x20 mm; hexane / 2-propanol 50:50; 12 ml/min; r.t. 170 mg / 1.5 ml ethanol; 5 x 0.3 ml; UV 254 nm). The peak eluting 7.0 - 8.2 min was collected to give 137 mg (37%) of 1-12 with a purity of 99.7 %. The material was not resolved into the enantiomers.

MS (ESI⁺): m/e = 479 (M + Na⁺), 457 (M + H⁺), 401 (M + H⁺ - C₄H₈), 357 (M + H⁺ - CO₂ -C₄H₈).

¹H NMR (DICHLOROMETHANE-d₂ ,400MHz): δ (ppm) 7.97 (d, *J*=8.3 Hz, 1H, Bt-H), 7.41 (ddd, *J*=8.1, 7.1, 0.8 Hz, 1H, Bt-H), 7.36 (ddd, *J*=8.3, 7.1, 1.3 Hz, 1H, Bt-H), 7.19 (d, *J*=8.1 Hz, 1H, Bt-H), 7.05 (d, *J*=8.8 Hz, 2H, Ar-H), 7.10 (d, *J*=8.8 Hz, 2H, Ar-H), 6.04 (s, 2H, OCH₂O), 5.1 (br. s., 1H, NH), 3.74 (s, 3H, OCH₃), 3.38 (d, br., *J*=13.4 Hz, 1H, 3-H), 3.17 (d, *J*=13.6 Hz, 1H, 3-H), 1.56 (s, 3H, 2-CH₃), 1.46 (s, 9H, Boc).

¹³C NMR (101 MHz, DICHLOROMETHANE-d₂) δ ppm 174.3 (C-1), 155.1 (2C-1), 154.2 (3C-4), 143.5 (Bt C-3a), 131.9 (3C-1), 131.5 (3C-2), 128.7 (Bt C-7a), 128.2 (Bt C-6), 124.6 (Bt C-5), 119.9 (Bt C-4), 115.8 (3C-3), 109.0 (Bt C-7), 98.9 (OCH₂O), 60.3 (C-2), 52.4 (1 C-1), 40.7 (br., C-3), 28.1 (2C-3), 23.4 (br., 2-CH₃).

### 2- Synthesis of radioactive compounds

Labelling of 1-1-1, 1-1-2, 1-2-1, 1-2-2, 1-2-3, 1-3, 1-4-1, 1-4-2, 1-5-1, 1-5-2, 1-5-3, 1-5-4, 1-6, 1-7, 1-8, 1-9, 1-10, 1-11-1, 1-11-2 and 1-11-3, 1-12.

### 2.1 General method for radiofluorination

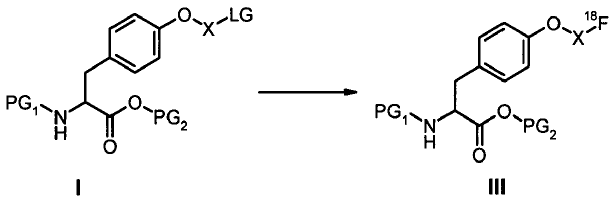

[¹⁸F]Fluoride was immobilized on a preconditioned QMA (Waters) cartridge (preconditioned by washing the cartridge with 5 ml 0.5 M potassium carbonate and 10 ml water), The [¹⁸F]fluoride was eluted using a solution of either:
I) potassium carbonate (1 mg) in 500 µl water and K₂₂₂ (5 mg) in 1500 µl acetonitrile
II) cesium carbonate (2.3 mg) in 500 µl water and K₂₂₂ (5 mg) in 1500 µl acetonitrile
III) 40% tetrabutylammonium hydroxide (aq) (8µl) in 500 µl water and K₂₂₂ (5 mg) in 1500 µl acetonitrile

This solution was dried at 120°C with a nitrogen flow of 150 ml/min. Additional acetonitrile (1 ml) was added and the drying step was repeated. This drying step was repeated once more. A solution of precursor (2 mg) in a solvent (300 µl) was added and heated at elevated temperature for a period of time (See Table 1 for details). The [¹⁸F]fluoride incorporation was analyzed by HPLC (ACE C18 3µ 50 x 4.6 mm; Solvent A: 10 mM dipotassium phosphate in water, Solvent B: 10 mM dipotassium phosphate in acetonitrile:water (7:3); Gradient: 5% B to 95% B in 7 min, 95% B to 100% B in 6 secs, 100% B for 92 secs, 100% B to 5% B in 12 secs, 5% B for 3 min; flow: 2 ml/min). For compound **1-11-3** the [¹⁸F]fluoride incorporation was analyzed via a slightly modified HPLC method was used (ACE C18 3µ 50 x 4.6 mm; Solvent A: 10 mM disodium phosphate in water pH 7.4, Solvent B: acetonitrile; Gradient: 5% B to 95% B in 7 min, 95% B to 100% B in 6 secs, 100% B for 92 secs, 100% B to 5% B in 12 secs, 5% B for 3 min; flow: 2 ml/min).

**Table 1. Radiofluorination results using with different precursors and reaction conditions.**

| **Cpd** | **PG1** | **PG2** | **LG** | **x** | **Base** | **Cryptand** | **Solvent** | **Temp.** | **Time (min)** | **Incorporation** |
|---|---|---|---|---|---|---|---|---|---|---|
| **1-1-1** | Boc | tBu | OBt | CH₂ | TBAOH | na | MeCN DMSO/MeCN (1:1) | 140°C | 15 | 38% |
| **1-1-1** | Boc | tBu | OBt | CH₂ | TBAOH | na | | 140°C | 15 | 40% |
| **1-1-2** | Boc | tBu | OAt | CH₂ | TBAOH | na | MeCN | 120°C | 15 | 26% |
| **1-2-1** | Boc | dicyclopropyl | OBt | CH₂ | TBAOH | na | DMSO | 150°C | 10 | 31% |
| **1-2-2** | Boc | dicyclopropyl | OBtCF₃ | CH₂ | Cs₂CO₃ | K222 (5mg) | DMSO/MeCN (1·1) | 140°C | 15 | 52% |
| **1-2-3** | Boc | dicyclopropyl | OBtNO₂ | CH₂ | TBAOH | na | DMSO/MeCN (1:1) | 140°C | 15 | 12% |
| **1-3** | Boc | DMB | OBt | CH₂ | TBAOH | na | DMSO/MeCN (1:1) | 140°C | 15 | 18% |
| **1-3** | Boc | DMB | OBt | CH₂ | K₂CO₃ | K222 (5mg) | DMSO/MeCN (1:1) | 140°C | 15 | 20% |
| **1-4-1** | Boc | cyclopropyl | OBt | CH₂ | TBAOH | na | DMSO/MeCN (1 1) | 140°C | 15 | 25% |
| **1-4-1** | Boc | cyclopropyl | OBt | CH₂ | K₂CO₃ | K222 (5mg) | DMSO/MeCN (1·1) | 140°C | 15 | 35% |
| **1-4-2** | Boc | cyclopropyl | triazoleC O₂Et | CH₂ | TBAOH | na | DMSO | 120°C | 10 | 12% |
| **1-4-2** | Boc | cyclopropyl | triazoleC O₂Et | CH₂ | Cs₂CO₃ | K222 (5mg) | DMSO | 120°C | 10 | 16% |
| **1-5-1** | Boc | PMB | OBt | CH₂ | K₂CO₃ | K222 (5mg) | DMSO/MeCN (1·1) | 140°C | 15 | 25% |
| **1-5-1** | Boc | PMB | OBt | CH₂ | TBAOH | na | DMSO/MeCN (1,1) | 140°C | 15 | 22% |
| **1-5-2** | Boc | PMB | OBt-Cl | CH₂ | K₂CO₃ | K222 (5mg) | DMSO/MeCN (1:1) | 140°C | 15 | 33% |
| **1-5-3** | Boc | PMB | OBtCF₃ | CH₂ | K₂CO₃ | na | DMSO/MeCN (1:1) | 140°C | 15 | 25% |
| **1-5-4** | Boc | PMB | OBtCF₃ | CH₂ | Cs₂CO₃ | na | DMSO/MeCN (1:1) | 140°C | 15 | 60% |
| **1-6** | Boc | αMeBn | OBt | CH₂ | TBAOH | na | DMSO/MeCN (1:1) | 140°C | 15 | 13% |
| **1-6** | Boc | αMeBn | OBt | CH₂ | K₂CO₃ | K222 (5mg) | DMSO/MeCN (1:1) | 140°C | 15 | 15% |
| **1-7** | Boc | cumyl | OBt | CH₂ | TBAOH | na | DMSO/MeCN (1:1) | 140°C | 15 | 21% |
| **1-7** | Boc | cumyl | OBt | CH₂ | K₂CO₃ | K222 (5mg) | DMSO/MeCN (1.1) | 140°C | 15 | 20% |
| **1-8** | Trt | tBu | OBt | CH₂ | K₂CO₃ | K222 (5mg) | DMSO/MeCN (1.1) | 140°C | 15 | 30% |
| **1-9** | Trt | PMB | OBt | CH₂ | TBAOH | na | DMSO/MeCN (1:1) | 140°C | 15 | 35% |
| **1-9** | Trt | PMB | OBt | CH₂ | K₂CO₃ | K222 (5mg) | DMSO/MeCN (1:1) | 140°C | 15 | 32% |
| **1-10** | Boc | cyclopropyl | OBt | CD₂ | TBAOH | na | DMSO/MeCN (1:1) | 140°C | 15 | 38% |
| **1-10** | Boc | cyclopropyl | OBt | CD₂ | K₂CO₃ | K222 (5mg) | DMSO/MeCN (1:1) | 140°C | 15 | 24% |
| **1-2-2** | Boc | dicycloprop yl | OBt | CH₂ | K₂CO₃ | K222 (5mg) | DMSO/MeCN (1:1) | 140°C | 15 | 29% |
| **1-11-1** | Trt | Dmb | OBt | CH₂ | K₂CO₃ | K222 (5mg) | DMSO/MeCN (1.1) | 140°C | 15 | 30% |
| **1-11-1** | Trt | Dmb | OBt | CH₂ | CS₂CO₃ | K222 (5mg) | DMSO/MeCN (1 1) | 140°C | 15 | 36% |
| **1-11-2** | Trt | Dmb | OBt-Cl | CH₂ | TBAOH | na | DMSO/MeCN (1 1) | 140°C | 15 | 27% |
| **1-11-2** | Trt | Dmb | OBt-Ct | CH₂ | K₂CO₃ | K222 (5mg) | DMSO/MeCN (1-1) | 140°C | 15 | 28% |
| **1-11-3** | Trt | Dmb | OBt-CF3 | CH₂ | K₂CO₃ | K222 (5mg) | DMSO/MeCN (1.1) | 140°C | 15 | 43% |
| **1-11-3** | Trt | Dmb | OBt-CF₃ | CH₂ | Cs₂CO₃ | K222 (5mg) | DMSO/MeCN (1:1) | 140°C | 15 | 56% |
| **1-12** | Boc | Me (α-Me) | OBtl | CH₂ | Cs₂CO₃ | K222 (5mg) | DMSO/MeCN (1·1) | 140°C | 15 | 40% |
| **1-12** | Boc | Me (α-Me) | OBt | CH₂ | K₂CO₃ | K222 (5mg) | DMSO/MeCN (1·1) | 140°C | 15 | 31% |

### 2.2 Example 13

### Radiosynthesis of O-[¹⁸F]Fluoromethyl tyrosine (Precursor 1-2-1)

[¹⁸F]Fluoride (1.72 GBq) was immobilized on a preconditioned QMA (Waters) cartridge (preconditioned by washing the cartridge with 5 ml 0.5M potassium carbonate and 10 ml water), The [¹⁸F]fluoride was eluted using a solution of potassium carbonate (1 mg) in 250 µl water and K₂₂₂ (5 mg) in 1250 µl acetonitrile. This solution was dried at 120°C with stirring under a nitrogen stream. Additional acetonitrile (1 ml) was added and the drying step was repeated. A solution of precursor **1-2-1** (2 mg) in dimethyl sulfoxide:acetonitrile (1:1; 300 µl) was added and heated at 140°C for 15 min. The reaction was diluted with water (20 ml) and passed through a C18 Plus Light (preconditioned by washing the cartridge with 5 ml ethanol and 10 ml water). The SPE cartridge was washed with water (10 ml) and eluted with acetonitrile (1 ml). The elution was concentrated at 70°C with stirring under a nitrogen stream. To this was added dichloromethane:trifluoroacetic acid (1:2, 500 µl) and stirred at r.t. for 2 min. The reaction was concentrated under a nitrogen stream. To the residue was added pH2 water (4ml, water pH adjusted to pH 2 with 0.1 M hydrochloric acid) and purified by HPLC (Synergi Hydro RP 4µ 250 x 10mm; 10% acetonitrile in water at pH 2; flow 5 ml/min). The product peak was collected, diluted with water (pH 2) and passed through a C18 Plus Environmental SPE (preconditioned by washing the cartridge with 5 ml ethanol and 10 ml water). The SPE cartridge was washed with water pH 2 (5 ml). The product was eluted with a 1:1 mixture of ethanol and water pH2 (3 ml). Starting from 1.72 GBq [¹⁸F]fluoride, 132 MBq (5,7 % d.c.) of desired product were obtained in 103 min.

### Example 14

### Radiosynthesis of O-[¹⁸F]Fluoromethyl tyrosine (Precursor 1-3)

[¹⁸F]Fluoride (1.697 GBq) was immobilized on a preconditioned QMA (Waters) cartridge (preconditioned by washing the cartridge with 5 ml 0.5M potassium carbonate and 10 ml water), The [¹⁸F]fluoride was eluted using a solution of potassium carbonate (1 mg) in 500 µl water and K₂₂₂ (5 mg) in 1500 µl acetonitrile. This solution was dried at 120°C with stirring under a nitrogen stream. Additional acetonitrile (1 ml) was added and the drying step was repeated. Additional acetonitrile (1 ml) was added and the drying step was repeated. A solution of precursor **1-3** (2 mg) in dimethyl sulfoxide:acetonitrile (1:1; 300 µl) was added and heated at 140°C for 15 min. The reaction was diluted with water (10 ml) and passed through a C18 Plus Light (preconditioned by washing the cartridge with 5 ml ethanol and 10 ml water). The SPE cartridge was washed with water (5 ml) and eluted with acetonitrile (1 ml). The elution was concentrated at 70°C with stirring under a nitrogen stream. To this was added dichloromethane:trifluoroacetic acid (1:2, 500 µl) and stirred at r.t. for 10 min. The reaction was concentrated under a nitrogen stream. To the residue was added pH2 water at (5ml, water pH adjusted to pH 2 with 0.1 M hydrochloric acid) and purified by HPLC (Synergi Hydro RP 4 µ 250 x 10 mm; 10% acetonitrile in water at pH 2; flow 5 ml/min). The product peak was collected, diluted with water (pH 2) and passed through a C18 Plus Environmental SPE (preconditioned by washing the cartridge with 5 ml ethanol and 10 ml water). The SPE cartridge was washed with water pH 2 (5 ml). The product was eluted with a 1:1 mixture of ethanol and water pH2 (2 ml). Starting from 1.697 GBq [¹⁸F]fluoride, 5.7 MBq (0.8 % d.c.) of desired product was isolated. The product was anaylzed by analytical HPLC (ACE C18 3µ 50 x 4.6 mm; Solvent A: 10 mM dipotassium phosphate in water, Solvent B: 10 mM dipotassium phosphate in acetonitrile:water (7:3); Gradient: 5% B to 95% B in 7 min, 95% B to 100% B in 6 secs, 100% B for 92 secs, 100% B to 5% B in 12 secs, 5% B for 3 min; flow: 2 ml/min).

### Example 15

### Radiosynthesis of O-[¹⁸F]Fluoromethyl-D-tyrosine (Precursor 1-11-1)

[¹⁸F]Fluoride (1063 MBq) was immobilized on a preconditioned QMA (Waters) cartridge (preconditioned by washing the cartridge with 5 ml 0.5M potassium carbonate and 10 ml water), The [¹⁸F]fluoride was eluted using a solution of potassium carbonate (1 mg) in 500 µl water and K₂₂₂ (5 mg) in 1500 µl acetonitrile. This solution was dried at 120°C with stirring under a nitrogen stream. Additional acetonitrile (1 ml) was added and the drying step was repeated. Additional acetonitrile (1 ml) was added and the drying step was repeated. A solution of precursor **1-11-1** (2 mg) in dimethyl sulfoxide:acetonitrile (1:1; 300 µl) was added and heated at 140°C for 15 min. The reaction was diluted with water (10 ml) and passed through a C18 Plus Light (preconditioned by washing the cartridge with 5 ml ethanol and 10 ml water). The SPE cartridge was washed with water (5 ml) and eluted with acetonitrile (1 ml). The elution was concentrated at 70°C with stirring under a nitrogen stream. To this was added dichloromethane:trifluoroacetic acid (1:2, 500 µl) and stirred at r.t. for 10 min. The reaction was concentrated under a nitrogen stream. To the residue was added pH2 water (5 ml, water pH adjusted to pH 2 with 0.1 M hydrochloric acid)and purified by HPLC (Synergi Hydro RP 4µ 250 x 10mm; 10% acetonitrile in water at pH 2; flow 5 ml/min). The product peak was collected, diluted with water (pH 2) and passed through a C18 Plus Environmental SPE (preconditioned by washing the cartridge with 5 ml ethanol and 10 ml water). The SPE cartridge was washed with water pH 2 (5 ml). The product was eluted with a 1:1 mixture of ethanol and water pH2 (2 ml). Starting from 1063 MBq [¹⁸F]fluoride, 1.7 MBq (0.4 % d.c.) of D-FMT was isolated. The product was anaylzed by analytical HPLC (ACE C18 3µ 50 x 4.6 mm; Solvent A: 10 mM dipotassium phosphate in water, Solvent B: 10 mM dipotassium phosphate in acetonitrile:water (7:3); Gradient: 5% B to 95% B in 7 min, 95% B to 100% B in 6 secs, 100% B for 92 secs, 100% B to 5% B in 12 secs, 5% B for 3 min; flow: 2 ml/min) and using a chiral HPLC (Astec Chirobiotic T 250 x 4.6 mm; Solvent A: Water, Solvent B: Ethanol; Gradient: 50% B in A isocratic; flow: 5 ml/min).

### Example 15

### Radiosynthesis of O-[¹⁸F]Fluoromethyl-D-tyrosine (Precursor 1-11-3)

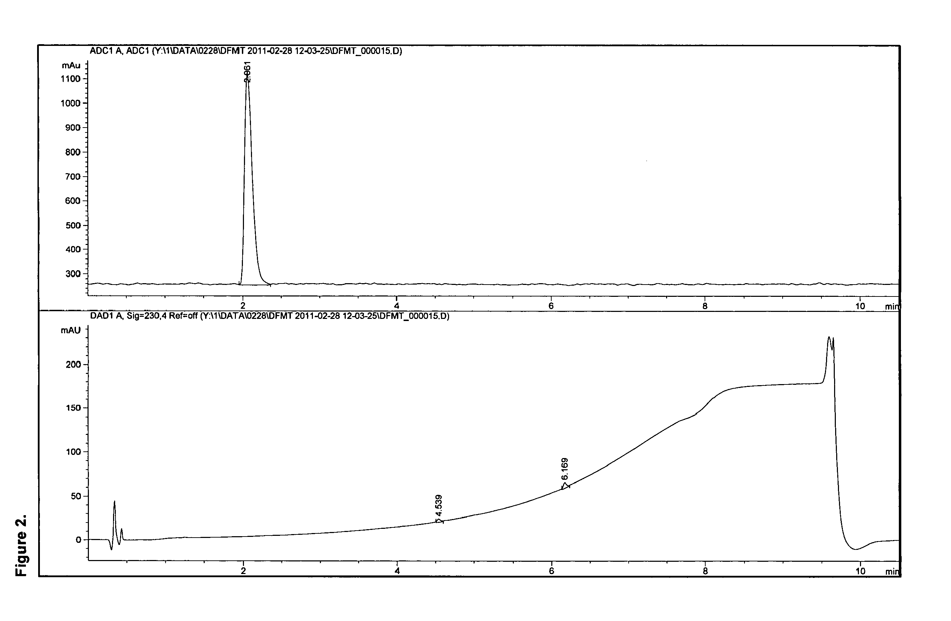

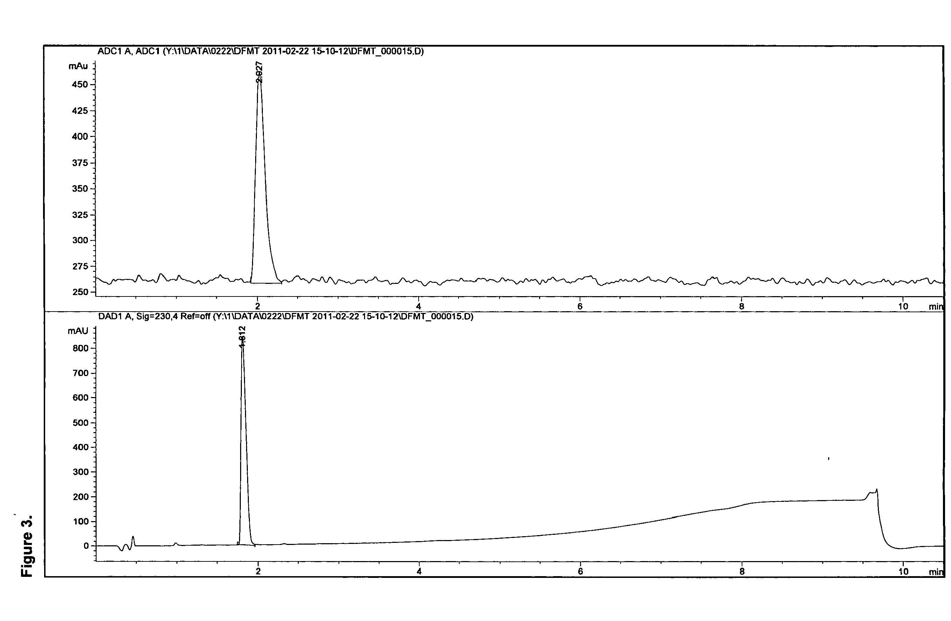

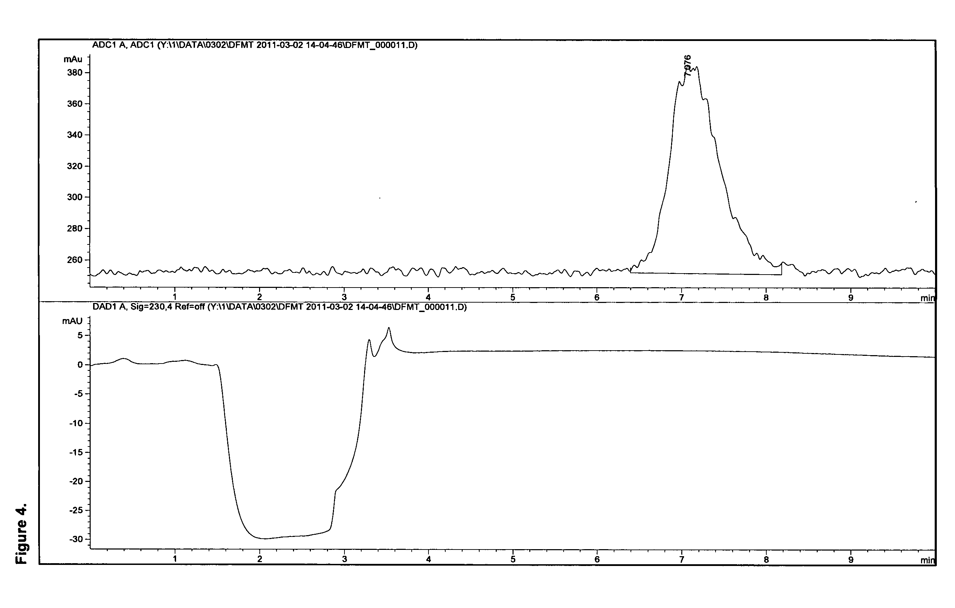

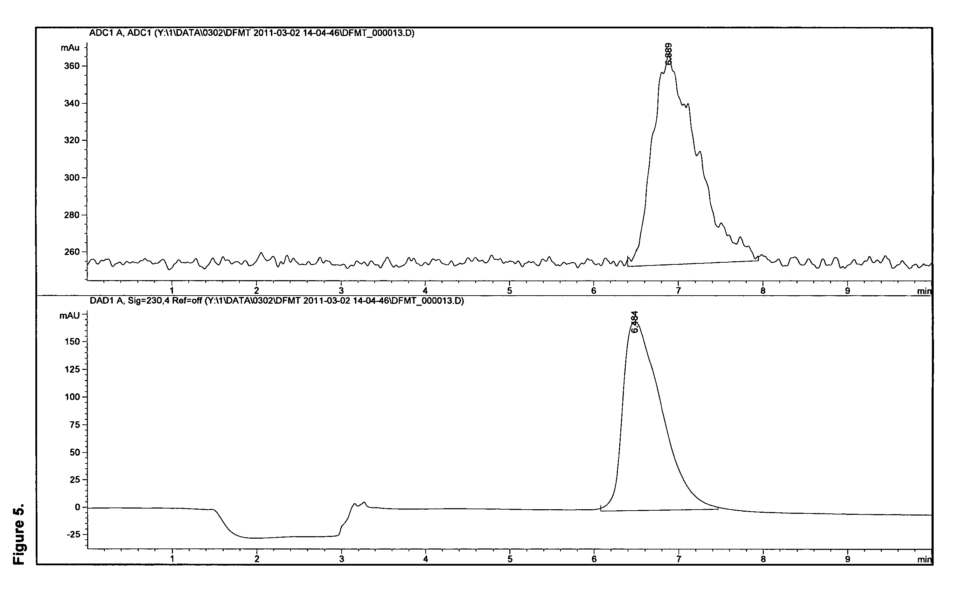

[¹⁸F]Fluoride (2086 MBq) was immobilized on a preconditioned QMA (Waters) cartridge (preconditioned by washing the cartridge with 5 ml 0.5M potassium carbonate and 10 ml water), The [¹⁸F]fluoride was eluted using a solution of potassium carbonate (1 mg) in 500 µl water and K₂₂₂ (5 mg) in 1500 µl acetonitrile. This solution was dried at 120°C with stirring under a nitrogen stream. Additional acetonitrile (1 ml) was added and the drying step was repeated. This azeotropic drying step was repeated twice more. A solution of precursor 1-11-3 (2 mg) in dimethyl sulfoxide:acetonitrile (1:1; 300 µl) was added and heated at 140°C for 15 min. The reaction mixture was diluted with 1,5ml MeCN (1.5 ml) and passed through a Silica Plus SPE (preconditioned with 5ml MeCN). The SPE was washed with MeCN (1.5 ml). This solution was purified by HPLC (ACE 5µ C18, 250 x 10 mm; 85% acetonitrile in water + 0.%TFA; flow 5 ml/min). The product peak was collected, diluted with pH2 water (10 ml, water pH adjusted to pH 2 with 0.1 M hydrochloric acid) and stood for 10min. This solution was passed through a SCX SPE (not preconditioned). The SPE cartridge was washed with pH2 water:MeCN (10 ml, 1:1). The SPE was kept wet for 2min and then air (10 ml) was passed through. through. The SPE cartridge was washed with pH2 water (10 ml, 1:1). The SPE was kept wet for 2min and then air (10 ml) was passed through. The desired product was eluted with a 10 ml buffer solution (7g Na₂HPO₄ and 6g NaCl in 1L). Starting from 2086 MBq [¹⁸ F]fluoride, 161.8 MBq (14.6 % d.c.) of D-FMT was isolated. The product was analyzed by analytical HPLC (Figure 2) (ACE C18 3N 50 x 4.6 mm; Solvent A: water + 0.1 % TFA, Solvent B: acetonitrile + 0.1 % TFA: Gradient: 5% B to 95% B in 7 min, 95% B to 100% B in 6 secs, 100% B for 92 secs, 100% B to 5% B in 12 secs, 5% B for 3 min; flow: 2 ml/min) and with co-injection of the cold standard (Figure 3). The product was also analyzed using a chiral HPLC (Figure 4) (Astec Chirobiotic T 250 x 4.6 mm; Solvent A: Water, Solvent B: Ethanol; Gradient: 50% B in A isocratic; flow: 5 ml/min) and with co-injection of the cold standard (Figure 5). Fig 2, 3, 4 and 5: HPLC Left UV-detector and Right γ-detector.

## Claims

1. A compound of Formula I 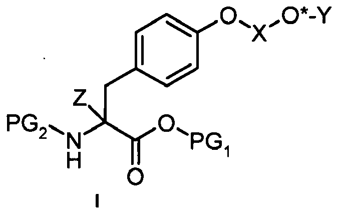 wherein:
**X** is CH₂, CHD or CD₂;
**Y** is a substituted or unsubstituted heteroaromatic ring containing one to four Nitrogen atoms (N)with the proviso that the oxygen (O*) is directly bound to one of the Nitrogen atoms (N) of the heteroaromatic ring and O*-Y acts as leaving group;
**Z** is Hydrogen or methyl;
**PG1** is a carboxylic acid protecting group, containing up to 20 carbon atoms, optionally containing independently one ore more O, N or S atoms; and
**PG2** is an amino protecting group, containing up to 20 carbon atoms, optionally containing one ore more O, N or S atoms and are optionally substituted with one to three halogens.

2. The compound according to claim 1 wherein independently from eachother
**X** is CH₂ or CD₂;
**Y** is a moiety of Formula **II** 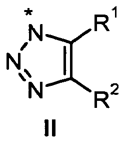
wherein
* indicates the position of the covalent bond to the Oxygen (O*) in Formula 1; **R¹** is H, CN, or COO**R⁴**, and **R²** is H, CN, or COO**R⁴**, or
**R¹** and **R²** form together a 6 membered aromatic ring, which optionally comprise 1
Nitrogen atoms (N) and 1 methine of the 6 membered ring is optionally substituted with halogen, NO₂, CN, COO**R³**, SO₂**R³** or CF₃,
**R³** is C₁-C₃ alkyl, and
**R⁴** is C₁-C₆ alkyl;
**PG1** is
Alkyl,
Alkyl substituted with one phenyl,
Alkyl substituted with one or two C₃-C₆ cydoalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

3. The compound according to claims 1 or 2 wherein
**X** is CH₂ or CD₂;
**Y** is 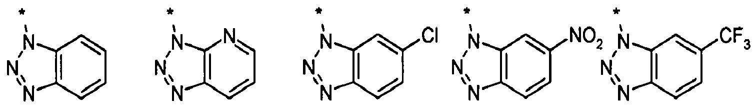
**or** 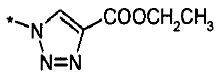
**Z** is Hydrogen or methyl;
**PG1** is dicyclopropylmethyl or2,4-dimethoxybenzyl, and
**PG2** is tert-Butoxycarbonyl (BOC) or Triphenylmethyl (trityl).

4. The compound according to claims 1 to 3 corresponding to compound of Formula (Ia) 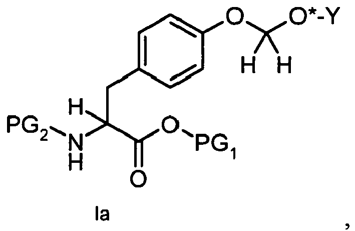 compound of Formula (Ib) 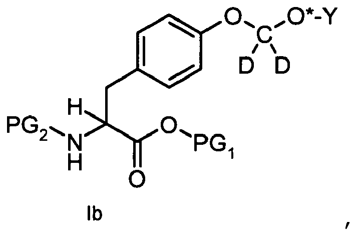 compound of Formula (Ic) 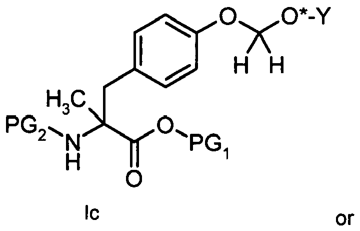 compound of Formula (Id) 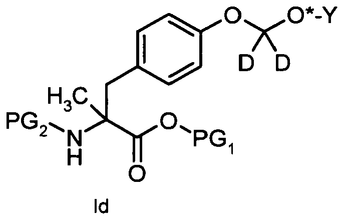 wherein **X, Y, Z, PG1** and **PG2** are defined according to claims 1 to 3.

5. The compound according to claims 1 to 4 corresponding to compound of Formula **(D-I), (D-Ia), (D-Ib), (D-Ic)** or **(D-Id)** 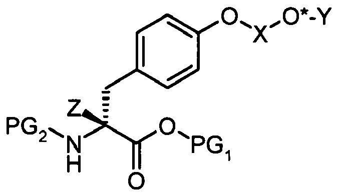
| **Formula\Substituent** | **Z** | **Z** |
|---|---|---|
| **D-I** | H, CH₃ | CH₂ ,CD₂ |
| **D-Ia** | H | CH₂ |
| **D-Ib** | H | CD₂ |
| **D-Ic** | CH₃ | CH₂ |
| **D-Id** | CH₃ | CD₂ |
wherein **Y, PG1** and **PG2** are defined according to claims 1 to 3.

6. The compound according to claims 1 to 5
*tert*-Butyl *O*-[(1*H*-benzotriazol-1-yloxy)methyl]-*N*-(tert-butoxycarbonyl)-D-tyrosinate 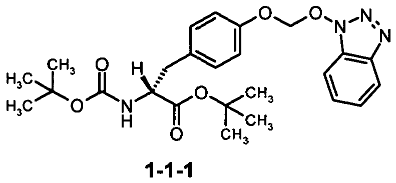
*tert*-Butyl *N*-(*tert*-butoxycarbonyl)-O-[(1*H*-1,2,3-triazolo[5,4-*b*]pyridin-1-yloxy)methyl] -D-tyrosinate 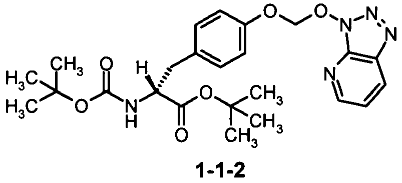
Dicyclopropylmethyl *O*-[(1*H*-benzotriazol-1-yloxy)methyl]-*N*-(tert-butoxycarbonyl) -D-tyrosinate 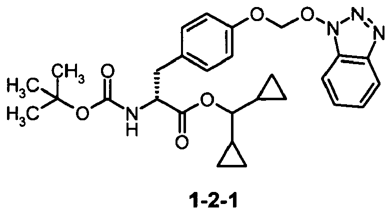
Dicyclopropylmethyl *O*-[(1*H*-benzotriazol-1-yloxy)methyl]-*N*-(*tert*-butoxycarbonyl) -L-tyrosinate 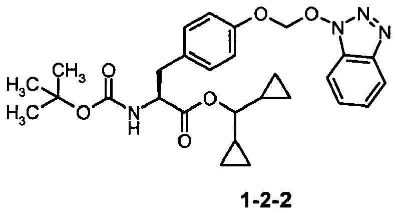
Dicyclopropylmethyl O-[(6-nitro-1H-benzotriazol-1-yloxy)methyl]-N-(*tert*-butoxy carbonyl)-D-tyrosinate 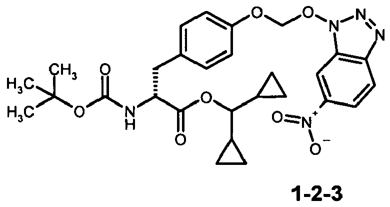
2,4-Dimethoxybenzyl *O*-[(1*H*-benzotriazol-1-yloxy)methyl]-*N*-(*tert*-butoxycarbonyl) -D-tyrosinate 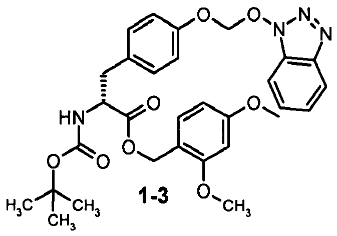
Cyclopropylmethyl *O*-[(1*H*-benzotriazol-1-yloxy)methyl]-*N*-(*tert*-butoxycarbonyl) -D-tyrosinate 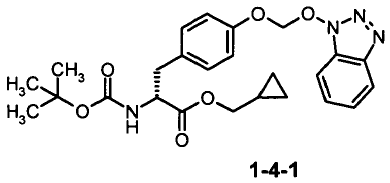
Cyclopropylmethyl *N*-(*tert*-butoxycarbonyl)-*O*-({[4-(ethoxycarbonyl)-1*H-*1,2,3-triazol-1-yl]oxy}methyl)-D-tyrosinate 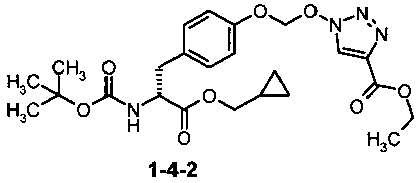
4-Methoxybenzyl *O*-[(1*H*-benzotriazol-1-yloxy)methyl]-*N*-(*tert*-butoxycarbonyl) -D-tyrosinate 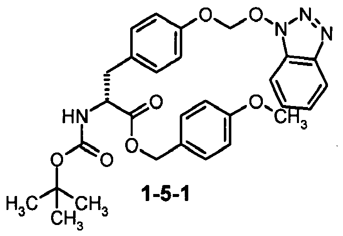
4-Methoxybenzyl *N*-(*tert*-butoxycarbonyl)-*O*-{[(6-chloro-1*H*-benzotriazol-1-yl) oxy]methyl}-D-tyrosinate 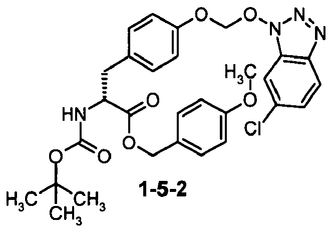
4-Methoxybenzyl *N*-(*tert*-butoxycarbonyl)-*O*-[(6-trifluoromethyl-1H-benzotriazol -1-yloxy)methyl]- D-tyrosinate 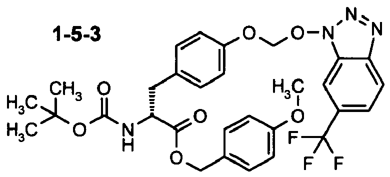
4-Methoxybenzyl *O*-[(6-trifluoromethyl-1*H*-benzotriazol-1-yloxy)methyl]-N-(*tert-*butoxycarbonyl)-L-tyrosinate. 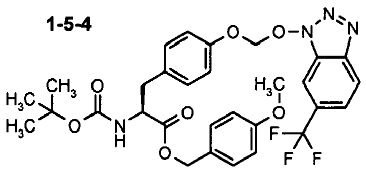
*alpha*-Methylbenzyl *O*-[(1*H*-benzotriazol-1-yloxy)methyl]-*N*-(*tert*-butoxycarbonyl) -D-tyrosinate 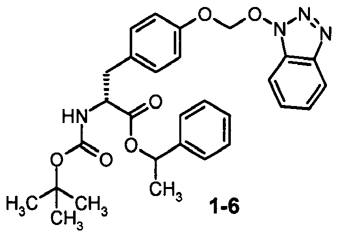
*alpha,alpha*-Dimethylbenzyl *O*-[(1*H*-benzotriazol-1-yloxy)methyl]-*N*-(*tert*-butoxycarbonyl) -D-tyrosinate 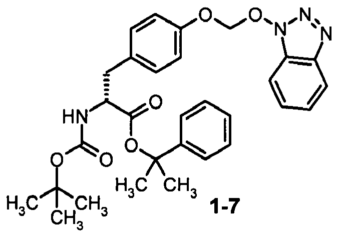
*tert*-Butyl *O*-[(1*H*-benzotriazol-1-yloxy)methyl]-*N*-trityl-D-tyrosinate 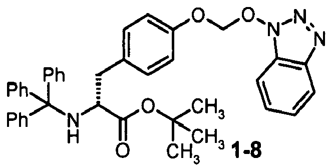
4-Methoxybenzyl *O*-[(1*H*-benzotriazol-1-yloxy)methyl]-*N*-trityl-D-tyrosinate 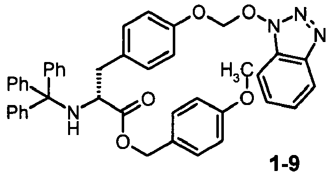
Cyclopropylmethyl *O*-[(1*H*-benzotriazol-1-yloxy)[²H_{2]}methyl]-*N*-(*tert*-butoxycarbonyl)-D-tyrosinate 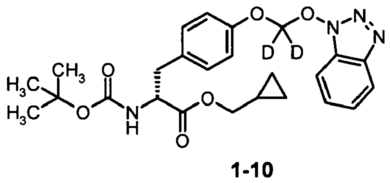
2,4-Dimethoxybenzyl *O*-[(1*H*-benzotriazol-1-yloxy)methyl]-*N*-trityl-D-tyrosinate 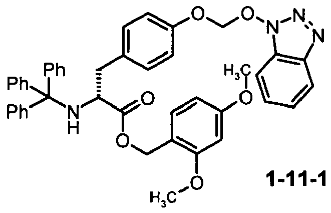
2,4-Dimethoxybenzyl *O*-{[(6-chloro-1*H*-benzotriazol-1-yl)oxy]methyl}-*N*-trityl-D-tyrosinate 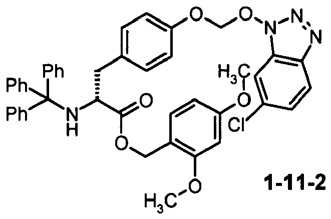
2,4-Dimethoxybenzyl *O*-{[(6-thf)uoromethyl-1*H*-benzotriazol-1-yl)oxy]methyl}-*N*-trityl-D-tyrosinate 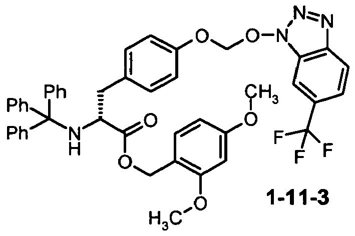
Methyl *O*-[(1*H*-benzotriazol-1-yloxy)methyl]-*N*-(tert-butoxycarbonyl)-*alpha-*methyltyrosinate

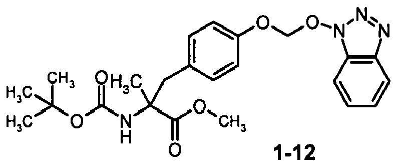

7. A compound of Formula III 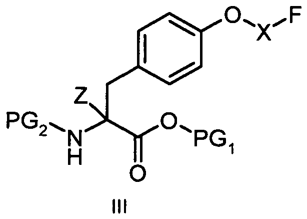 wherein:
**X** is CH_{2,}CHD or CD₂;
**F** is ¹⁸F or¹⁹F;
**Z** is Hydrogen or methyl;
**PG1** is a carboxylic protecting group, containing up to 20 carbon atoms, optionally containing independently one ore more O, N or S atoms; and
**PG2** is an amino protecting group, containing up to 20 carbon atoms, optionally containing one ore more O, N or S atoms and are optionally substituted with one or two halogens.

8. The compound according to claim 7 wherein independently from eachother **X** is CH₂ or CD₂;
**F** is ¹⁸F or¹⁹F;
**PG1** is
Alkyl,
Alkyl substituted with one phenyl,
Alkyl substituted with one or two C₃-C₆ cycloalkyl,
Alkyl substituted with one phenyl and one C₃-C₆ cycloalkyl, or fluorenylmethyl
wherein
Alkyl is a branched or linear C₁-C₆ alkyl, and optionally substituted with C₁-C₃ alkoxy, and
Phenyl is optionally substituted with up to three C₁-C₃ alkyl, C₁-C₃ alkoxy or halogen; and
**PG2** is selected from the group comprising Carbobenzyloxy (Cbz), p-Methoxybenzyl carbonyl (Moz or MeOZ), tert-Butoxycarbonyl (BOC), 9-Fluorenylmethoxycarbonyl (FMOC), Triphenylmethyl (trityl), 4-Methylphenyl-diphenylmethyl (Mtt) and 4-Methoxyphenyldiphenylmethyl (MMTr).

9. The compound according to claims 7 or 8 wherein
**X** is CH₂ or CD₂;
**F** is ¹⁸F;
**Z** is Hydrogen or methyl;
**PG1** is dicyclopropylmethyl or 2,4-dimethoxybenzyl, and
**PG2** is tert-Butoxycarbonyl (BOC) or Triphenylmethyl (trityl).

10. The compound according to claims 7 to 9 corresponding to compound of Formula **(IIIa)** 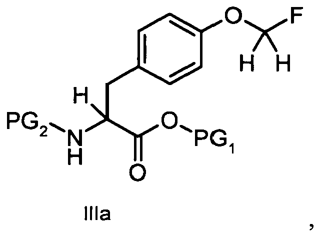 compound of Formula **(IIIb)** 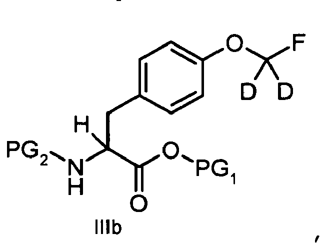 compound of Formula **(IIIc)** 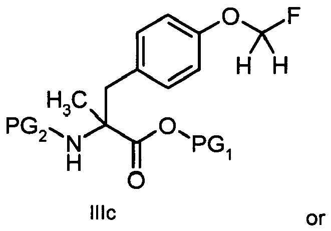 compound of Formula **(IIId)** 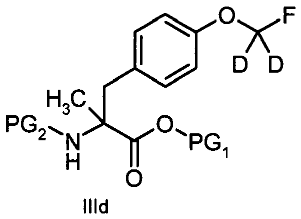 wherein **X, F, Z, PG1** and **PG2** are defined according to claims 7 to 9.

11. The compound according to claims 7 to 10 corresponding to compound of Formula **(D-III), (D-IIia), (D-IIIb), (D-IIIc)** or **(D-IIId)** 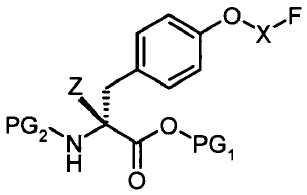
| **Formula\Substituent** | **Z** | **X** |
|---|---|---|
| **D-III** | H, CH₃ | CH₂,CD₂ |
| **D-IIIa** | H | CH₂ |
| **D-IIIb** | H | CD₂ |
| **D-IIIc** | CH₃ | CH₂ |
| **D-IIId** | CH₃ | CD₂ |
wherein **F, PG1** and **PG2** are defined according to claims 7 to 10.

12. The compound according to claims 7 to 11
*tert*-Butyl *N*-(*tert-*butooycarbonyl)-*O*-(fluoromethyl)-D-tyrosinate 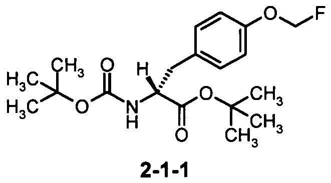
Dicyclopropylmethyl *N*-(*tert*-butoxycarbonyl)-*O*-(fluoromethyl)-D-tyrosinate 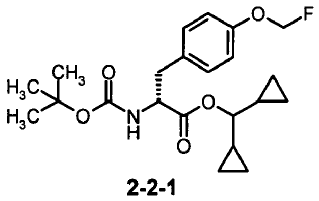
Dicyclopropylmethyl *N*-(*tert*-butoxycarbonyl)-*O*-(fluoromethyl)-L-tyrosinate 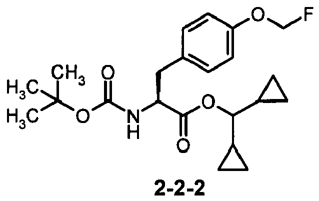
*tert*-Butyl *O*-(fluoromethyl)-*N*-trityl-D-tyrosinate 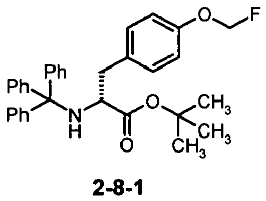
2,4-Dimethoxybenzyl *O*-(fluoromethyl)-*N*-trityl-D-tyrosinate 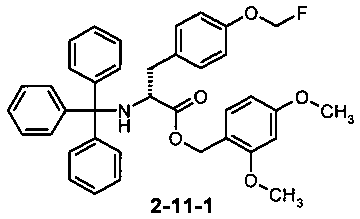
Methyl *N*-(*tert*-butoxycarbonyl)-*O*-(fluoromethyl)-*alpha*-methyl-D-tyrosinate 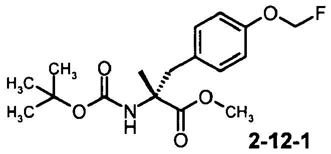
Methyl *N*-(*tert*-butoxycarbonyl)-*O*-(fluoromethyl)-*alpha*-methyl-L-tyrosinate

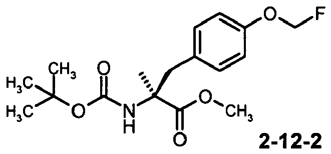

13. The compound according to claims 7 to 11
*tert*-Butyl *N*-(*tert*-butoxycarbonyl)-*O*-([¹⁸F]fluoromethyl)-D-tyrosinate. Labelling of 1-1-1 and 1-1-2
Dicyclopropylmethyl *N*-(*tert*-butoxycarbonyl)-*O*-([¹⁸F]fluoromethyl)-D-tyrosinate. Labelling of 1-2-1 and 1-2-3
Dicyclopropylmethyl *N*-(tert-butoxycarbonyl)-*O*-([¹⁸F]fluoromethyl)-L-tyrosinate. Labelling of 1-2-2
2,4-Dimethoxybenzyl *N*-(*tert*-butoxycarbonyl)-*O*-([¹⁸F]fluoromethyl)-D-tyrosinate. Labelling of 1-3
Cyclopropylmethyl *N*-(*tert*-butoxycarbonyl)-*O*-([¹⁸F]fluoromethyl)-D-tyrosinate. Labelling of 1-4-1 and 1-4-2
4-Methoxybenzyl *N*-(*tert*-butoxycarbonyl)-*O*-([¹⁸F]fluoromethyl)-D-tyrosinate. Labelling of 1-5-1, 1-5-2 and 1-5-3
4-Methoxybenzyl *N*-(*tert*-butoxycarbonyl)-*O*-([¹⁸F]fluoromethyl)-L-tyrosinate. Labelling of 1-5-4
*alpha*-Methylbenzyl *N*-(*tert*-butoxycarbonyl)-*O*-([¹⁸F]fluoromethyl)-D-tyrosinate. Labelling of 1-6
*alpha*,*alpha*-Dimethylbenzyl *N*-(*tert*-butoxycarbonyl)-*O*-([¹⁸F]fluoromethyl)- D-tyrosinate. Labelling of 1-7
*tert*-Butyl *O*-([¹⁸F]fluoromethyl)-*N*-trityl-D-tyrosinate. Labelling of 1-8
4-Methoxybenzyl *O*-([¹⁸F]fluoromethyl)-*N*-trityl-D-tyrosinate. Labelling of 1-9
Cyclopropylmethyl *N*-(*tert*-butoxycarbonyt)-*O*-([¹⁸F]fluoro]²H₂]methy)-D-tyrosinate. Labelling of 1-10
2,4-Dimethoxybenzyl *O*-([¹⁸F]fluoromethyl)-*N*-trityl-D-tyrosinate Labelling of 1-11-1, 1-11-2 and 1-11-3
Methyl *N*-(*tert*-butoxycarbonyl)-*O*-([¹⁸F]fluoromethyl)-*alpha*-methyl-DL-tyrosinate. Labelling of 1-12

14. A composition comprising compound(s) of the Formula **III, IIIa, IIIb, IIIc, IIId, (D-III), (D-IIIa), (D-IIIb), (D-IIIc)** or **(D-IIId)** independently or mixtures thereof and reagents suitable for deprotection of the amino group and the ester function of the tyrosine,

15. A composition comprising compound(s) of the Formula **I, Ia, Ib, Ic, Id, (D-I), (D-Ia), (D-Ib), (D-Ic)** or **(D-Id)** independently or mixtures thereof and reagents suitable for fluoro labelling.

16. A kit comprising a sealed vial containing a predetermined quantity of a compound of Formula **I, Ia, Ib, Ic, Id, (D-I), (D-Ia), (D-Ib), (D-Ic) or (D-Id)** independently or mixtures thereof and suitable salts of inorganic or organic acids, hydrates and solvates.

17. A method for obtaining compounds of Formula **I** comprising the step of
- Reacting compound of Formula **V** first with N-Chloro-succinimide (NCS) and then with anion of H-O*-**Y** for obtaining compounds of Formula **I,**
wherein 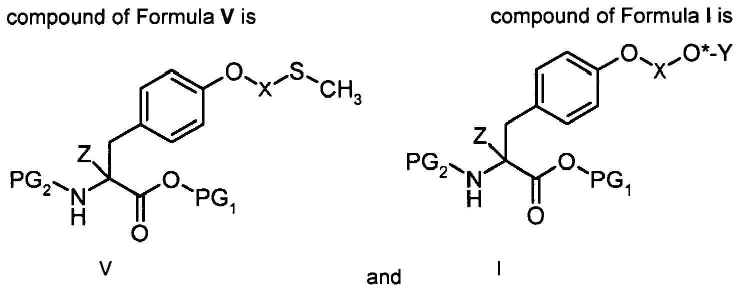 **Z, PG1, PG2, X,** and **Y** are as defined according to claims 1 to 6.

18. A method for obtaining compounds of Formula **III** comprising the step of
- Reacting compound of Formula **I** with a ¹⁸F-Fluorination agent, and
- [Optionally] converting obtained compound into a suitable salts of inorganic or organic bases thereof, hydrates, complexes, and solvates thereof
wherein 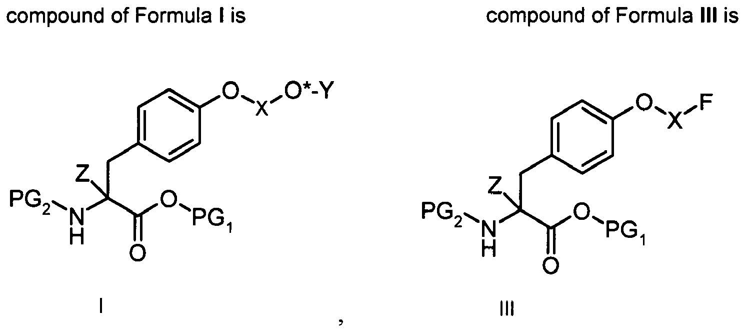 and **F, Z, PG1, PG2, X,** and **Y** are as defined according to claims 1 to 6 and 7 to 13.
